(19) Europäisches Patentamt
European Patent Office
Office européen des brevets

(11) EP 2 394 987 A1

(12) **EUROPEAN PATENT APPLICATION**
published in accordance with Art. 153(4) EPC

(43) Date of publication:
**14.12.2011 Bulletin 2011/50**

(21) Application number: **10738632.8**

(22) Date of filing: **08.02.2010**

(51) Int Cl.:
***C07C 279/22*** *(2006.01)* ***A61K 31/085*** *(2006.01)*
***A61K 31/167*** *(2006.01)* ***A61K 31/192*** *(2006.01)*
***A61K 31/216*** *(2006.01)* ***A61K 31/235*** *(2006.01)*
***A61K 31/277*** *(2006.01)* ***A61K 31/341*** *(2006.01)*
***A61K 31/351*** *(2006.01)* ***A61K 31/36*** *(2006.01)*
***A61K 31/366*** *(2006.01)* ***A61K 31/381*** *(2006.01)*
***A61K 31/404*** *(2006.01)* ***A61K 31/415*** *(2006.01)*
***A61K 31/423*** *(2006.01)* ***A61K 31/4245*** *(2006.01)*
***A61K 31/426*** *(2006.01)* ***A61K 31/44*** *(2006.01)*
***A61K 31/47*** *(2006.01)* ***A61K 31/472*** *(2006.01)*
***A61K 31/505*** *(2006.01)*

(86) International application number:
**PCT/JP2010/051756**

(87) International publication number:
**WO 2010/090304 (12.08.2010 Gazette 2010/32)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **09.02.2009 JP 2009026838**

(71) Applicant: **Astellas Pharma Inc.**
**Tokyo 103-8411 (JP)**

(72) Inventors:
- **KINOYAMA, Isao**
**Tokyo 103-8411 (JP)**
- **MIYAZAKI, Takehiro**
**Tokyo 103-8411 (JP)**
- **KOGANEMARU, Yohei**
**Tokyo 103-8411 (JP)**
- **SHIRAISHI, Nobuyuki**
**Tokyo 103-8411 (JP)**
- **KAWAMOTO, Yuichiro**
**Tokyo 103-8411 (JP)**
- **WASHIO, Takuya**
**Tokyo 103-8411 (JP)**

(74) Representative: **HOFFMANN EITLE**
**Patent- und Rechtsanwälte**
**Arabellastraße 4**
**81925 München (DE)**

# (54) ACYLGUANIDINE DERIVATIVE

(57) An object of the present invention is to provide an excellent agent for treating or preventing dementia, schizophrenia based on a serotonin 5-$HT_{5A}$ receptor modulating action.

It was confirmed that acylguanidine derivatives, which has the characteristic structure in which the guanidine is bonded to one ring of a naphthalene via a carbonyl group and a cyclic group is bonded to the other ring thereof, exhibit potent 5-$HT_{5A}$ receptor modulating action and excellent pharmacological action based on the action. The present invention is useful as an excellent agent for treating or preventing dementia, schizophrenia, bipolar disorder or attention deficit hyperactivity disorder.

EP 2 394 987 A1

## Description

Technical Field

The present invention relates to pharmaceuticals, particularly to acylguanidine derivatives with 5-$HT_{5A}$ receptor modulating action, useful as an agent for treating or preventing dementia, schizophrenia, and the like.

Background Art

In recent years, it has been suggested that the 5-$HT_{5A}$ receptor which is one of the subtypes of serotonin receptors plays an important role in dementia and schizophrenia. For example, it has been reported that new exploratory behaviors are increased in the 5-$HT_{5A}$ receptor knock-out mice, and hyperactivity by LSD is inhibited in the 5-$HT_{5A}$ receptor knock-out mice (Neuron, 22, 581-591, 1999). From the results of gene expression analysis, it has been reported that the 5-$HT_{5A}$ receptor is highly expressed in human and rodent brain, and in brain, it is highly expressed in hippocampal CA1 and CA3 pyramidal cells which are related to memory, and frontal lobe (cerebral cortex) which is deeply related to schizophrenia (Molecular Brain Research, 56, 1-8, 1998). Furthermore, it has been reported that gene polymorphism of the 5-$HT_{5A}$ receptor relates to schizophrenia (Neuroreport 11, 2017-2020, 2000; Mol. Psychiatr. 6, 217-219, 2001; and J. Psychiatr. Res. 38, 371-376, 2004). Accordingly, it is suggested that regulation of 5-$HT_{5A}$ receptor action leads to the improvement of dementia and schizophrenia and compounds with such function are needed.

Hitherto, several kinds of compounds having affinity for the 5-$HT_{5A}$ receptor have been reported. For example, it has been described that a guanidine derivative represented by the following general formula binds to the 5-$HT_{5A}$ receptor and thus is used for treating multiple central diseases such as a neurodegenerative diseases and a neurophychiatric diseases (Patent Document 1).

[Chem. 1]

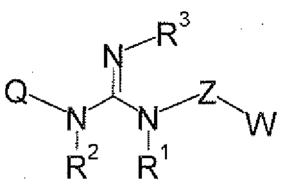

W:

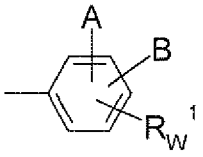

or

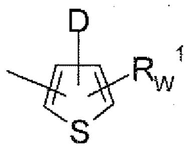

(A represents $NO_2$ $NH_2$, or the like; B represents a hydrogen atom, or the like; $R_w^1$ represents a hydrogen atom, or the like; D represents a group represented by A; Q represents a di-substituted 5-membered heteroaryl; $R^1$, $R^2$, and $R^3$ each represent a hydrogen atom, or the like; and Z represents $-(CR_z^1R_z^2)_a-(V_z)_b-(CR_z^3R_z^4)_c-$, in which a and c each represent 0 to 4, b represents 0 or 1, $R_z^1$, $R_z^2$, $R_z^3$ and $R_z^4$ each represents a hydrogen atom, or the like, and $V_z$ represents CO, or the like. For details on these, refer to the publication.)

None of the 5-$HT_{5A}$ receptor modulators which have been reported has a structure in which the guanidine is bonded to a naphthalene via a carbonyl group. On the other hand, several compounds having the aforesaid structure, which are used for other uses, are known.
For example, it has been reported that a derivative represented by the following general formula has an antiviral activity, and is useful in the treatment of HIV, HCV infections, and the like (Patent Document 2).

[Chem. 2]

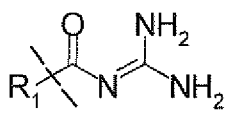

R1:

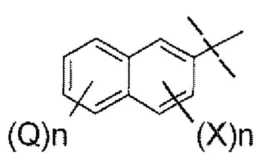

,

and the like
($R^1$ represents phenyl, substituted phenyl, naphthyl, substituted naphthyl, or the above structure; n represents 1, 2, 3

or 4; Q independently represents hydrogen, cycloalkyl, thienyl, furyl, pyrazolyl, pyridyl, substituted pyridyl, phenyl, substituted phenyl, or the like; and X represents hydrogen or alkoxy. For details on these, refer to the publication.)
Furthermore, a patent application regarding a compound having similar structure has been filed by the present applicants (Patent Document 3). These publications have no description concerning the 5-$HT_{5A}$ receptor modulating action of the above derivatives, or their use for treating schizophrenia of dementia.

In addition, naphthalene derivatives which exhibit inhibitory action on $Na^+$/$H^+$ exchange mechanisms and are useful for the treatment of myocardial infarction, angina pectoris or the like have been reported (Patent Documents 4 to 7 and Non-patent Document 1). None of these documents describes the 5-$HT_{5A}$ receptor modulating action of naphthalene derivatives, or their use for treating dementia or schizophrenia.

List of the Documents

Patent Document

Patent Document 1: WO 05/08287 pamphlet
Patent Document 2: WO 06/135978 pamphlet
Patent Document 3: WO 04/112687 pamphlet
Patent Document 4: U.S. Patent No. 6087304 Specification
Patent Document 5: U.S. Patent No. 6093729 Specification
Patent Document 6: Japanese Patent Publication JP-A-8-225513
Patent Document 7: U.S. Patent No. 5824691 Specification

Non-patent Document

Non-patent Document 1: Takeshi Yamamoto, et al., Chemical and Pharmaceutical Bulletin, 1997, Vol. 45, No. 8, p. 1282-1286.

Disclosure of the Invention

Problem that the Invention is to Solve

An object of the present invention is to provide an excellent agent for treating or preventing dementia, schizophrenia, or the like, based on the 5-$HT_{5A}$ receptor modulating action.

Means for Solving the Problem

As a result of intense research on compounds exhibiting 5-$HT_{5A}$ receptor modulating action, the present inventors discovered that acylguanidine derivatives, in which the guanidine is bonded to the 2-position of a naphthalene via a carbonyl group, and a cyclic group is bonded to the 8-position thereof, exhibit potent 5-$HT_{5A}$ receptor modulating action and therefore excellent pharmacological activities, and that they can be an agent for treating or preventing dementia, schizophrenia or the like, thereby completed the present invention.
That is, the present invention relates to compound of formula (I) or a pharmaceutically acceptable salt thereof.
[Chem. 3]

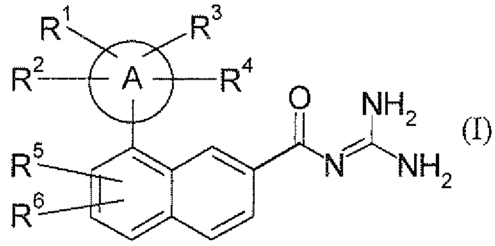

(I)

(wherein symbols have the following meanings:

[Chem. 4]

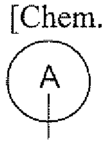

rep

represents phenyl, naphthyl, cycloalkyl, monocyclic or bicyclic heteroaryl, or a saturated or partially unsaturated monocyclic oxygen-containing heterocyclic group;

$R^1$, $R^2$, $R^3$ and $R^4$ are the same as or different from each other and represent H, lower alkyl, halogen, halogeno-lower alkyl, -CN, $-NO_2$ $-NR^bR^c$, $-OR^a$, -O-halogeno-lower alkyl, $-C(O)NR^bR^c$, $-C(O)R^a$, $-CO_2R^a$, $NR^bC(O)R^a$, lower alkylene-$OR^a$, phenyl, or, monocyclic nitrogen-containing heteroaryl, or $R^1$ and $R^2$ are combined together to form $-O-(CH_2)_n-O-$, $-O-CF_2-O-$, $-O-C_2H_4-$, or $-CO-C_2H_4-$,

in which the monocyclic nitrogen-containing heteroaryl may be substituted with lower alkyl;

n is 1,2 or 3;

$R^a$, $R^b$ and $R^c$ are the same as or different from each other and represent H or lower alkyl; and

$R^5$ and $R^6$ are the same as or different from each other and represent H, halogen or lower alkyl).

In this connection, unless otherwise specifically noted, when a symbol in a chemical formula is used in another chemical formula in the present specification, the same symbols have the same meaning.

In addition, the present invention relates to a pharmaceutical composition containing compound of the aforesaid formula (I) or a pharmaceutically acceptable salt thereof and a pharmaceutically acceptable excipient; for example, the aforesaid pharmaceutical composition which is a $5-HT_{5A}$ receptor modulator; in another example, the aforesaid pharmaceutical composition which is a preventive or therapeutic agent for dementia, schizophrenia, bipolar disorder or attention deficit hyperactivity disorder; in yet another example, the aforesaid pharmaceutical composition which is a preventive or therapeutic agent for dementia or schizophrenia.

Also, in another embodiment of the present invention, it is use of the compound of the aforesaid formula (I) or a pharmaceutically acceptable salt thereof for the manufacture of a $5-HT_{5A}$ receptor modulator, for example, a preventive or therapeutic agent for dementia, schizophrenia, bipolar disorder or attention deficit hyperactivity disorder, in particular, a preventive or therapeutic agent for dementia or schizophrenia; in another embodiment, it is a method for preventing or treating dementia, schizophrenia, bipolar disorder or attention deficit hyperactivity disorder, in particular, a method for preventing or treating dementia or schizophrenia, comprising administering a therapeutically effective amount of the compound of the aforesaid formula (I) or a pharmaceutically acceptable salt thereof to a mammal.

Effects of the Invention

Compounds of the present invention have an advantage of potent $5-HT_{5A}$ receptor modulating action, and excellent pharmacological actions based on it. Thus, pharmaceutical compositions of the present invention are useful for treatment or prevention of $5-HT_{5A}$ receptor-related diseases, and particularly, for prevention or treatment of dementia, schizophrenia, bipolar disorder, or attention deficit hyperactivity disorder.

Modes for Carrying Out the Invention

Hereinafter, the present invention is described in more detail.

In the present specification, the "$5-HT_{5A}$ receptor modulator" is a generic term referring to a compound that inhibits activation of the $5-HT_{5A}$ receptor by antagonizing with an endogenous ligand ($5-HT_{5A}$ antagonist), and a compound that

shows function by activation of the 5-$HT_{5A}$ receptor (5-$HT_{5A}$ agonist).

The "lower alkyl" is a linear or branched alkyl having 1 to 6 carbon atoms (hereinafter simply referred to as $C_{1-6}$), and specifically, methyl, ethyl, n-propyl, isopropyl, n-butyl, isobutyl, sec-butyl, tert-butyl, n-pentyl, n-hexyl groups, and the like. In one embodiment, it is $C_{1-4}$ alkyl, and in another embodiment, it is methyl, ethyl, n-propyl, and isopropyl groups.

The "lower alkylene" is linear or branched $C_{1-6}$ alkylene, and specifically, methylene, ethylene, trimethylene, tetramethylene, pentamethylene, hexamethylene, propylene, methylmethylene, ethylethylene, 1,2-dimethylethylene, 1,1,2,2-tetramethylethylene groups, and the like. In another embodiment, it is $C_{1-4}$ alkylene, and in another embodiment, it is methylene, ethylene, trimethylene, and propylene groups.

The "halogen" means F, Cl, Br, and I.

The "halogeno-lower alkyl," is $C_{1-6}$ alkyl substituted with one or more halogen. For example, it is $C_{1-6}$ alkyl substituted with 1 to 5 halogens, and in another embodiment difluoromethyl and trifluoromethyl groups.

The "cycloalkyl" is a $C_{3-10}$ saturated hydrocarbon ring group, which may have a bridge. Specifically, it is cyclopropyl, cyclobutyl, cyclopentyl, cyclohexyl, cycloheptyl, cyclooctyl, and adamantyl groups; in another embodiment, it is $C_{3-6}$ cycloalkyl, and in another embodiment cyclopropyl group.

The "monocyclic heteroaryl" refers to a 5- or 6-membered unsaturated group which contains 1 to 4 hetero atoms selected from oxygen, sulfur and nitrogen. Sulfur or nitrogen atoms which form the monocycle, may be oxidized and thus form oxide or dioxide. Specific examples of monocyclic heteroaryl include pyridyl, pyrrolyl, pyrazinyl, pyrimidinyl, pyridazinyl, imidazolyl, triazolyl, thienyl, furyl, pyranyl, thiazolyl, pyrazolyl, isothiazolyl, oxazolyl, oxadiazolyl, isooxazolyl, and tetrazolyl groups; in another embodiment, it is pyridyl, pyrimidinyl, thienyl, thiazolyl, pyrazolyl, and oxadiazolyl groups; in yet another embodiment, it is a pyridyl group.

The "bicyclic heteroaryl" refers to a group formed by condensation of two of the aforesaid "monocyclic heteroaryl" rings; or a group formed by condensation of one of the aforesaid "monocyclic heteroaryl" ring and a benzene ring. Examples thereof include quinolyl, isoquinolyl, quinazolinyl, quinoxalinyl, phthalazinyl, benzimidazolyl, benzofuryl, benzothienyl, benzothiadiazolyl, benzothiazolyl, benzoisothiazolyl, benzoxazolyl, benzoisooxazolyl, indolyl, isoindolyl, indolinyl, indazolyl groups; in another embodiment, it is a cyclic group containing a nitrogen atom among them; in yet another embodiment, it is a quinolyl, isoquinolyl, indolyl and benzoxazolyl group.

The "monocyclic nitrogen-containing heteroaryl" refers to an unsaturated 5- to 6-membered monocyclic group which contains one nitrogen atom and may further contain hetero atoms selected from nitrogen, oxygen and sulfur, among the "monocyclic heteroaryl" above. Examples of the monocyclic nitrogen-containing heteroaryl include pyridyl, pyrimidinyl, thiazolyl, pyrazolyl and oxadiazolyl groups.

The "saturated or partially unsaturated monocyclic oxygen-containing cyclic group" refers to a 3- to 7-membered saturated or partially unsaturated monocyclic group which contains one oxygen atom, and may additionally contain one hetero atom selected from nitrogen, oxygen, and sulfur, and examples thereof include oxylanyl, oxetanyl, tetrahydrofuryl, tetrahydropyranyl, dihydropyranyl, and 1,4-dioxanyl groups; in another embodiment, it is a tetrahydropyranyl or dihydropyranyl group.

Some embodiments of compound of formula (I) are shown below.

(1) The compound wherein

[Chem. 5]

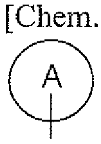

represents phenyl, naphthyl, cyclopropyl, pyridyl, pyrimidinyl, thienyl, thiazolyl, pyrazolyl, oxadiazolyl, quinolyl, isoquinolyl, indolyl, benzoxazolyl, tetrahydropyranyl or dihydropyranyl group; in another embodiment, phenyl or pyridyl group.

(2) The compound wherein $R^1$, $R^2$, $R^3$ and $R^4$ are the same as or different from each other and represent H, lower alkyl, halogen, halogeno-lower alkyl, -CN, -$OR^a$, -O-halogeno-lower alkyl, -C(O)$NR^bR^c$, lower alkylene-$OR^a$, phenyl or oxadiazolyl optionally substituted with methyl group; in another embodiment, H, F, Cl, CN or -$OR^a$; in another embodiment, $R^1$ and $R^2$ are combined together to form $-O-(CH_2)_n-O-$, $-O-CF_2-O-$, $-O-C_2H_4-$, or $-CO-C_2H_4-$.

(3) The compound mentioned in (2) wherein n represents 1 or 2.

(4) The compound mentioned in (2) wherein $R^a$, $R^b$ and $R^c$ are the same as or different from each other and represent H, methyl or ethyl.

(5) The compound wherein $R^5$ and $R^6$ are the same as or different from each other and represent H, F, Cl or methyl.

(6) The compound with the groups mentioned in (1) and (2) above.

(7) The compound with the groups mentioned in (1) and (4) above.
(8) The compound with the groups mentioned in (1), any one of (2) to (4) and (5) above.
(9) The compound or a salt thereof selected from the group consisting of
N-(diaminomethylene)-8-(2,4,6-trifluorophenyl)-2-naphthamide,
8-(2-cyano-3-fluorophenyl)-N-(diaminomethylene)-2-naphthamide,
N-(diaminomethylene)-8-(3,5-difluoropyridin-4-yl)-2-naphthamide,
8-(3-chloro-5-fluoropyridin-2-yl)-N.(diaminomethylene)-2-naphthamide,
8-(4-cyano-2-methoxyphenyl)-N-(diaminomethylene)-2-naphthamide,
N-(diaminomethylene)-8-(2,5-dichloropyridin-4-yl)-2-naphthamide,
8-(3-chloropyridin-4-yl)-N-(diaminomethylene)-2-naphthamide,
8-(2-chloro-6-fluorophenyl)-N-(diaminomethylene)-2-naphthamide,
N-(diaminomethylene)-8-(2-fluoro-6-hydroxyphenyl)-2-naphthamide,
8-(2-chloro-4-fluorophenyl)-N-(diaminomethylene)-2-naphthamide,
N-(diaminomethylene)-8-quinolin-5-yl-2-naphthamide, and,
N-(diaminomethylene)-8-(2,4-difluoro-6-hydroxyphenyl)-2-naphthamide.

Compound of formula (I) may exist as other tautomers, geometrical isomers, or optical isomers, depending on the kind of the substituents. The present invention includes these isomers, isolated forms, or mixtures thereof.
Furthermore, pharmaceutically acceptable prodrugs of compound of formula (I) are also included in the present invention. Pharmaceutically acceptable prodrugs refer to compounds which have a group that can be converted into an amino group, OH, $CO_2H$, or the like by solvolysis or under physiological conditions, thus releasing compound of formula (I) *in vivo* after administration. Examples of the group forming prodrugs include the groups described in "Prog. Med., 5, 2157-2161 (1985), and "Iyakuhin no Kaihatsu (Development of Medicines)" (Hirokawa Publishing company, 1990), vol. 7, Bunshi Sekkei (Molecular Design)", 163-198.
Furthermore, compound of formula (I) may form an acid addition salt, or may form a salt with a base depending on the kind of substituents, and the salts are included in the present invention as long as they are pharmaceutically acceptable salts. Specifically, examples of these salts include acid addition salts with inorganic acids such as hydrochloric acid, hydrobromic acid, hydroiodic acid, sulfuric acid, nitric acid, and phosphoric acid, and with organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid, and glutamic acid, salts with inorganic bases such as sodium, potassium, magnesium, calcium, and aluminum, and organic bases such as methylamine, ethylamine, ethanolamine, lysine, and ornithine, and ammonium salts.
In addition, compound of formula (I) and a pharmaceutically acceptable salt thereof may exist as hydrates, solvates, and crystal polymorphs and the present invention includes them all. Also, compound of formula (I) and a pharmaceutically acceptable salt thereof include those labeled with radioactive or non-radioactive isotopes.

(Production Processes)

Compound of formula (I) and a pharmaceutically acceptable salt thereof can be produced by applying various known synthetic methods, utilizing its basic skeleton or type of substituents. Protection of the functional groups with suitable protecting groups (a group which can be easily converted into the original functional group) may be effective in technical means, depending on the kind of the functional group, in any step from starting materials to intermediates. Examples of the functional group include amino group, hydroxyl group, and carboxyl group, and examples of the protecting group include those described in "Green's Protective Groups in Organic Synthesis (4th Edition, 2006)", edited by P. G. M. Wuts and T. W. Greene, which can be optionally selected and used depending on the reaction conditions. In this way, a desired compound can be obtained by introducing a protecting group to carry out the reaction, and then, removing the protecting group, if desired.
In addition, prodrugs of compound of formula (I) can be produced by introducing a specific group during any step from starting materials to intermediates, in a similar way to the aforementioned protecting groups, or by carrying out a reaction using the obtained compound of formula (I). The reaction may be carried out by employing a method known to a skilled person in the art, such as ordinary esterification, amidation, and dehydration.
Hereinbelow, representative production processes of compound of formula (I) are described. Each production process can be carried out according to the references cited in the description. Further, production processes of the present invention are not limited to the examples as shown below.

(General production processes)

[Chem. 6]

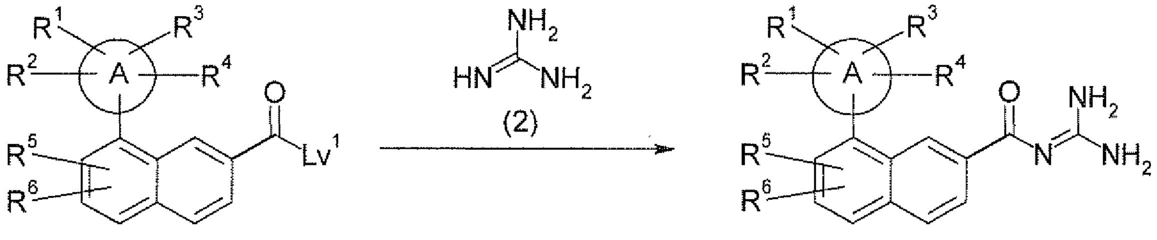

(1)

(2)

(I)

($Lv^1$ represents -OH or a leaving group.)

Compound of formula (I) can be produced by reaction of a carboxylic acid or a reactive derivative thereof (1) with guanidine (2) or a salt thereof.

The reaction can be carried out using equivalent amounts of the carboxylic acid or a reactive derivative thereof (1) and guanidine (2), or excess amount of guanidine. It can be carried out under cooling to under heating, preferably from -20°C to 80°C, in a solvent inert to the reaction, such as aromatic hydrocarbons such as benzene, toluene, or xylene; halogenated hydrocarbons such as dichloromethane, 1,2-dichloroethane, or chloroform; ethers such as diethylether, tetrahydrofuran (THF), dioxane, or dimethoxyethane (DME); N,N-dimethylformamide (DMF); dimethylsulfoxide (DMSO); N-methylpyrolidone (NMP); ethyl acetate; acetonitrile; or water; or mixtures thereof.

When a carboxylic acid wherein $Lv^1$ is OH is used as starting compound (1), it is desirable to carry out the reaction in the presence of a condensing agent. In this case, examples of the condensing agent include N,N'-dicyclohexylcarbodiimide (DCC), 1-[3-(dimethylamino)propyl]-3-ethylcarbodiimide (WSC), 1,1'-carbonyldiimidazole (CDI), 2-(1H-benzotriazol-1-yl)-1,1,3,3-tetramethyluronium hexafluorophosphate (HBTU), diphenylphosphoryl azide (DPPA), and phosphorous oxychloride. In some cases, it is preferable to further use additive agents (e.g., N-hydroxysuccinimide (HONSu), 1-hydroxybenzotriazole (HOBt) and the like). The condensing agent is usually used in an equivalent amount or excess to the carboxylic acid.

Examples of the reactive derivative of the carboxylic acid when $Lv^1$ is a leaving group in starting compound (1), are acid halides (acid chloride, acid bromide, or the like), acid anhydrides (mixed acid anhydride with phenyl chlorocarbonate, p-toluenesulfonic acid, isovaleric acid, or the like or symmetric acid anhydrides), active esters (esters which can be prepared using phenol that may be substituted with an electron withdrawing group such as a nitro group or a fluorine atom, HOBt, HONSu and the like), lower alkyl esters. Each of them can be produced from carboxylic acid using reactions obvious to those skilled in the art. Addition of bases (organic bases such as triethylamine, diisopropylethylamine (DIPEA), N-methylmorpholine, pyridine, or 4-(N,N-dimethylamino)pyridine, or inorganic bases such as sodium hydrogen carbonate, or the like) may be advantageous for smooth progress of the reaction, depending on the kinds of the reactive derivatives. Pyridine can also serve as a solvent. In this connection, when a lower alkyl ester is used as the reactive derivative, it is preferable to carry out the reaction from room temperature to refluxing with heating.

Starting compound (1) for general production processes may be prepared by known methods or any variation thereof. For example, starting compound (1a) may be prepared in accordance with the following reaction scheme (Production process of the starting compound).

(Production process of the starting compound)

[Chem. 7]

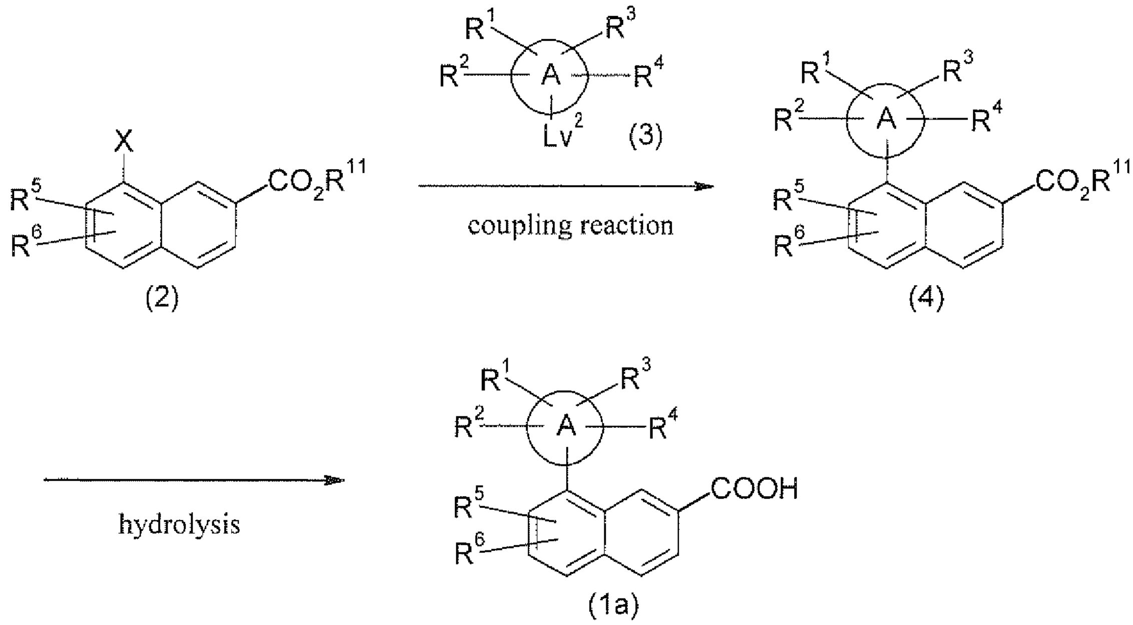


(2) (3) (4) (1a)

(In the formula, X represents trifluoromethanesulfonyloxy, $-B(OH)_2$ or -B(OZ)OW, $R^{11}$ represents a protecting group of a carboxyl group such as lower alkyl or benzyl, and $Lv^2$ represents a leaving group. Here, Z and W are the same as or different from each other and represent lower alkyl, or Z and W are combined together to form a lower alkylene.)

Compound (1a) may be obtained by coupling reaction of compound (2) with compound (3) to obtain compound (4) and hydrolyzing compound (4).

Examples of leaving groups represented by $Lv^2$ include halogen, methanesulfonyloxy, p-toluenesulfonyloxy, trifluoromethanesulfonyloxy groups, and the like.

Compound (4) may be synthesized by stirring compound (2) and compound (3) in equivalent amounts or in excess amount of one of them; in a reaction inert solvent in the presence of a base and palladium catalyst at room temperature or under refluxing with heating for usually 0.1 hours to 5 days. The reaction is carried out preferably under an inert gas atmosphere. Examples of solvents used herein include, but are not particularly limited to, aromatic hydrocarbons, ethers, halogenated hydrocarbons, alcohols, DMF, DMSO, and mixed solvent thereof. As the bases, inorganic bases such as sodium carbonate, potassium carbonate and sodium hydroxide are preferred. As the palladium catalysts, tetrakis(triphenylphosphine)palladium, dichlorobis(triphenylphosphine)palladium, palladium-1,1'-bis(diphenylphosphino)ferrocene chloride and the like are preferred.

The coupling reaction may be carried out with reference to the following documents.

[Documents]

A. d. Meijere and F. Diederich et al., "Metal-Catalyzed Cross-Coupling Reactions", 1st edition, VCH Publishers Inc., 1997

The Chemical Society of Japan, "Courses in Experimental Chemistry (5th edition)" Vol. 13 (2005) (Maruzen)

Subsequently, compound (4) is subjected to a hydrolysis reaction to obtain compound (1a). The hydrolysis reaction may be carried out with reference to P. G. M. Wuts and T. W. Greene, "Green's Protective Groups in Organic Synthesis (4th edition, 2006)".

(Other production processes)

In addition, the above described compounds (2) and (3) (Production process of the starting compound) may be prepared by known methods or any variation thereof, for example, in accordance with the methods mentioned in the following Preparation Examples.

Compound of formula (I) prepared in accordance with the aforementioned methods is isolated and purified as

a free compound, as a pharmaceutically acceptable salt thereof, as a hydrate or as solvate thereof, or a crystalline polymorph thereof. Pharmaceutically acceptable salts of compound of formula (I) may be prepared using salt preparation methods well-known to those skilled in the art.
Isolation and purification are carried out by applying common chemical operations such as extraction, fractional crystallization and fractional chromatography.
A variety of isomers may be isolated by selecting suitable starting compounds or using differences in physicochemical properties among the isomers. For example, optical isomers may be led into stereo chemically pure isomers by a general optical resolution method (for example, fractional crystallization to lead into diastereomer salts with an optically active base or acid, or chromatography using a chiral column). Also, it can be prepared from suitable optical active starting compounds.

Examples

Hereinafter, production processes of compound of formula (I) are described as Examples. In addition, production processes of compounds used as starting compounds are described as Preparation Examples. Production processes of compound of formula (I) are not limited to the production processes of the following specific Examples, but the compounds may be prepared by combining these production processes or known production processes.

Preparation Example 1

One drop of perchloric acid was added to a mixture of methyl 7-methyl-8-oxo-5,6,7,8-tetrahydronaphthalene-2-carboxylate (600 mg), acetic anhydride (2.8 g) and carbon tetrachloride (2.4 mL), followed by stirring at room temperature overnight. The reaction mixture was diluted with ethyl acetate and washed with aqueous saturated sodium bicarbonate and then saturated brine and the organic layer was concentrated under reduced pressure. The resulting residue was purified by silica gel column chromatography (hexane/ethyl acetate) to obtain methyl 8-acetoxy-7-methyl-5,6-dihydronaphthalene-2-carboxylate (569 mg).

Preparation Example 2

Boron tribromide (1M dichloromethane solution, 4.1 mL) was added under ice cooling to a mixture of methyl 8-(2-fluoro-6-methoxyphenyl)-2-naphthalene carboxylate (420 mg) and dichloromethane (10 mL), followed by stirring at the same temperature for 16 hours. Water was slowly added to the reaction mixture, followed by stirring for 5 minutes and extraction with ethyl acetate. The organic layer was washed with water, dried and concentrated under reduced pressure to obtain 8-(2-fluoro-6-laydroxyphenyl)-2-naphthalene carbonic acid (380 mg).

Preparation Example 3

A mixture of 2-bromo-5-fluorophenol (3 g), sodium chlorodifluoroacetate (6 g), cesium carbonate (7.7 g), water (3 mL) and DMF (30 mL) was stirred under heating at an oil temperature of 100°C for 15 hours. The reaction mixture was cooled to room temperature, diluted with water and extracted with ethyl acetate. The organic layer was washed with 1M aqueous sodium hydroxide solution, further washed with water, dried and concentrated under reduced pressure to obtain 1-bromo-2-(difluoromethoxy)-4-fluorobenzene (2.77 g).

Preparation Example 4

A mixture of methyl 8-hydroxy-2-naphthalene carboxylate (615 mg), 2,3,4,5,6,6-hexachloro-2,4-cyclohexadien-1-one (1,0 g), DMF (5 mL) and carbon tetrachloride (30 mL) was stirred at room temperature for one day. The reaction mixture was diluted with water and extracted with ethyl acetate. The organic layer was concentrated under reduced pressure and the resulting residue was purified by silica gel column chromatography (hexane/ethyl acetate) to obtain methyl 7-chloro-8-hydroxy-2-naphthalene carboxylate (245 mg).

Preparation Example 5

A mixture of methyl 8-hydroxy-2-naphthalene carboxylate (532 mg), sulfuryl chloride (781 mg) and chloroform (150 mL) was stirred at room temperature for one day. The reaction mixture was diluted with water and extracted with chloroform. The organic layer was concentrated under reduced pressure and the resulting residue was purified by silica gel column chromatography (hexane/ethyl acetate) to obtain methyl 5,7-dichloro-8-hydroxy-2-naphthalene carboxylate (606 mg).

Preparation Example 6

A mixture of methyl 8-acetoxy-7-methyl-5,6-dihydronaphthalene-2-carboxylate (560 mg), 4,5-dichloro-3,6-dioxocyclohexa-1,4-diene-1,2-dicarbonitrile (1.47 g) and 1,4-dioxane (20 mL) was stirred under heating at an oil temperature of 80°C for 3 hours. The reaction mixture was cooled to room temperature, diluted with ethyl acetate and washed with saturated brine, and the organic layer was concentrated under reduced pressure. The resulting residue was purified by silica gel column chromatography (hexane/ethyl acetate) to obtain methyl 8-acetoxy-7-methyl-2-naphthalene carboxylate (359 mg).

Preparation Example 7

A mixture of 2-chloro-5-fluoro-3-nitropyridine (4 g), iron powder (6.3 g), ammonium chloride (606 mg), THF (20 mL), water (20 mL) and ethanol (40 mL) was stirred under refluxing with heating for 5 hours. The reaction mixture was cooled to room temperature, the insoluble matter was separated by filtration and the filtrate was extracted with ethyl acetate. The organic layer was washed with water, dried and concentrated under reduced pressure to obtain 2-chloro-5-fluoropyridin-3-amine (3.3 g).

Preparation Example 8

A mixture of methyl 8-acetoxy-7-methyl-2-naphthalene carboxylate (380 mg), potassium carbonate (407 mg) and methanol (16 mL) was stirred at room temperature for 2 hours. The reaction mixture was diluted with a saturated aqueous ammonium chloride solution and extracted with ethyl acetate and the organic layer was concentrated under reduced pressure. The resulting residue was purified by silica gel column chromatography (hexane/ethyl acetate) to obtain methyl 8-hydroxy-7-methyl-2-naphthalene carboxylate (318 mg).

Preparation Example 9

n-Butyl lithium (1.58 M n-hexane solution, 6.5 mL) was added at -78°C to a solution of diisopropylamine (1.5 mL) in THF (40 mL), followed by stirring at 0°C for 30 minutes. Methyl 8-oxo-5,6,7,8-tetrahydronaphthalene-2-carboxylate (2.0 g) was added at -78°C to the reaction mixture, followed by stirring at the same temperature for one hour. Hexamethyl phosphoramide (5 mL) and methyl iodide (1 mL) were further added to the reaction mixture, followed by stirring at room temperature for one hour. The reaction mixture was diluted with water and extracted with ethyl acetate. The organic layer was concentrated under reduced pressure and the resulting residue was purified by silica gel column chromatography (hexane/ethyl acetate) to obtain methyl 7-methyl-8-oxo-5,6,7,8-tetrahydronaphthalene-2-carboxylate (897 mg).

Preparation Example 10

A mixture of methyl 8-(2,6-difluoro-4-formylphenyl)-2-naphthalene carboxylate (226 mg), sodium borohydride (26 mg), THF (10 mL) and methanol (30 mL) was stirred at room temperature for 3 hours. The reaction mixture was diluted with water and extracted with ethyl acetate. The organic layer was washed with water, dried and concentrated under reduced pressure to obtain methyl 8-[2,6-difluoro-4-(hydroxymethyl)phenyl]-2-naphthalene carboxylate (227 mg).

Preparation Example 11

A mixture of methyl 8-(2-chloro-6-fluorophenyl)-2-naphthalene carboxylate (676 mg), a 1M aqueous sodium hydroxide solution (7 mL), THF (10 mL) and ethanol (10 mL) was stirred at room temperature for 16 hours. The reaction mixture was diluted with water, concentrated under reduced pressure and neutralized with 1M hydrochloric acid. The precipitate was collected by filtration to obtain 8-(2-chloro-6-fluorophenyl)-2-naphthalenecarbonic acid (620 mg).

Preparation Example 12

A mixture of methyl 8-{2,6-difluoro-4-[(hydroxyimino)methyl]phenyl}-2-naphthalene carboxylate (349 mg), a 1M aqueous sodium hydroxide solution (5 mL) and methanol (20 mL) was stirred at room temperature for 7 hours. The reaction mixture was concentrated under reduced pressure, the resulting residue was diluted with water and neutralized with 1M hydrochloric acid, and the precipitate was collected by filtration. A mixture of the resulting solid and acetic anhydride (3 mL) was stirred under refluxing with heating for one day. The reaction mixture was concentrated under reduced pressure and the resulting residue was diluted with water and extracted with ethyl acetate. The organic layer was concentrated under reduced pressure and the resulting residue was purified by silica gel column chromatography

(chloroform/methanol) to obtain 8-(4-cyano-2,6-difluorophenyl)-2-naphthalene carbonic acid (95 mg).

Preparation Example 13

A mixture of 2-cyclopropyl-4-methyl-1,3-thiazole (890 mg), N-bromosuccinimide (1.25 g) and acetonitrile (50 mL) was stirred under refluxing with heating for 3 hours. The reaction mixture was cooled to room temperature, diluted with water and extracted with ethyl acetate. The organic layer was concentrated under reduced pressure and the resulting residue was purified by silica gel column chromatography (hexane/ethyl acetate) to obtain 5-bromo-2-cyclopropyl-4-mcthyl-1,3-thiazole (320 mg).

Preparation Example 14

A mixture of methyl 8-(1-methyl-1H-pyrazol-5-yl)-2-naphthalene carboxylate (100 mg), N-chlorosuccinimide (50 mg) and acetic acid (5 mL) was stirred at room temperature for 3 hours and stirred under heating at an oil temperature of 80°C for 12 hours. The reaction mixture was cooled to room temperature, diluted with water and extracted with ethyl acetate. The organic layer was concentrated under reduced pressure to obtain methyl 8-(4-chlora-1-methyl-1H-pyrazol-5-yl)-2-naphthalene carboxylate (107 mg).

Preparation Example 15

A mixture of sodium nitrite (1.5 g) and water (4 mL) was added dropwise to a mixture of 2-chloro-5-fluoropyridin-3-amine (2 g) and concentrated hydrochloric acid (30 mL) at below 5°C, followed by stirring at the same temperature for 10 minutes. A mixture of copper (I) chloride (1.35 g) and concentrated hydrochloric acid (10 mL) was further added at the same temperature to the reaction mixture, followed by stirring at room temperature for 2 hours. The reaction mixture was neutralized and diluted with ethyl acetate and the insoluble matter was separated by filtration. The filtrate was subjected to liquid separation and the organic layer was concentrated under reduced pressure. The resulting residue was purified by silica gel column chromatography (hexane/ethyl acetate) to obtain 2,3-dichloro-5-fluoropyridine (1.0 g).

Preparation Example 16

Trifluoromethanesulfonic anhydride (21.6 g) was added at 0°C to a mixture of methyl 8-hydroxy-2-naphthalene carboxylate (10 g), triethylamine (8.0 g) and dichloromethane (100 mL), followed by further stirring at room temperature for 3 days. The reaction mixture was concentrated under reduced pressure, diluted with water and extracted with ethyl acetate and the organic layer was concentrated under reduced pressure. The resulting residue was purified by silica gel column chromatography (hexane/ethyl acetate) to obtain methyl 8-{[(trifluoromethyl)sulfonyl]oxy}-2-naphthalene carboxylate (12.5 g).

Preparation Example 17

A mixture of methyl 8-(4-cyanophenyl)-2-naphthalene carboxylate (270 mg), hydroxylamine hydrochloride (98 mg), diisopropylethylamine (0.49 mL), methanol (30 mL) and THF (30 mL) was stirred under refluxing with heating for 4 hours. The reaction mixture was cooled to room temperature, diluted with water and extracted with ethyl acetate. The organic layer was washed with water, dried and concentrated under reduced pressure to obtain methyl 8-{4-[amino (hydroxyimino)methyl]phenyl}-2-naphthalene carboxylate (320 mg).

Preparation Example 18

Sodium hydride (55% dispersed in liquid paraffin, 25 mg) was added to a mixture of methyl 8-[2,6-difluoro-4-(hydroxylmethyl)phenyl]-2-naphthalene carboxylate (123 mg), iodomethane (266 mg) and THF (10 mL), followed by stirring at room temperature for 3 hours. The reaction mixture was diluted with 1M hydrochloric acid and extracted with ethyl acetate. The organic layer was concentrated under reduced pressure and the resulting residue was purified by silica gel column chromatography (chloroform/methanol) to obtain methyl 8-[2,6-difluoro-4-(methoxymethyl)phenyl]-2-naphthalene carboxylate (84 mg).

Preparation Example 19

Concentrated sulfuric acid (769 mg) was added to a mixture of 5-fluoro-8-hydroxy-2-naphthalenecarbonic acid (539 mg) and methanol (10 mL), followed by stirring under refluxing with heating for 15 hours. Water was added to the

reaction mixture, followed by extraction with ethyl acetate. The organic layer was washed with water, dried and concentrated under reduced pressure to obtain methyl 5-fluoro-8-hydroxy-2-naphthalene carboxylate (530 mg).

Preparation Example 20

Acetyl chloride (0.1 mL) was added at 0°C to a mixture of methyl 8-{4-[amino(hydroxyimino)methyl]phenyl}-2-naphthalene carboxylate (320 mg) and pyridine (20 mL), followed by stirring under refluxing with heating for 3 days. The reaction mixture was concentrated under reduced pressure, and the resulting residue was diluted with water and extracted with ethyl acetate. The organic layer was concentrated under reduced pressure and the resulting residue was purified by silica gel column chromatography (hexane/ethyl acetate) to obtain methyl 8-[4-(5-methyl-1,2,4-oxadiazol-3-yl) phenyl]-2-naphthalene carboxylate (160 mg).

Preparation Example 21

A mixture of 2-amino-6-bromophenol (1 g) and trimethylorthoacetate (3.5 g) was stirred under refluxing with heating for 10 hours. The reaction mixture was concentrated under reduced pressure and the resulting residue was purified by silica gel column chromatography (hexane/ethyl acetate) to obtain 7-bromo-2-methyl-1,3-benzoxadiazole (873 mg).

Preparation Example 22

A mixture of methyl 8-(2,6-difluoro-4-formylphenyl)-2-naphthalene carboxylate (308 mg), hydroxylamine hydrochloride (197 mg), triethylamine (478 mg) and methanol (20 mL) was stirred at room temperature for one day. The reaction mixture was diluted with water and extracted with ethyl acetate, and the organic layer was concentrated under reduced pressure to obtain methyl 8-{2,6-difluoro-4-[(hydroxyimino)methyl]phenyl}-2-naphthalene carboxylate (349 mg).

Preparation Example 23

n-Butyl lithium (1.66 M n-hexane solution, 6.5 mL) was added to a solution of diisopropylamine (2.4 g) in THF (60 mL) at -78°C under an argon gas atmosphere, followed by stirring at the same temperature for 30 minutes. A mixture of 3,5-difluorobenzonitrile (3 g) and THF (20 mL) was added dropwise at -78°C to the reaction mixture, followed by stirring at the same temperature for 2 hours. A mixture of chlorotrimethylsilane (2.6 g) and THF (20 mL) was further added dropwise to the reaction mixture, followed by stirring at the same temperature for one hour and warming to room temperature. The reaction mixture was diluted with water, the insoluble matter was separated by filtration and the filtrate was extracted with diethylether. The organic layer was washed with aqueous saturated sodium bicarbonate, dried and concentrated under reduced pressure, and the resulting residue was purified by silica gel column chromatography (hexane/ethyl acetate) to obtain 3,5-difluoro-4-(trimethylsilyl)benzonitrile (3.1 g).

Preparation Example 24

A mixture of cyclopropane carbothioamide (673 mg), I-bromoacetone (1.1 g), toluene (30 mL) and chloroform (30 mL) was stirred under heating at an oil temperature of 50°C for 3 hours. The reaction mixture was concentrated under reduced pressure and the resulting residue was purified by silica gel column chromatography to obtain 2-cyclopropyl-4-methyl-1,3-thiazole (900 mg).

Preparation Example 25

A mixture of methyl 8-{[(trifluoromethyl)sulfonyl]oxy}-2-naphthalene carboxylate (2 g), bis(pinacolato)diborone (1.7 g), chlorobis(triphenylphosphine)palladium (210 mg), triphenylphosphine (160 mg) and potassium acetate (1.77 g) and 1,4-dioxane (40 mL) was stirred with heating at an oil temperature of 100°C for 18 hours. The reaction mixture was cooled to room temperature, the insoluble matter was separated by filtration and the filtrate was concentrated under reduced pressure. The resulting residue was diluted with water and extracted with ethyl acetate, and the organic layer was concentrated under reduced pressure. The resulting residue was purified by silica gel column chromatography (hexane/ethyl acetate) to obtain methyl 8-(4,4,5,5-tetramethyl-1,3,2-dioxaborolan-2-yl)-2-naphthalene carboxylate (1.65 g).

Preparation Example 26

A mixture of methyl 8-oxo-5,6,7,8-tetrahydronaphthalene-2-carboxylate (3.0 g), 1-fluoro-4-hydroxy-1,4-diazaniabicyclo[2,2,2]octanebis(tetrafluoroborate) (5.2 g) and methanol (140 mL) was stirred under refluxing with heating for 3 hours. The reaction mixture was concentrated under reduced pressure and diluted with dichloromethane and the insoluble matter was separated by filtration. The filtrate was concentrated under reduced pressure and the resulting residue was purified by silica gel column chromatography (hexane/ethyl acetate) to obtain methyl 7-fluoro-8-oxo-5,6,7,8-tetrahydronaphthalene-2-carboxylate (2.8 g).

Preparation Example 27

n-Butyl lithium (1.55M hexane solution, 10 mL) was added at -78°C to a mixture of diisopropylamine (1.4 g) and THF (20 mL), followed by stirring at the same temperature for 30 minutes. A mixture of 5-chloro-2-methoxypyridine (1 g) and THF (5 mL) was added dropwise to the reaction mixture at -78°C, followed by stirring at the same temperature for one hour. A mixture of triisopropyl borate (2.62 g) and THF (5 mL) was further added to the reaction mixture at the same temperature, followed by warming the reaction mixture to room temperature and stirring for 2 days. The reaction mixture was diluted with water and a 1M aqueous sodium hydroxide solution was added thereto, followed by extraction with ethyl acetate. The resulting aqueous layer was neutralized with 1M hydrochloric acid and extracted with ethyl acetate. The resulting organic layer was washed with water, dried and concentrated under reduced pressure to obtain (5-chloro-2-methoxypyridin-4-yl)boric acid (1.28 g).

Preparation Example 28

n-Butyl lithium (1.55M hexane solution, 10 mL) was added to a mixture of 2,2,6,6-tetramethylpiperidine (2.2 g) and THF (20 mL) at 78°C, followed by stirring at the same temperature for 30 minutes. A mixture of 2-chloronicotinonitrile (1 g) and THF (5 mL) was added dropwise at -78°C, followed by stirring at the same temperature for one hour. A mixture of triisopropyl borate (2.62 g) and THF (5 mL) was further added to the reaction mixture at the same temperature, followed by warming the reaction mixture to room temperature and stirring for one hour. The reaction mixture was diluted with water and a 1M aqueous sodium hydroxide solution was added thereto, followed by extraction with ethyl acetate. The resulting aqueous layer was neutralized with 1M hydrochloric acid and extracted with ethyl acetate. The resulting organic layer was washed with water, dried and concentrated under reduced pressure to obtain (2-chloro-3-cyanopyridin-4-yl) boric acid (972 mg).

Preparation Example 29

n-Butyl lithium (1.55M hexane solution, 7.5 mL) was added to a mixture of N,N,N',N'-tetramethylethylenediamine (1.5 g) and diethylether (40 mL) under an argon gas atmosphere at -78°C, followed by stirring at the same temperature for 30 minutes. A mixture of 3,5-difluoropyridine (1.2 g) and diethylether (10 mL) was added slowly to the reaction mixture, followed by stirring at the same temperature for 2 hours. Iodine (4.0 g) was further added to the reaction mixture, followed by stirring at the same temperature for one hour and warming to room temperature. The reaction mixture was diluted with water, the formed solid was separated by filtration, and the filtrate was extracted with diethylether and washed with a saturated aqueous sodium hydrogen carbonate solution. The organic layer was washed with saturated brine, dried over anhydrous magnesium sulfate and concentrated under reduced pressure to obtain 3,5-difluoro-4-iodopyridine (820 mg).

Preparation Example 30

A mixture of methyl 8-(5-bromo-2,3-dihydro-1-benzofuran-7-yl)-2-naphthalene carboxylate (184 mg), triethylamine (97 mg), 10% palladium on carbon (water content of 50%, 100 mg) and methanol (20 mL) was stirred under a hydrogen gas atmosphere of 3 atm at room temperature for 18 hours. The insoluble matter was separated by filtration and the filtrate was diluted with water and extracted with ethyl acetate. The organic layer was washed with water, dried and concentrated under reduced pressure to obtain methyl 8-(2,3-dihydro-1-benzofuran-7-yl)-2-naphthalene carboxylate (144 mg).

Preparation Example 31

A mixture of 5-fluoro-8-methoxy-1-tetralone (5.46 g), sodium hydride (55%, 2.8 g), dimethyl carbonate (10 g) and THF (164 mL) was stirred under refluxing with heating at an oil temperature of 60°C for 3 hours. The reaction mixture

was diluted with an aqueous ammonium chloride solution and extracted with ethyl acetate, and the organic layer was concentrated under reduced pressure. The resulting residue was purified by silica gel column chromatography (hexane/ethyl acetate) to obtain methyl 5-fluoro-8-methoxy-1-oxo-1,2,3,4-tetrahydronaphthalene-2-carboxylate (3.03 g).

Preparation Example 32

A mixture of methyl 5-fluoro-1-hydroxy-8-methoxy-1,2,3,4-tetrahydronaphthalene-2-carboxylate (3.0 g), p-toluenesulfonic acid monohydrate (225 mg) and toluene (30 mL) was stirred with heating at an oil temperature of 80°C for one hour. The reaction mixture was concentrated under reduced pressure, and the resulting residue was diluted with water and extracted with ethyl acetate. The organic layer was concentrated under reduced pressure and the resulting residue was purified by silica gel column chromatography (hexane/ethyl acetate) to obtain methyl 5-fluoro-8-methoxy-3,4-dihydronaphthalene-2-carboxylate (971 mg).

Preparation Example 33

Lithium hexamethyldisilazide (1M hexane solution, 3.3 mL) was added to a mixture of methyl 7-fluoro-8-oxo-5,6,7,8-tetrahydronaphthalene-2-carboxylate (491 mg) and THF (20 mL), followed by stirring at room temperature for one hour, adding ethyl chlorocarbonate (719 mg) thereto and further stirring for one hour. The reaction mixture was diluted with a saturated aqueous ammonium chloride solution and extracted with ethyl acetate, and the organic layer was concentrated under reduced pressure. The resulting residue was purified by silica gel column chromatography (hexane/ethyl acetate) to obtain methyl 8-[(ethoxycarbonyl)oxy]-7-fluoro-5,6-dihydronaphthalene-2-carboxylate (310 mg).

Preparation Example 34

A mixture of methyl 8-{[(trifluoromethyl)sulfonyl]oxy}-2-naphthalene carboxylate (750 mg), 2-chloro-6-fluorophenyl boric acid (600 mg), tetrakis(triphenylphosphine)palladium (1.3 g), triethylamine (581 mg) and 1,4-dioxane (75 mL) was stirred under refluxing with heating at an oil temperature of 95°C for 17 hours. The reaction mixture was cooled to room temperature, the insoluble matter was separated by filtration and the filtrate was concentrated under reduced pressure. The resulting residue was purified by silica gel column chromatography (hexane/ethyl acetate) to obtain methyl 8-(2-chloro-6-fluorophenyl)-2-naphthalene carboxylate (684 mg).

Preparation Example 35

A mixture of methyl 8-(4,4,5,5-tetramethyl-1,3,2-dioxaborolan-2-yl)-2-naphthalene carboxylate (250 mg), 4-bromo-2-methoxypyridine (226 mg), [1,1'-bis(diphenylphosphino)ferrocene]dichloro palladium (II) (29 mg), cesium fluoride (243 mg) and 1,2-dimethoxyethane (15 mL) was stirred under refluxing with heating under an argon atmosphere for one day. The reaction mixture was cooled to room temperature, diluted with water and extracted with ethyl acetate. The organic layer was concentrated under reduced pressure and the resulting residue was purified by silica gel column chromatography (hexane/ethyl acetate) to obtain methyl 8-(2-methoxypyridin-4-yl)-2-naphthalene carboxylate (200 mg).

Preparation Example 36

A mixture of methyl 8-(2,5-dichloropyridin-4-yl)-2-naphthalene carboxylate (161 mg), cyclopropylboric acid (52 mg), palladium (II) acetate (16 mg), potassium triphosphate (360 mg), tricyclohexylphosphoniumtetrafluoroborate (54 mg) and toluene (20 mL) was stirred under refluxing with heating for one day. The reaction mixture was cooled to room temperature, diluted with water and extracted with ethyl acetate. The organic layer was concentrated under reduced pressure and the resulting residue was purified by silica gel column chromatography (hexane/ethyl acetate) to obtain methyl 8-(5-chloro-2-cyclopropylpyridin-4-yl)-2-naphthalene carboxylate (96 mg).

Preparation Example 37

A mixture of methyl 7-chloro-8-{[(trifluoromethyl)sulfonyl]oxy}-2-naphthalene carboxylate (200 mg), 3-fluoropyridin-4-ylboric acid (191 mg), bis(dibenzylideneacetone)palladium (31 mg), 2-dicyclohexylphosphino-2',4',6'-triisopropylbiphenyl (52 mg), potassium triphosphate (345 mg) and n-butanol (7 mL) was stirred with heating at an oil temperature of 100°C under an argon gas atmosphere for 18 hours. The reaction mixture was cooled to room temperature, diluted with water and extracted with ethyl acetate. The organic layer was concentrated under reduced pressure and the resulting residue was purified by silica gel column chromatography (hexane/ethyl acetate) to obtain butyl 7-chloro-8-(3-fluoropyridin-4-yl)-2-naphthalene carboxylate (83 mg).

Preparation Example 38

A mixture of methyl 8- {[(trifluoromethyl)sulfonyl]oxy} -2-naphthalene carboxylate (300 mg), pyridin-4-yl boric acid (276 mg), tetrakis(triphenylphosphine) palladium (104 mg), sodium carbonate (380 mg), water (2 mL), ethanol (1 mL) and 1,2-dimethoxyethane (10 mL) was stirred with heating at an oil temperature of 100°C for 18 hours. The reaction mixture was cooled to room temperature, diluted with water and extracted with ethyl acetate. The organic layer was concentrated under reduced pressure and the resulting residue was purified by silica gel column chromatography (hexane/ethyl acetate) to obtain methyl 8-pyridin-4-yl-2-naphthalene carboxylate (165 mg).
The compounds of Preparation Examples shown in Tables 1 to 38 below were prepared using the corresponding starting materials in the same manner as the above shown Preparation Examples 1 to 38. In addition, physical data for the compounds of Preparation Examples are shown in Tables 39 and 46.

Example 1

A mixture of 8-(2-chloro-6-fluorophenyl)-2-naphthalenecarbonic acid (298 mg), CDI (250 mg), and DMF (10 mL) was stirred under heating at an oil temperature of 60°C for 30 minutes, the reaction mixture was cooled to room temperature, and guanidine carbonate (450 mg) was added thereto, followed by further stirring at room temperature for 21 hours. The reaction mixture was diluted with water and the precipitate was collected by filtration. This was recrystallized with ethyl acetate and further treated with a 4M hydrogen chloride/ethyl acetate solution to obtain 8-(2-chloro-6-fluorophenyl)-N-(diaminomethylene)-2-naphthamide hydrochloride (185 mg).
The compounds of the Examples shown in the Tables 47 to 64 below were prepared using the corresponding starting materials in the same manner as in Example 1 above. The physical data for the compounds of Examples are shown in Tables 65 to 69.
The following abbreviations are used in the tables below.

PEx: Preparation Example number, Ex: Example number, Str: structural formula, Dat: physical data (ESI+: ESI-MS $[M+H]^+$; ESI-:ESI-MS$[M-H]^-$; FAB+: FAB-MS$[M+H]^+$ or FAB-MS$[M]^+$; FAB-: FAB-MS$[M-H]^-$; APCI+: APCI-MS$[M+H]^+$; APCI-:APCI-MS$[M-H]^-$; $EI^+$: EI[M]+; A/E+: simultaneous measurement of APCI and ESI (cations); A/E-: simultaneous measurement of APCI and ESI (anions); NMR: δ(ppm) of peaks by $^1$HNMR in $CDCl_3$ or DMSO-$d_6$); Sal: salt (blank or no description represents a free form, and the numeral present before the acidic ingredient represents a molar ratio; for example, the case in which 2HCl is described shows that the compound is dihydrochloride); Me: methyl; Et: ethyl, nBu: butyl, Ph: phenyl, Tf: trifluoromethanesulfonyl, Fum: fumaric acid, RSyn: production process (the numeral shows that, in the same manner as in the compound having the number as its Preparation Example number, the compound was produced using the corresponding starting material). In the formulae, in the case of a compound in which a bond is represented by two cross lines, it is shown that the bond is a double bond and its geometrical arrangement is unknown.

[Table 1]

| REx | Sal | Str |
|---|---|---|
| 1 | | 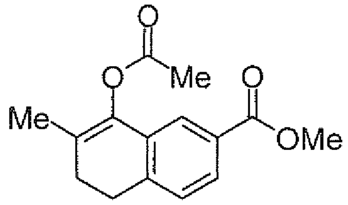 |
| 2 | | 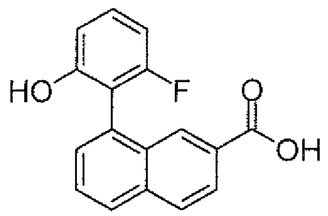 |
| 3 | | 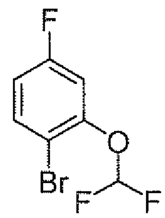 |
| 4 | | 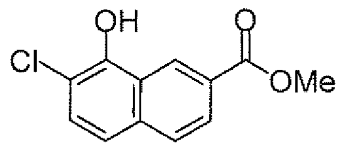 |
| 5 | | 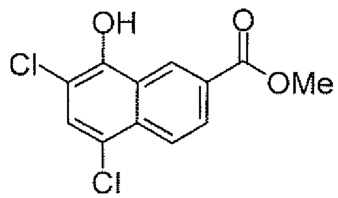 |
| 6 | | 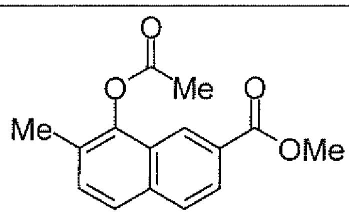 |
| 7 | | 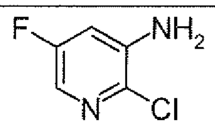 |
| 8 | | 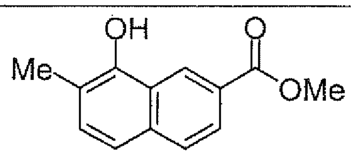 |
| 9 | | 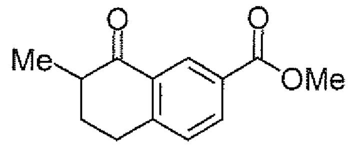 |
| 10 | | 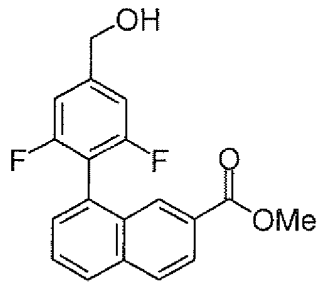 |
| 11 | | 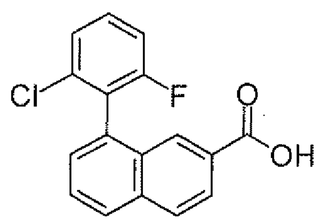 |
| 12 | | 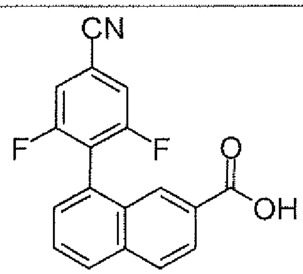 |
| 13 | | 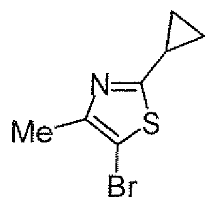 |
| 14 | | 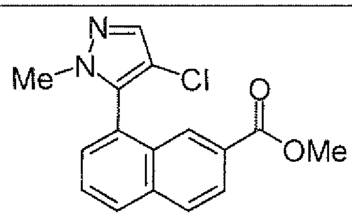 |
| 15 | | 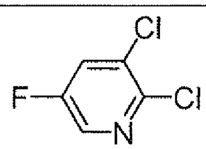 |

[Table 2]

| REx | Sal | Str |
|---|---|---|
| 16 | | 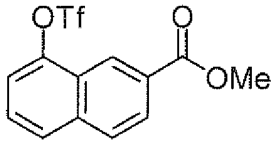 |
| 17 | | 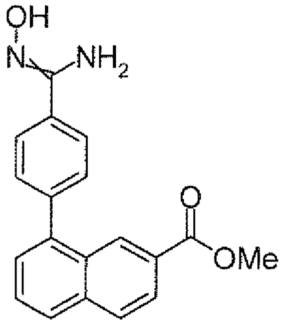 |
| 18 | | 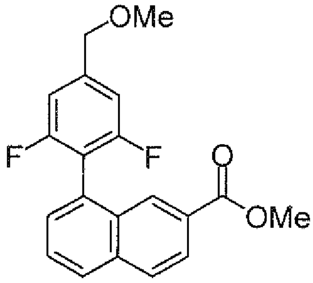 |
| 19 | | 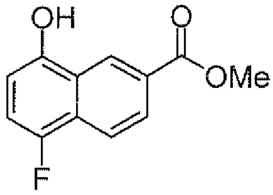 |
| 20 | | 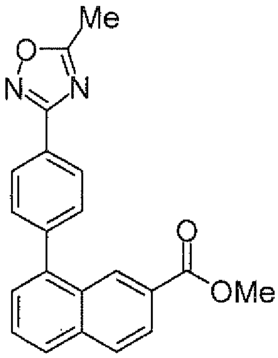 |
| 21 | | 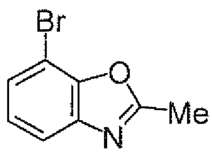 |
| 22 | | 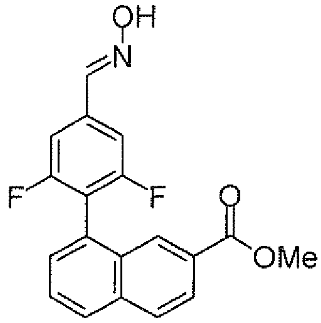 |
| 23 | | 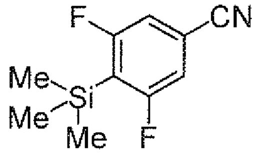 |
| 24 | | 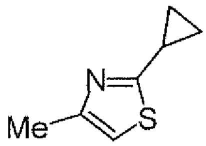 |
| 25 | | 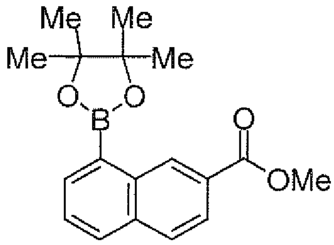 |
| 26 | | 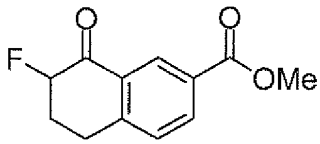 |
| 27 | | 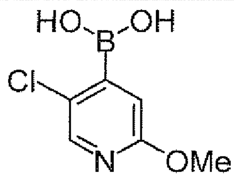 |
| 28 | | 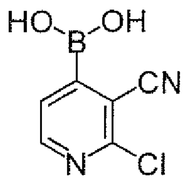 |

[Table 3]

| REx | Sal | Str |
|---|---|---|
| 29 | | 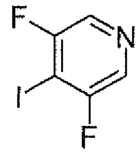 |
| 30 | | 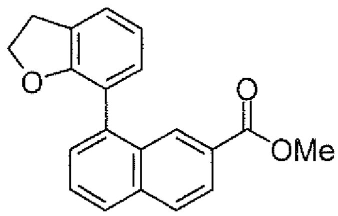 |
| 31 | | 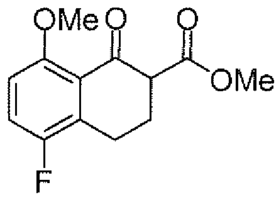 |
| 32 | | 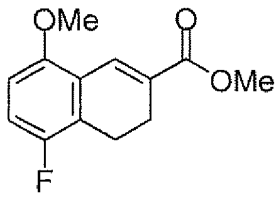 |
| 33 | | 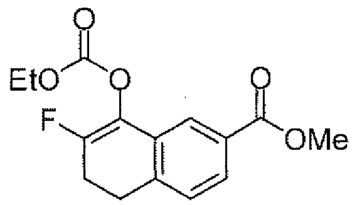 |
| 34 | | 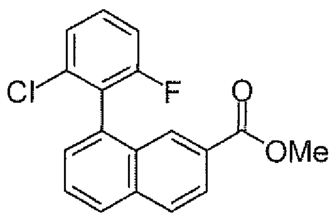 |
| 35 | | 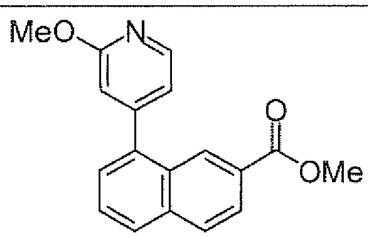 |
| 36 | | 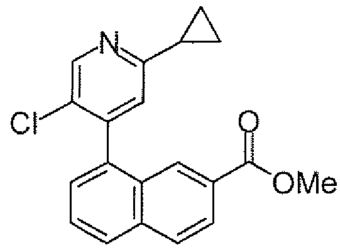 |
| 37 | | 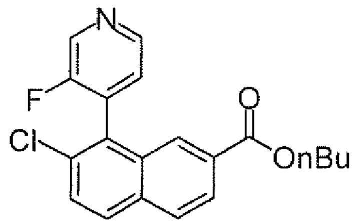 |
| 38 | | 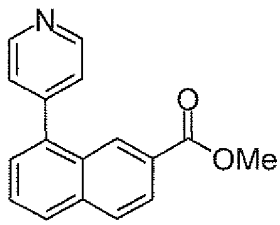 |
| 39 | | 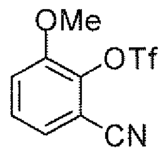 |
| 40 | | 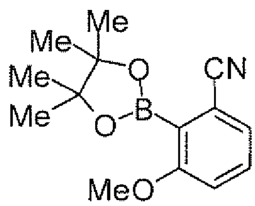 |
| 41 | | 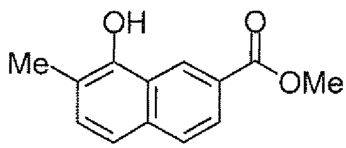 |
| 42 | | 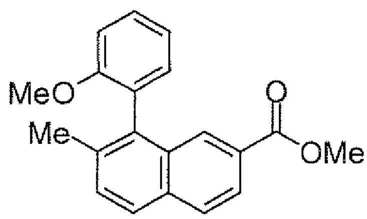 |

[Table 4]

| REx | Sal | Str |
|---|---|---|
| 43 | | 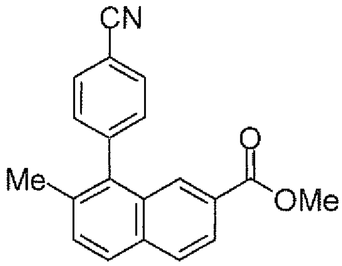 |
| 44 | | 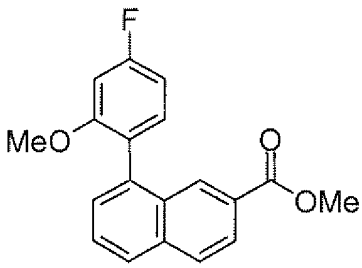 |
| 45 | | 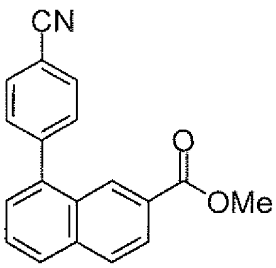 |
| 46 | | 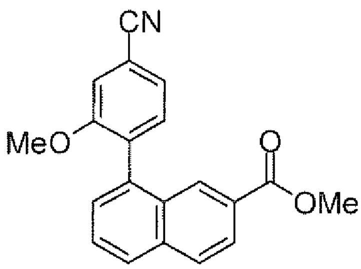 |
| 47 | | 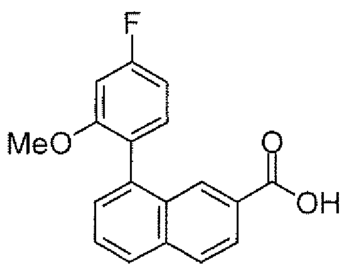 |

| REx | Sal | Str |
|---|---|---|
| 48 | | 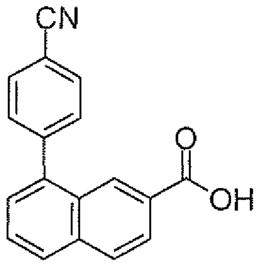 |
| 49 | | 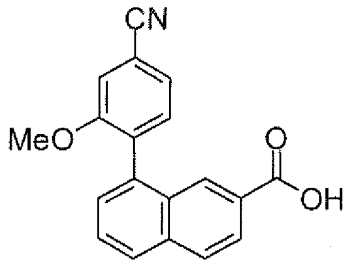 |
| 50 | | 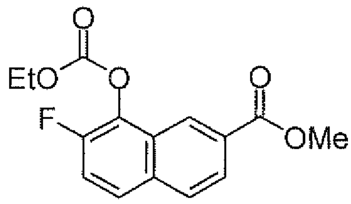 |
| 51 | | 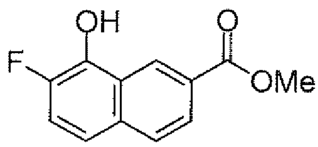 |
| 52 | | 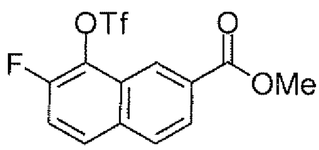 |
| 53 | | 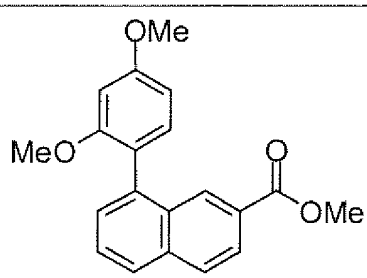 |

[Table 5]

| REx | Sal | Str |
|---|---|---|
| 54 | | 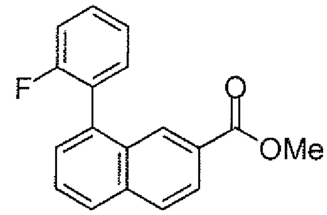 |
| 55 | | 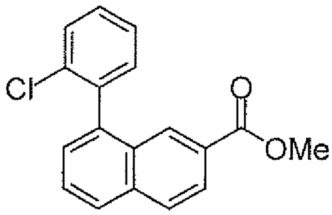 |
| 56 | | 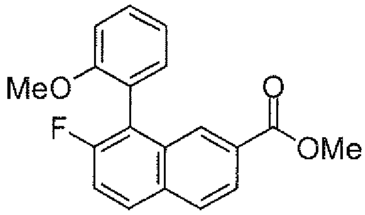 |
| 57 | | 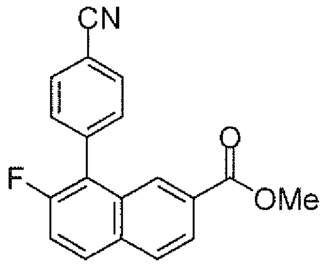 |
| 58 | | 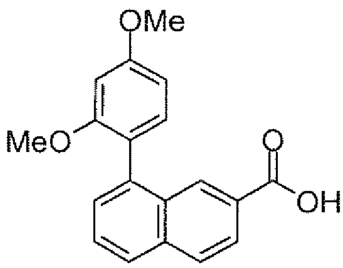 |
| 59 | | 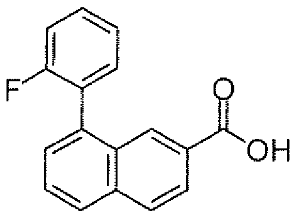 |
| 60 | | 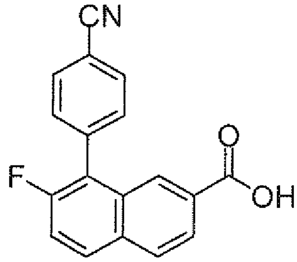 |
| 61 | | 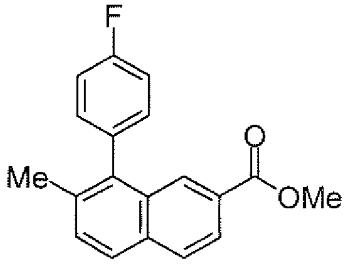 |
| 62 | | 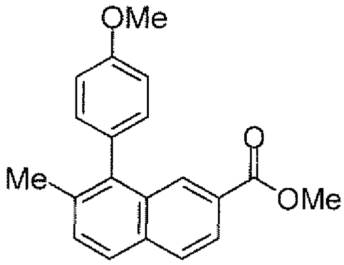 |
| 63 | | 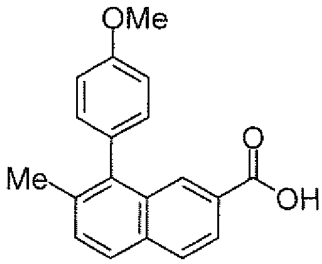 |
| 64 | | 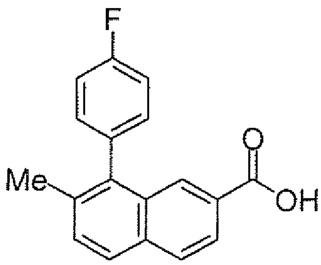 |

[Table 6]

| REx | Sal | Str |
|---|---|---|
| 65 | HCl | 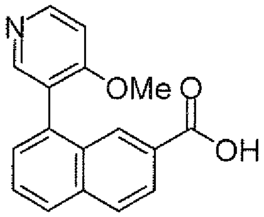 |
| 66 | | 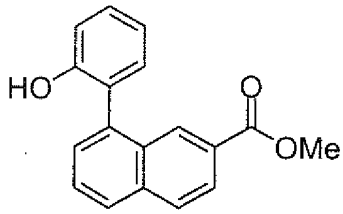 |
| 67 | | 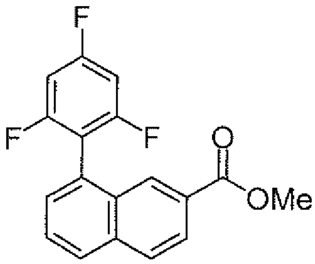 |
| 68 | | 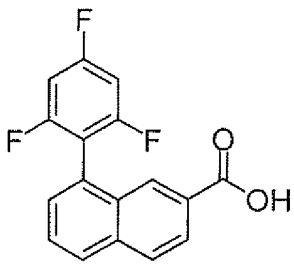 |
| 69 | | 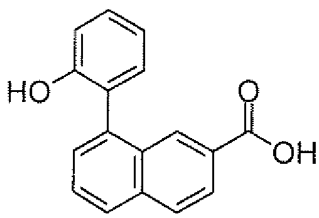 |
| 70 | | 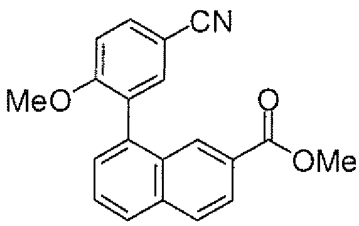 |
| 71 | | 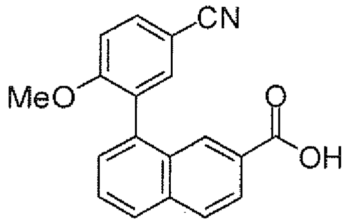 |
| 72 | | 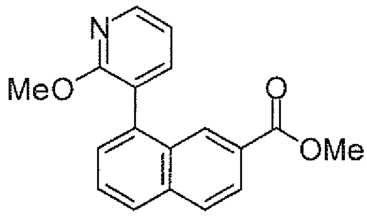 |
| 73 | | 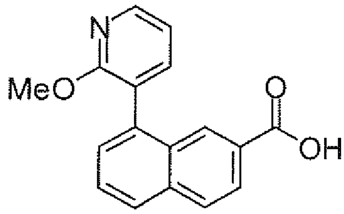 |
| 74 | | 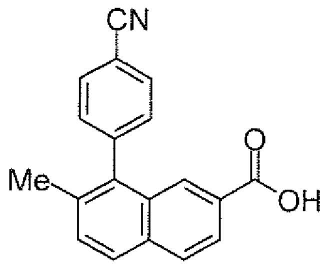 |
| 75 | | 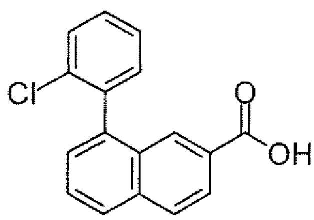 |
| 76 | | 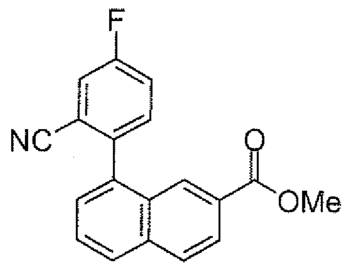 |

[Table 7]

| REx | Sal | Str |
|---|---|---|
| 77 | | 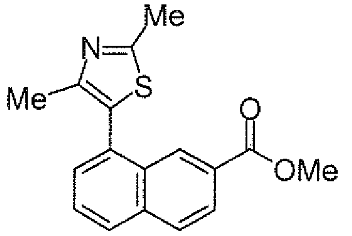 |
| 78 | | 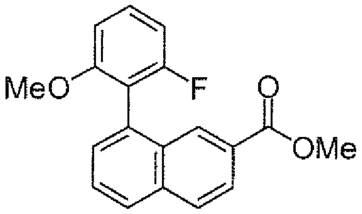 |
| 79 | | 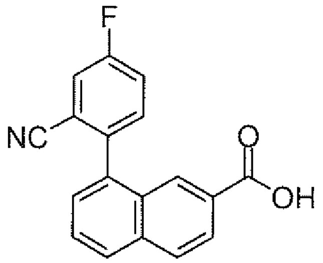 |
| 80 | | 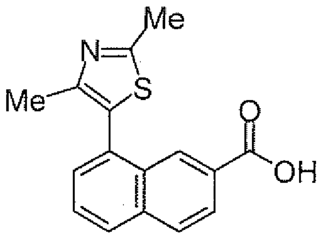 |
| 81 | | 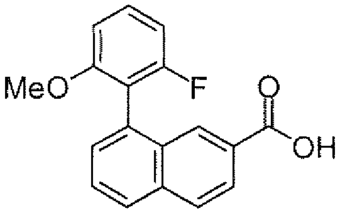 |
| 82 | | 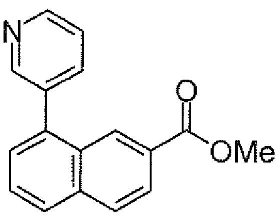 |
| 83 | | 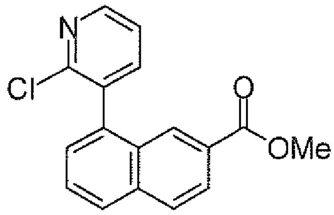 |
| 84 | | 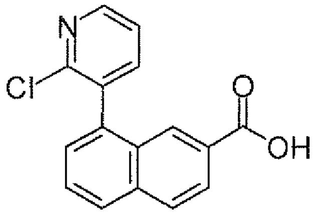 |
| 85 | | 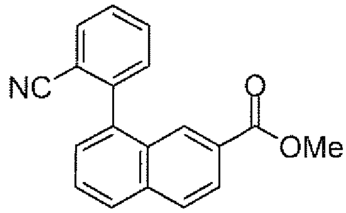 |
| 86 | | 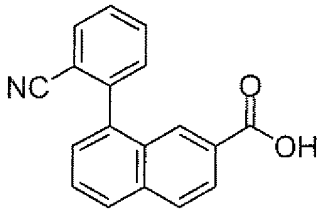 |
| 87 | | 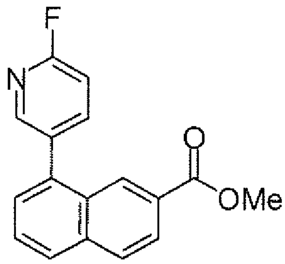 |
| 88 | | 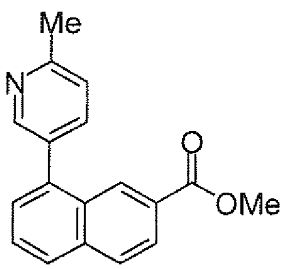 |

[Table 8]

| REx | Sal | Str |
|---|---|---|
| 89 | | 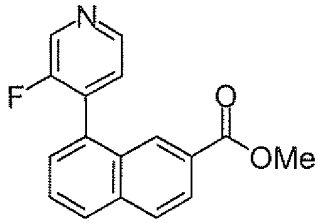 |
| 90 | HCl | 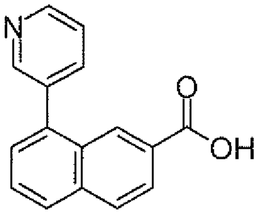 |
| 91 | HCl | 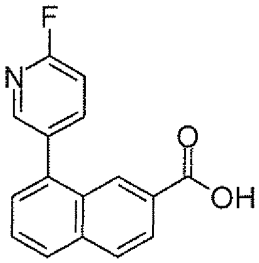 |
| 92 | HCl | 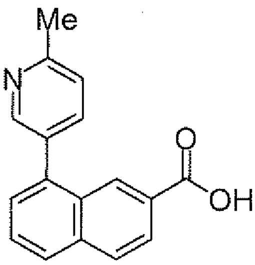 |
| 93 | HCl | 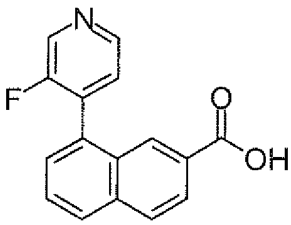 |
| 94 | | 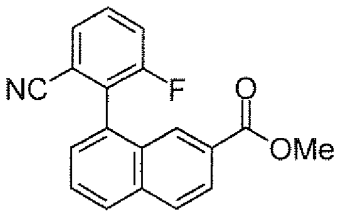 |
| 95 | | 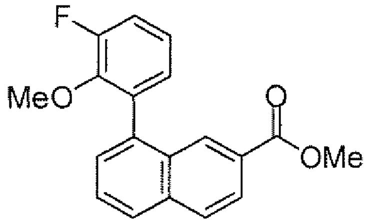 |
| 96 | | 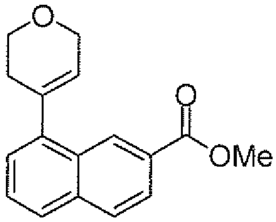 |
| 97 | | 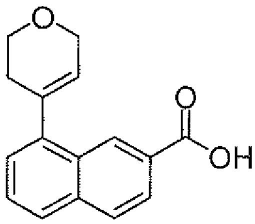 |
| 98 | | 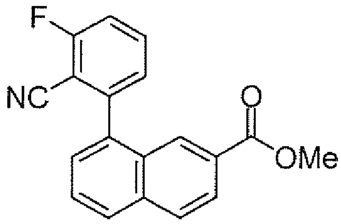 |
| 99 | | 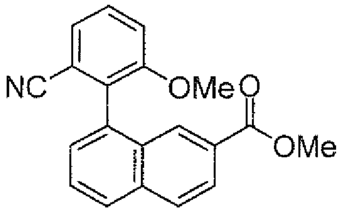 |
| 100 | | 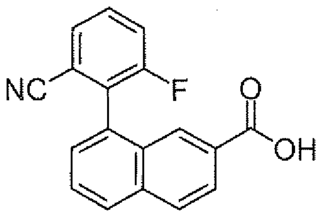 |

[Table 9]

| REx | Sal | Str |
|---|---|---|
| 101 | | 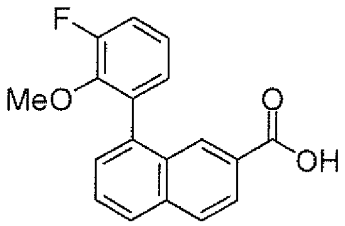 |
| 102 | | 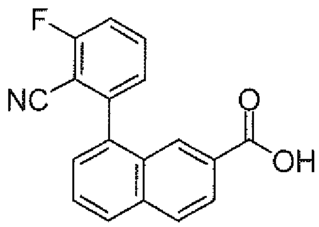 |
| 103 | | 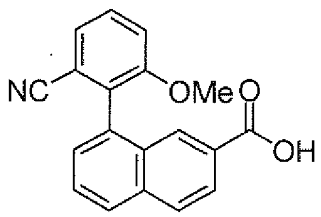 |
| 104 | | 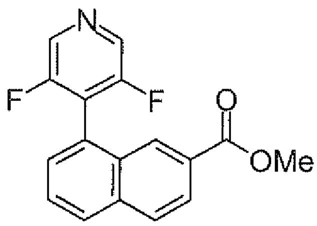 |
| 105 | HCl | 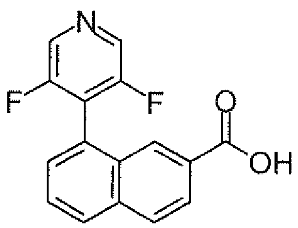 |
| 106 | | 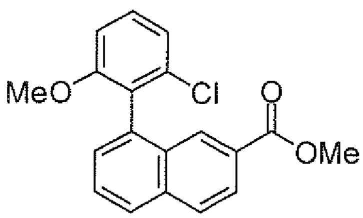 |
| 107 | | 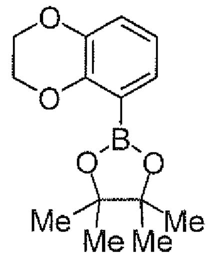 |
| 108 | | 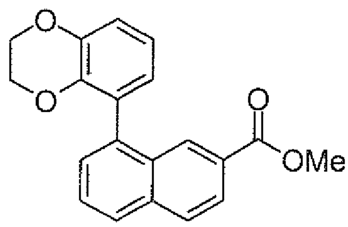 |
| 109 | | 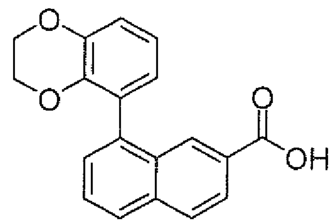 |
| 110 | | 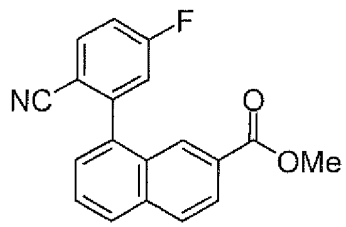 |
| 111 | | 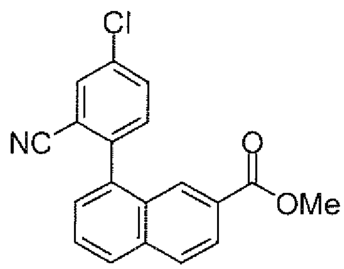 |
| 112 | | 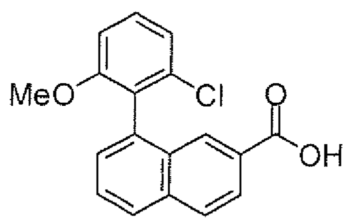 |

[Table 10]

| REx | Sal | Str |
|---|---|---|
| 113 | | 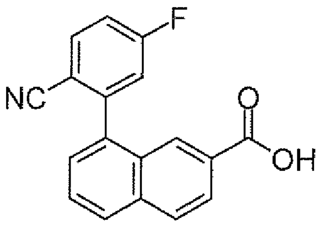 |
| 114 | | 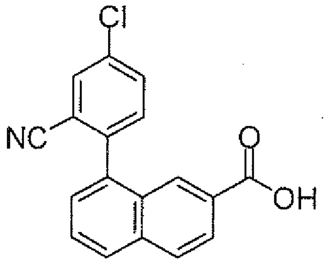 |
| 115 | | 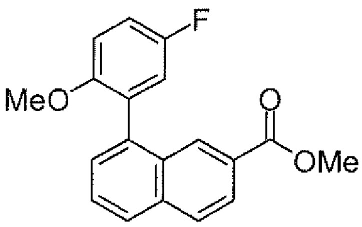 |
| 116 | | 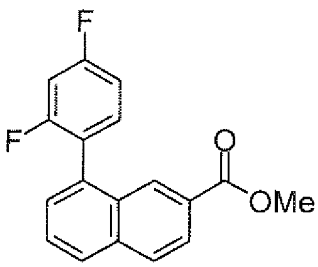 |
| 117 | | 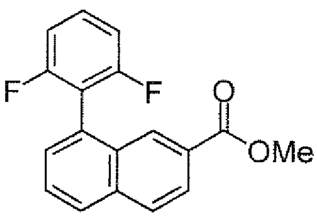 |
| 118 | | 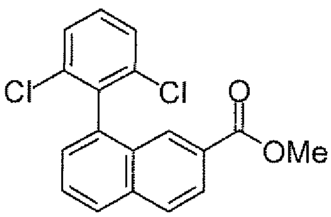 |
| 119 | | 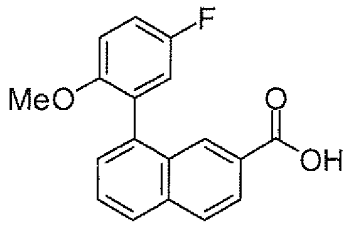 |
| 120 | | 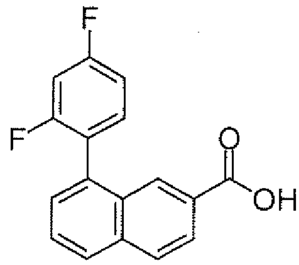 |
| 121 | | 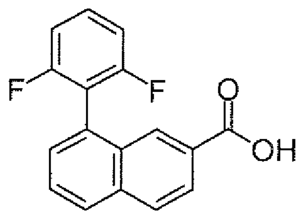 |
| 122 | | 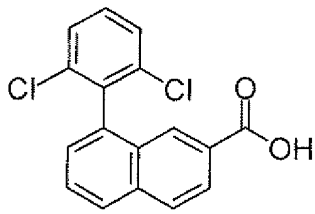 |
| 123 | | 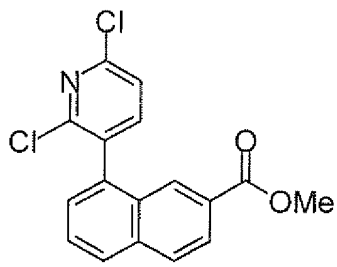 |
| 124 | | 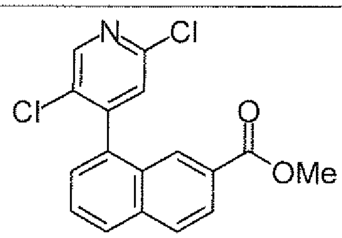 |

[Table 11]

| REx | Sal | Str |
|---|---|---|
| 125 | | 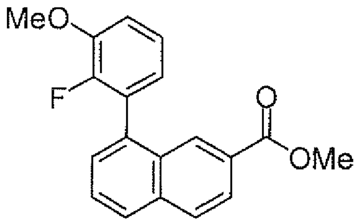 |
| 126 | | 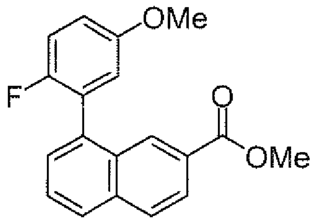 |
| 127 | | 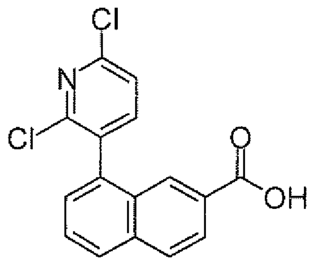 |
| 128 | | 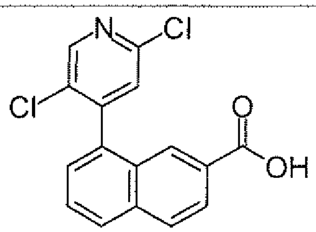 |
| 129 | | 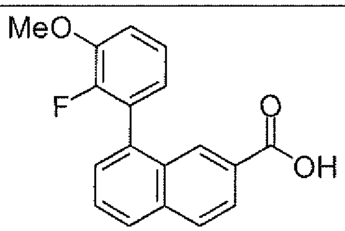 |
| 130 | | 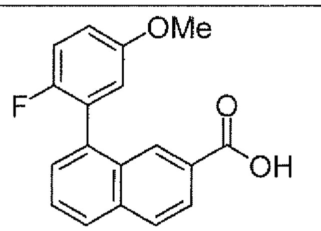 |
| 131 | | 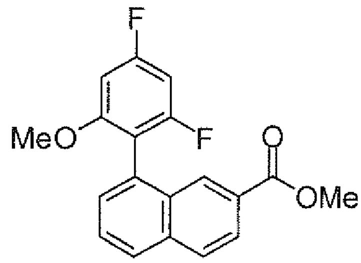 |
| 132 | | 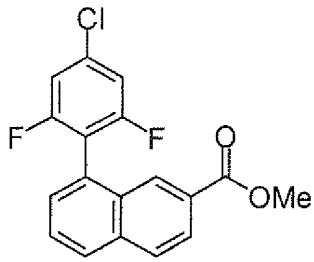 |
| 133 | | 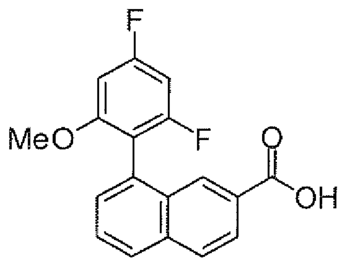 |
| 134 | | 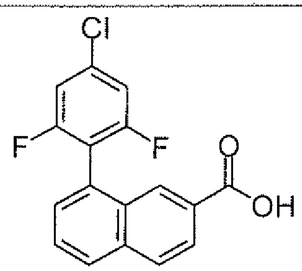 |
| 135 | | 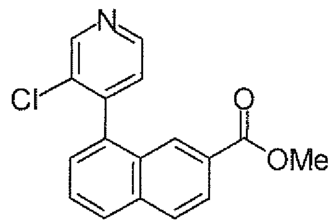 |
| 136 | | 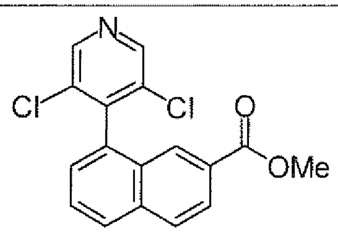 |

[Table 12]

| REx | Sal | Str |
|---|---|---|
| 137 | | 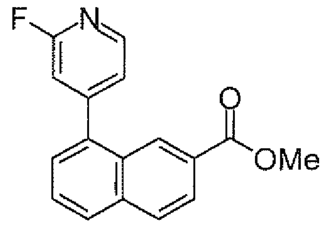 |
| 138 | | 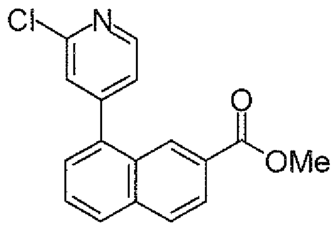 |
| 139 | HCl | 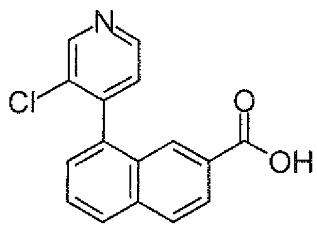 |
| 140 | HCl | 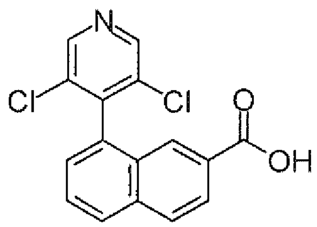 |
| 141 | | 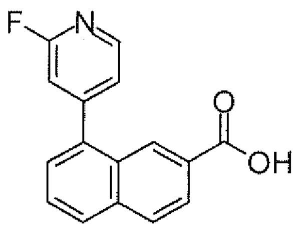 |
| 142 | | 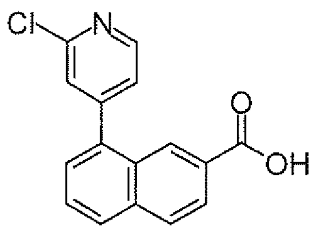 |
| 143 | | 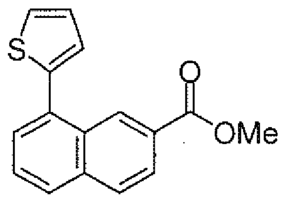 |
| 144 | | 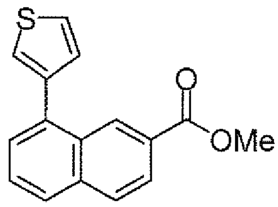 |
| 145 | | 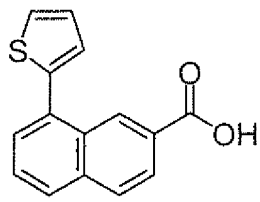 |
| 146 | | 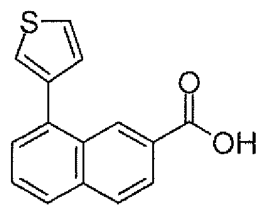 |
| 147 | | 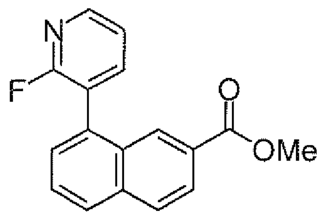 |
| 148 | | 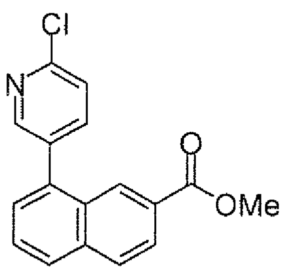 |

[Table 13]

| REx | Sal | Str |
|---|---|---|
| 149 | | 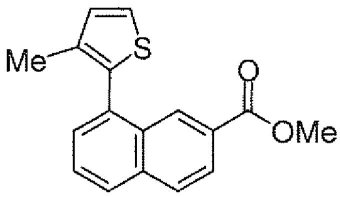 |
| 150 | | 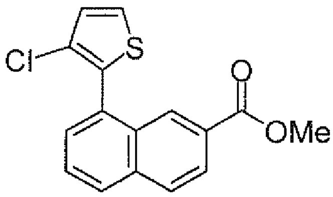 |
| 151 | | 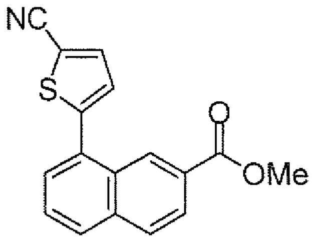 |
| 152 | | 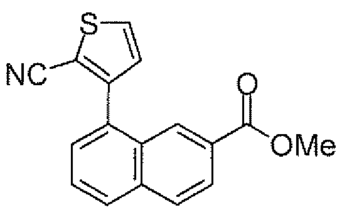 |
| 153 | | 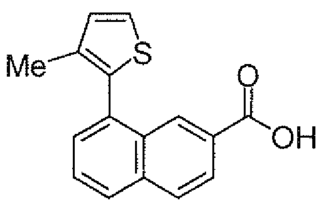 |
| 154 | | 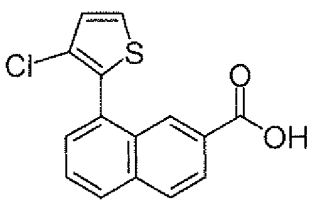 |
| 155 | | 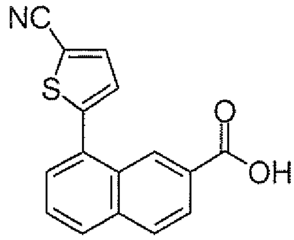 |
| 156 | | 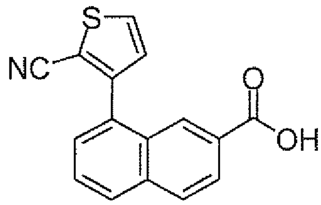 |
| 157 | | 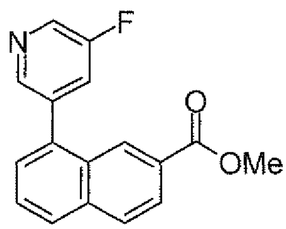 |
| 158 | | 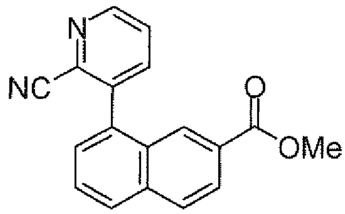 |
| 159 | HCl | 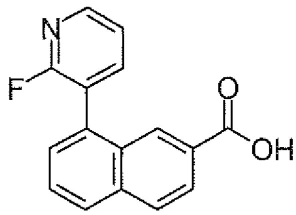 |
| 160 | HCl | 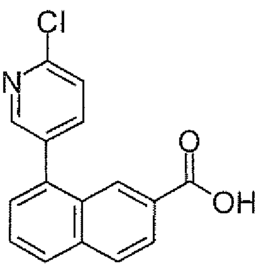 |

[Table 14]

| REx | Sal | Str |
|---|---|---|
| 161 | HCl | 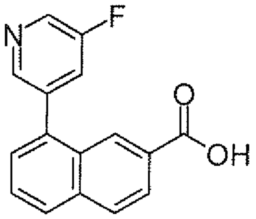 |
| 162 | | 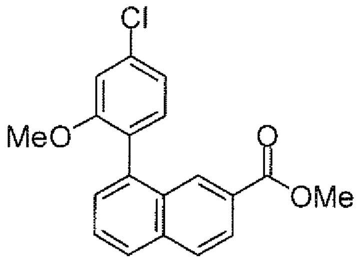 |
| 163 | | 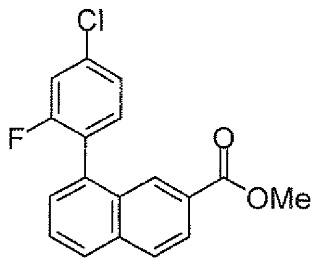 |
| 164 | | 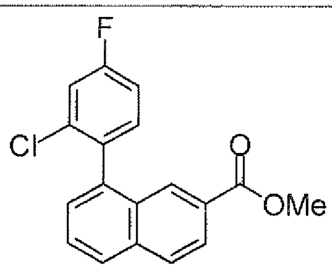 |
| 165 | | 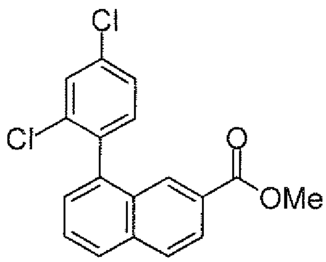 |
| 166 | HCl | 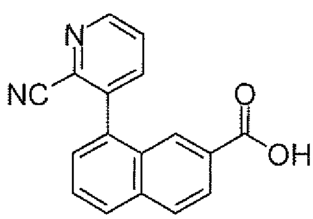 |

| REx | Sal | Str |
|---|---|---|
| 167 | | 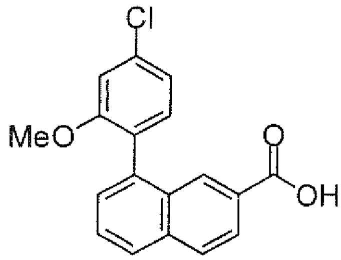 |
| 168 | | 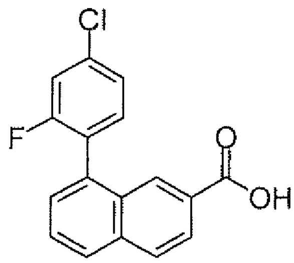 |
| 169 | | 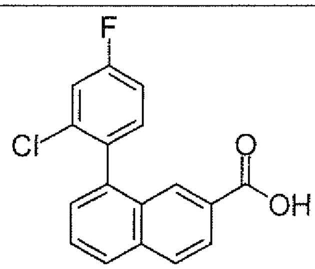 |
| 170 | | 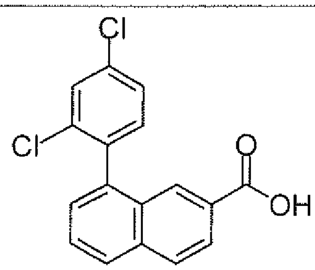 |
| 171 | | 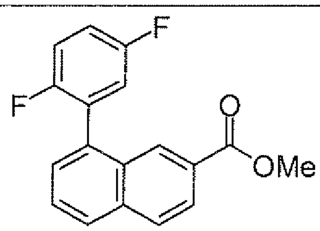 |
| 172 | | 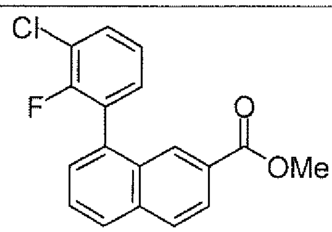 |

[Table 15]

| REx | Sal | Str |
|---|---|---|
| 173 | | 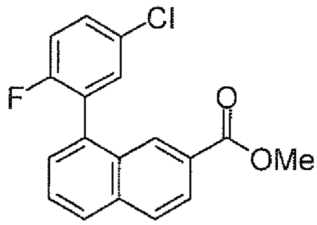 |
| 174 | | 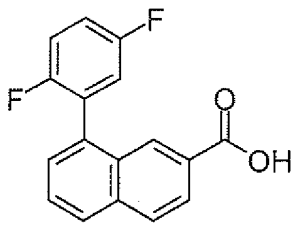 |
| 175 | | 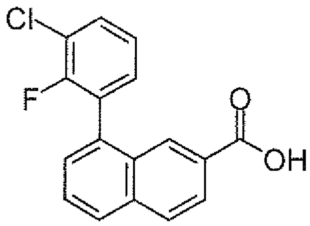 |
| 176 | | 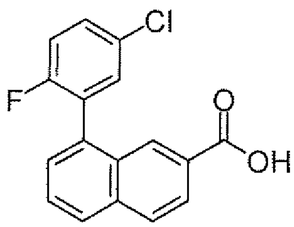 |
| 177 | | 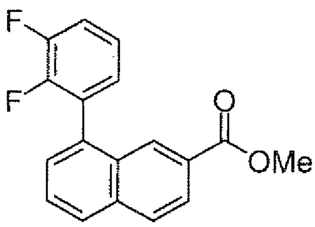 |
| 178 | | 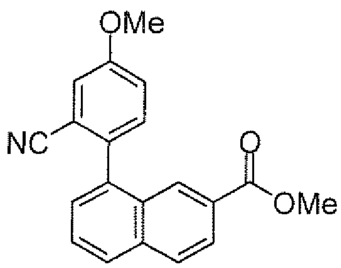 |

| REx | Sal | Str |
|---|---|---|
| 179 | | 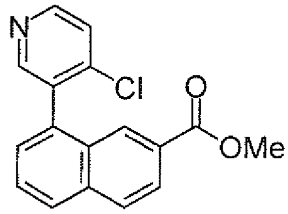 |
| 180 | | 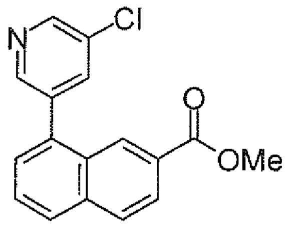 |
| 181 | | 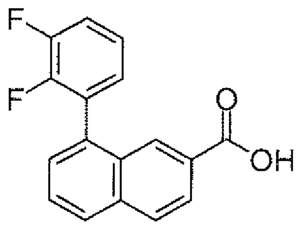 |
| 182 | | 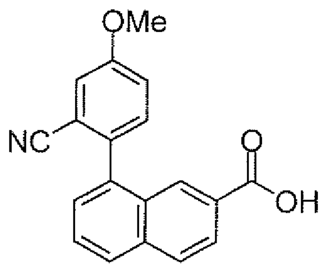 |
| 183 | HCl | 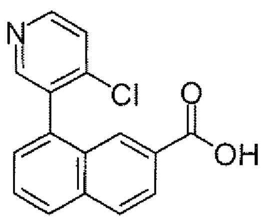 |
| 184 | | 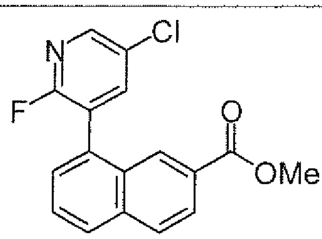 |

[Table 16]

| REx | Sal | Str |
|---|---|---|
| 185 | | 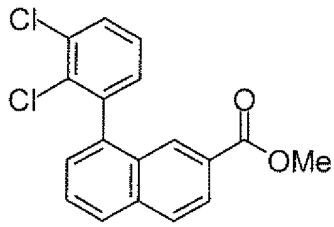 |
| 186 | | 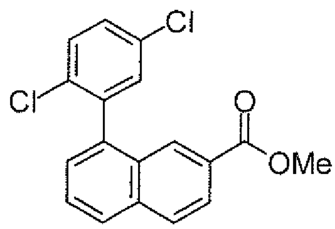 |
| 187 | HCl | 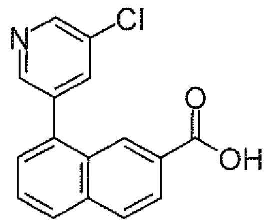 |
| 188 | HCl | 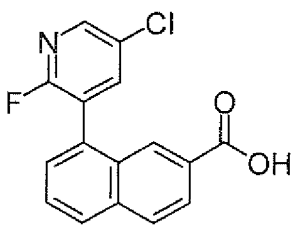 |
| 189 | | 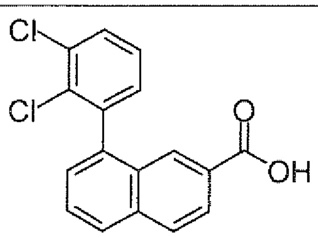 |
| 190 | | 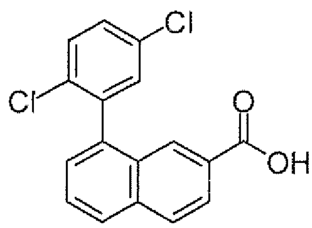 |

| REx | Sal | Str |
|---|---|---|
| 191 | | 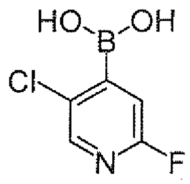 |
| 192 | | 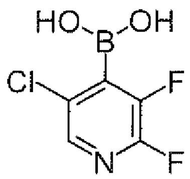 |
| 193 | | 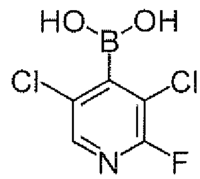 |
| 194 | | 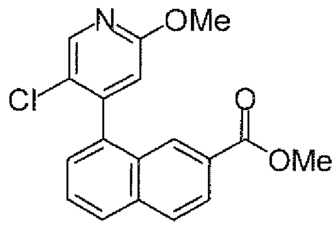 |
| 195 | | 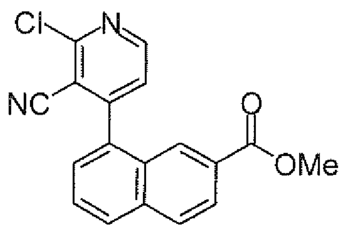 |
| 196 | | 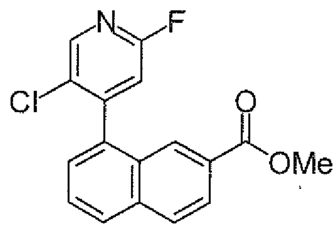 |
| 197 | | 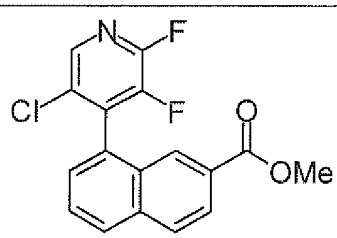 |

[Table 17]

| REx | Sal | Str | REx | Sal | Str |
|---|---|---|---|---|---|
| 198 | | 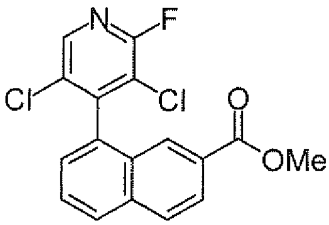 | 204 | | 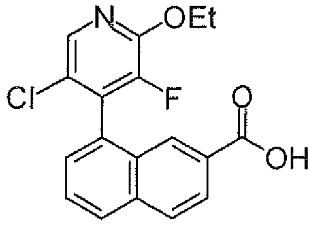 |
| 199 | | 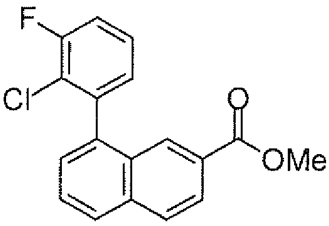 | 205 | | 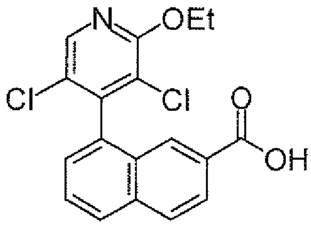 |
| 200 | | 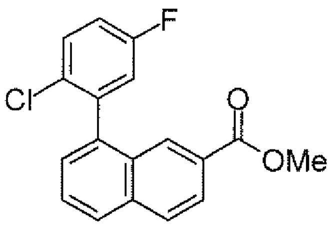 | 206 | | 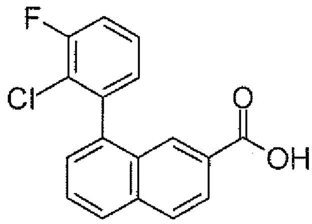 |
| 201 | | 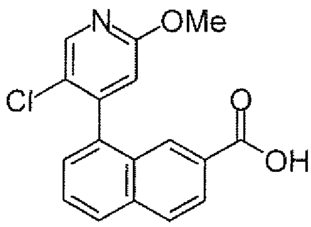 | 207 | | 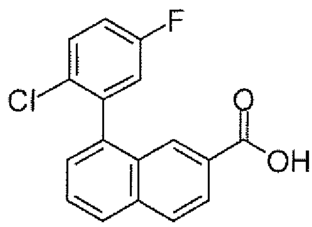 |
| 202 | | 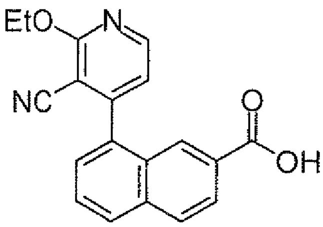 | 208 | | 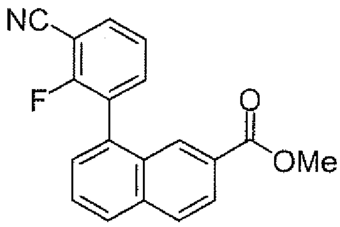 |
| 203 | | 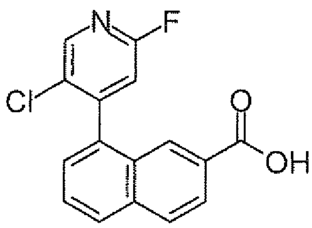 | 209 | | 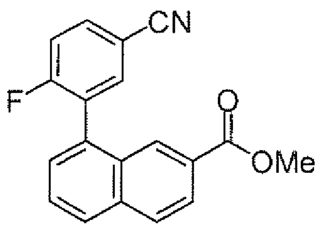 |

[Table 18]

| REx | Sal | Str |
|---|---|---|
| 210 | | 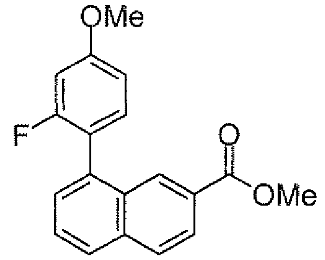 |
| 211 | | 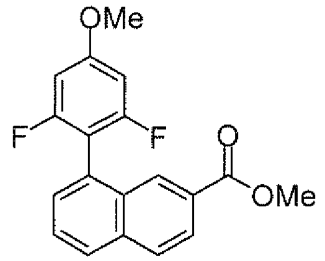 |
| 212 | | 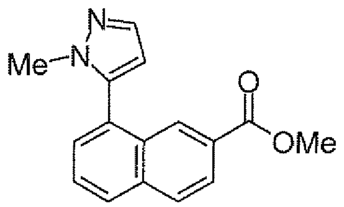 |
| 213 | | 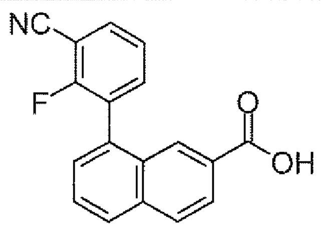 |
| 214 | | 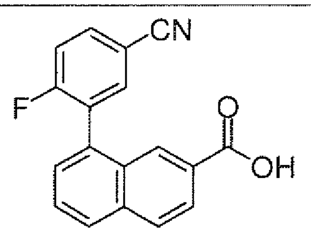 |
| 215 | | 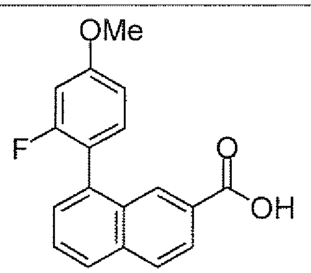 |
| 216 | | 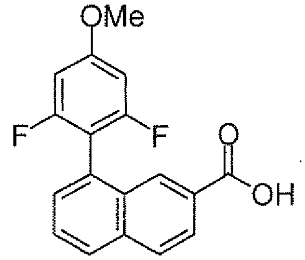 |
| 217 | | 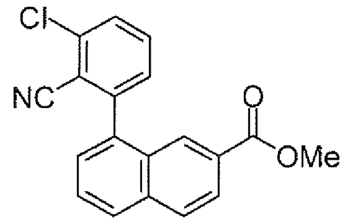 |
| 218 | | 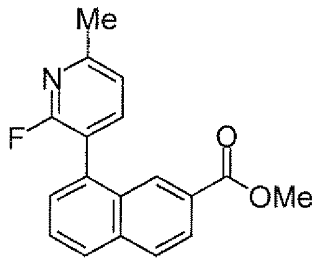 |
| 219 | | 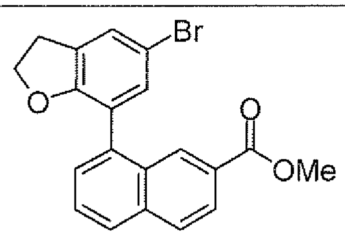 |
| 220 | | 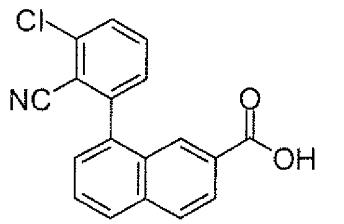 |
| 221 | | 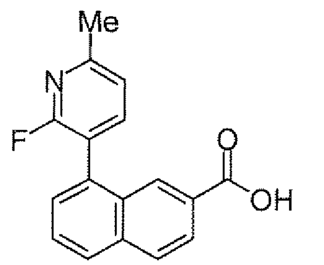 |

[Table 19]

| REx | Sal | Str |
|---|---|---|
| 222 | | 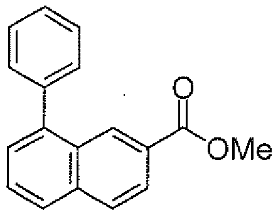 |
| 223 | | 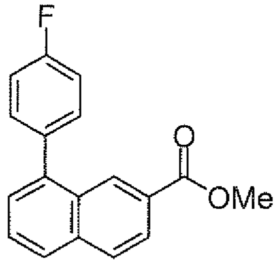 |
| 224 | | 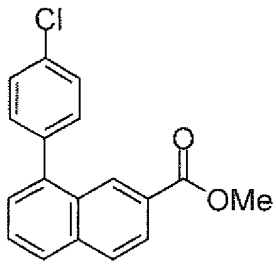 |
| 225 | | 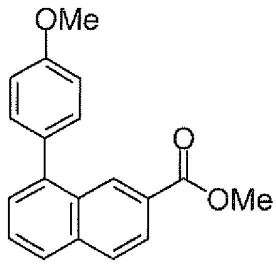 |
| 226 | | 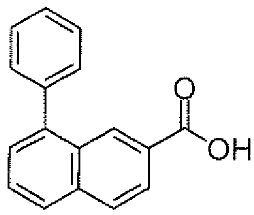 |
| 227 | | 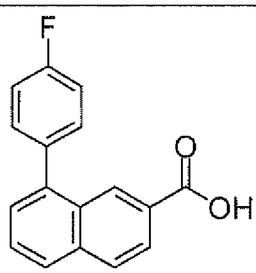 |
| 228 | | 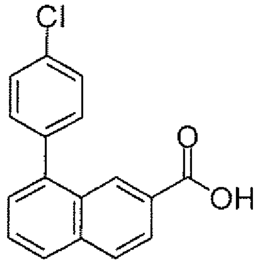 |
| 229 | | 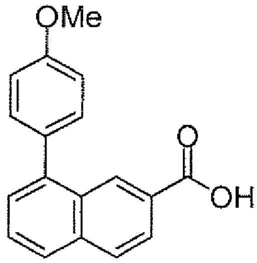 |
| 230 | | 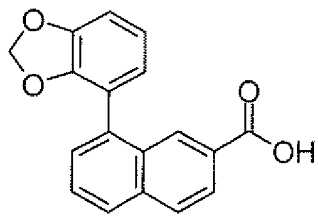 |
| 231 | | 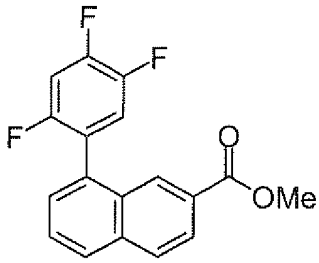 |
| 232 | | 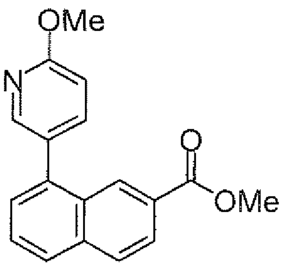 |
| 233 | | 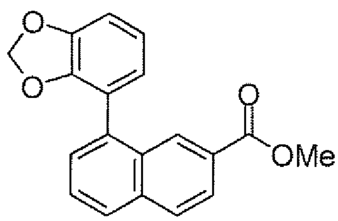 |

[Table 20]

| REx | Sal | Str |
|---|---|---|
| 234 | | 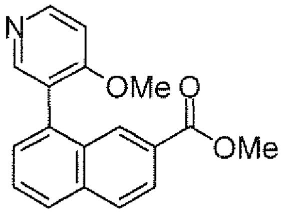 |
| 235 | | 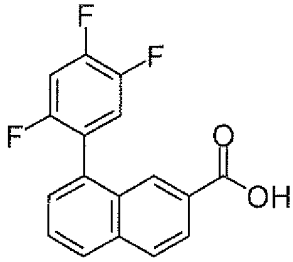 |
| 236 | HCl | 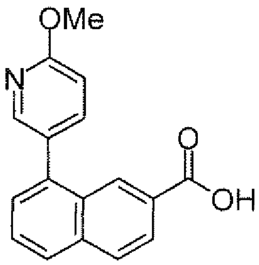 |
| 237 | | 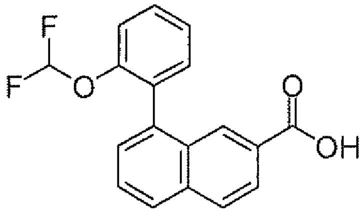 |
| 238 | | 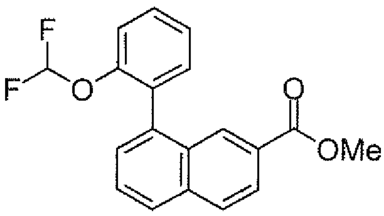 |
| 239 | | 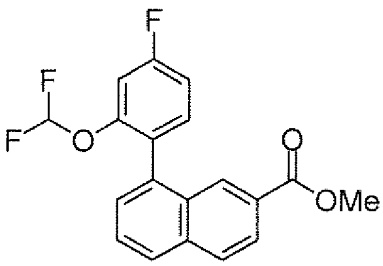 |

| REx | Sal | Str |
|---|---|---|
| 240 | | 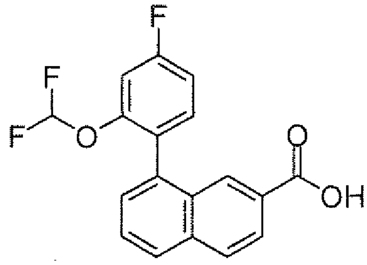 |
| 241 | | 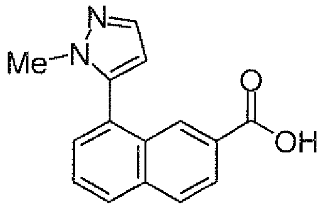 |
| 242 | | 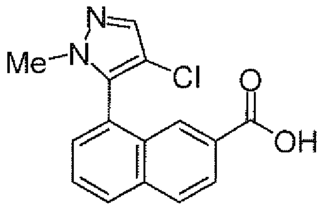 |
| 243 | | 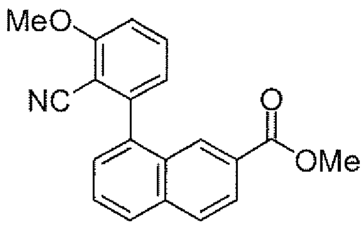 |
| 244 | | 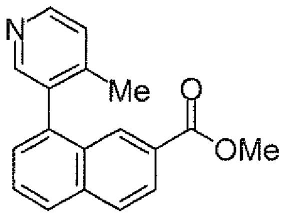 |
| 245 | | 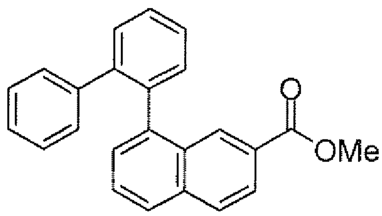 |

[Table 21]

| REx | Sal | Str |
|---|---|---|
| 246 | | 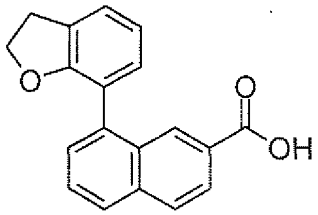 |
| 247 | | 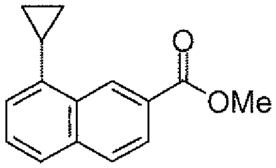 |
| 248 | | 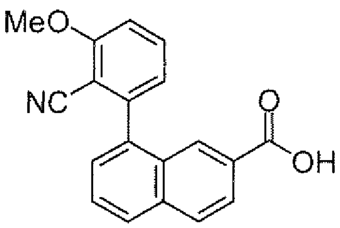 |
| 249 | HCl | 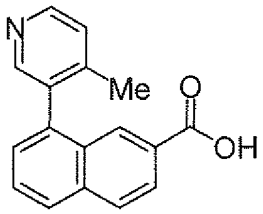 |
| 250 | | 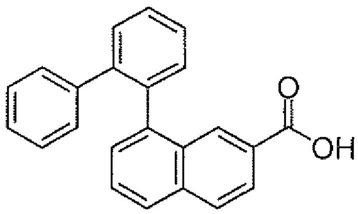 |
| 251 | | 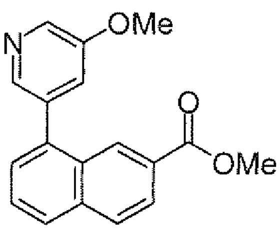 |
| 252 | | 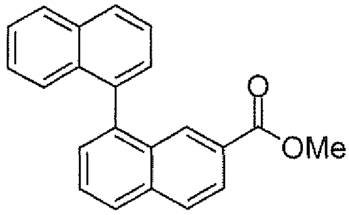 |
| 253 | | 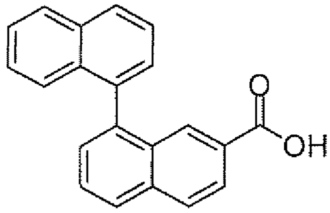 |
| 254 | | 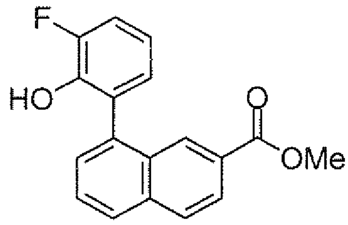 |
| 255 | | 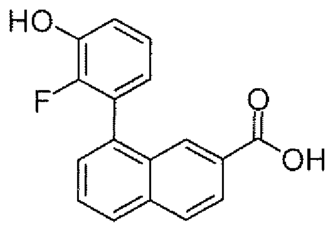 |
| 256 | | 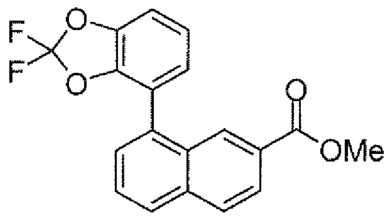 |
| 257 | | 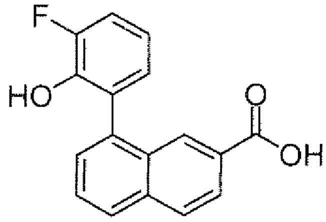 |

[Table 22]

| REx | Sal | Str |
|---|---|---|
| 258 | | 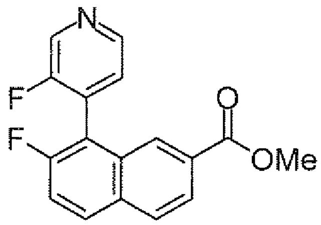 |
| 259 | | 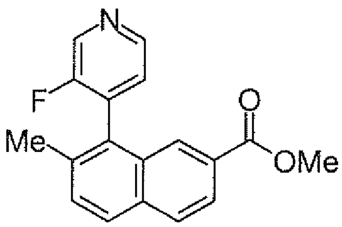 |
| 260 | HCl | 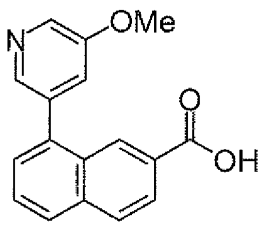 |
| 261 | HCl | 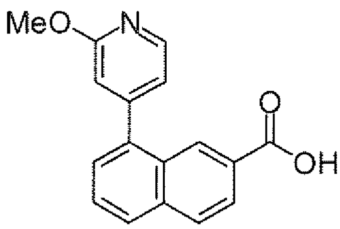 |
| 262 | HCl | 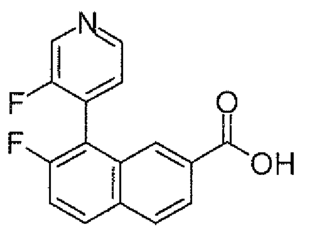 |
| 263 | HCl | 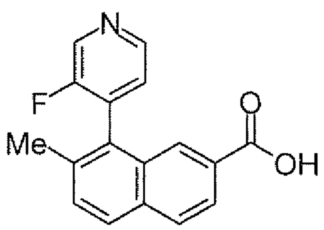 |

| REx | Sal | Str |
|---|---|---|
| 264 | | 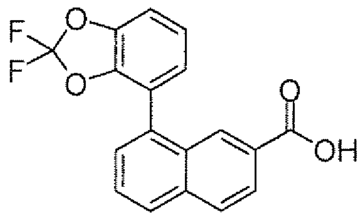 |
| 265 | | 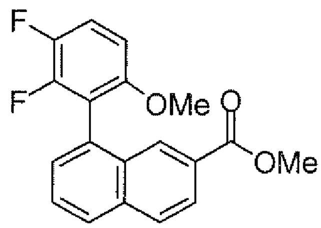 |
| 266 | | 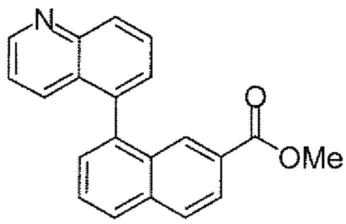 |
| 267 | | 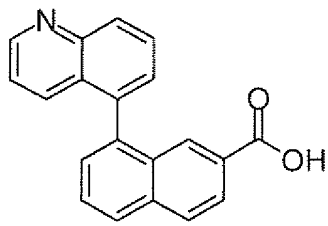 |
| 268 | | 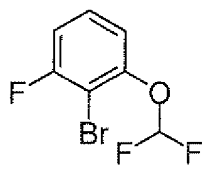 |
| 269 | | 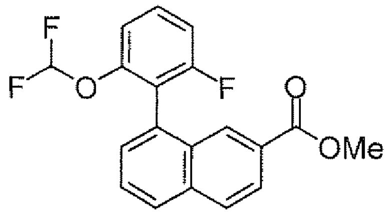 |

[Table 23]

| REx | Sal | Str |
|---|---|---|
| 270 | | 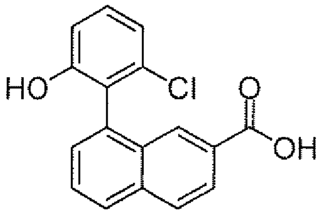 |
| 271 | | 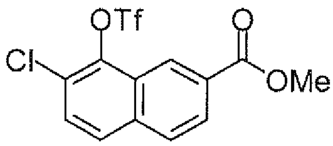 |
| 272 | | 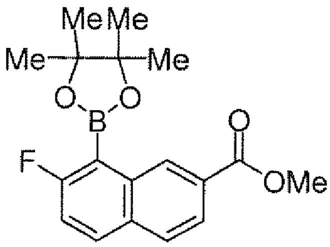 |
| 273 | | 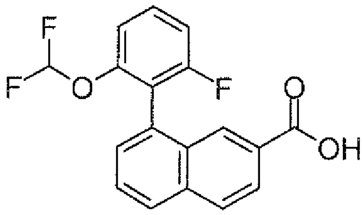 |
| 274 | | 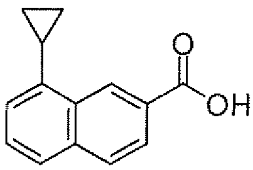 |
| 275 | | 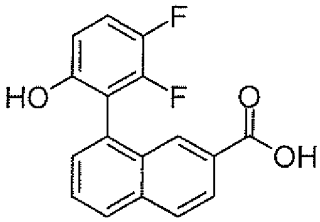 |
| 276 | | 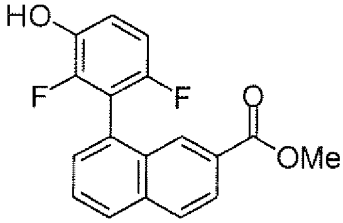 |
| 277 | | 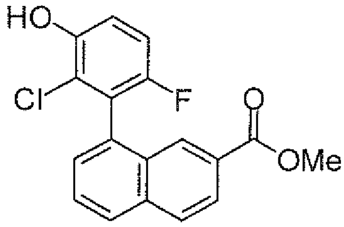 |
| 278 | | 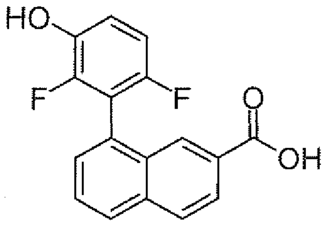 |
| 279 | | 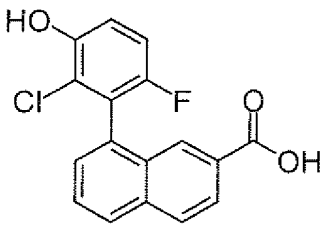 |
| 280 | | 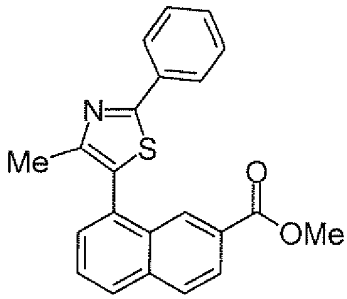 |

[Table 24]

| REx | Sal | Str |
|---|---|---|
| 281 | | 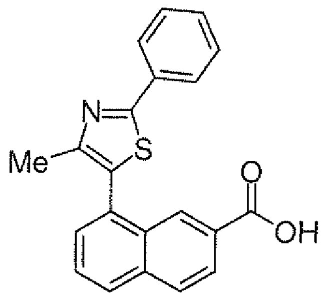 |
| 282 | | 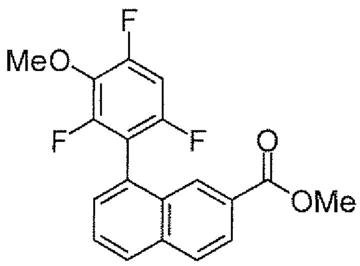 |
| 283 | | 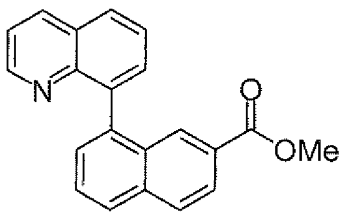 |
| 284 | | 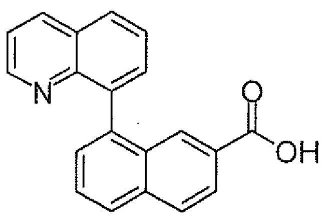 |
| 285 | | 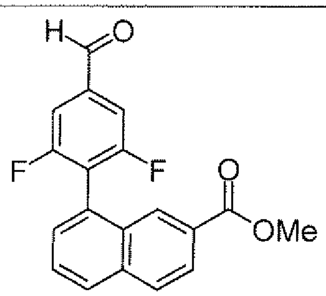 |
| 286 | | 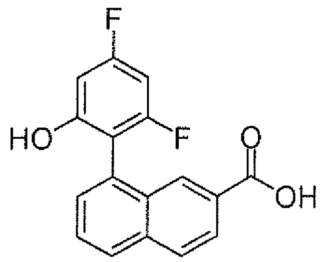 |
| 287 | | 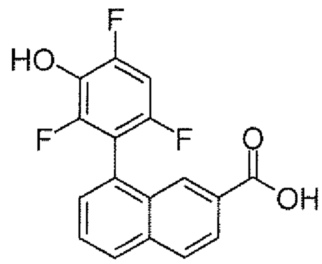 |
| 288 | | 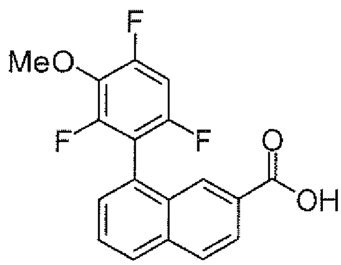 |
| 289 | HCl | 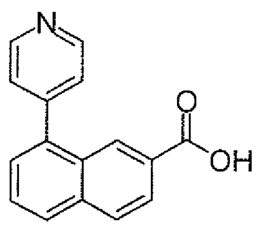 |
| 290 | | 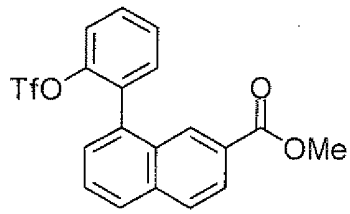 |
| 291 | | 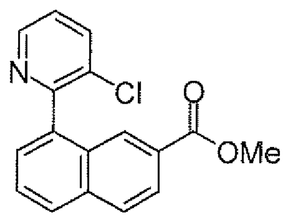 |

[Table 25]

| REx | Sal | Str |
|---|---|---|
| 292 | | 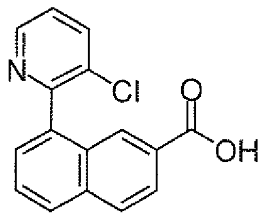 |
| 293 | | 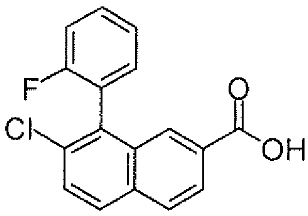 |
| 294 | | 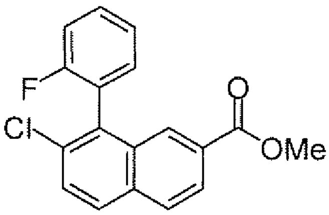 |
| 295 | | 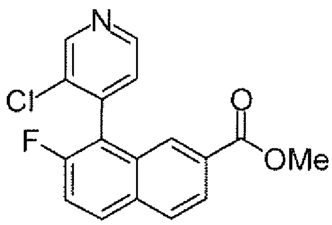 |
| 296 | | 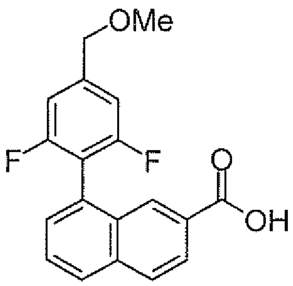 |
| 297 | | 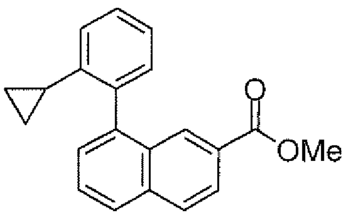 |
| 298 | | 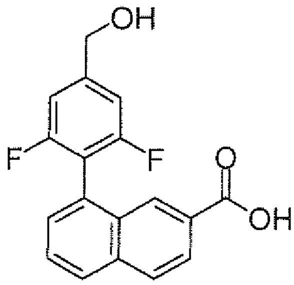 |
| 299 | | 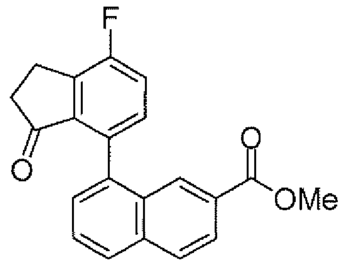 |
| 300 | | 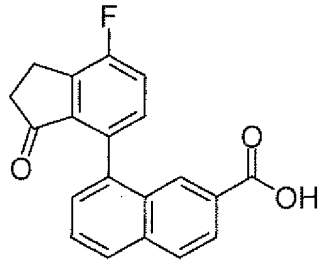 |
| 301 | | 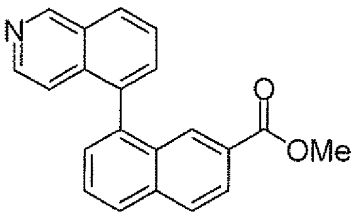 |
| 302 | | 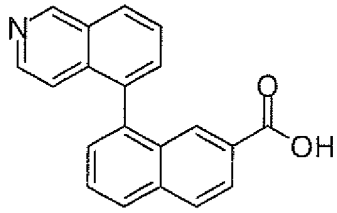 |
| 303 | | 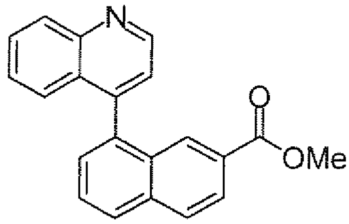 |

[Table 26]

| REx | Sal | Str |
|---|---|---|
| 304 | | 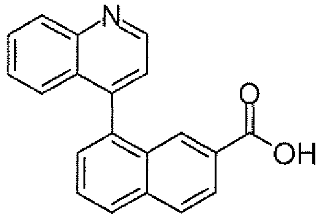 |
| 305 | | 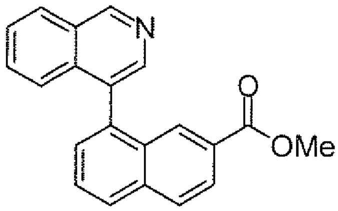 |
| 306 | | 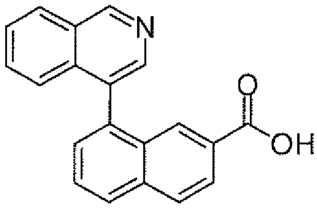 |
| 307 | | 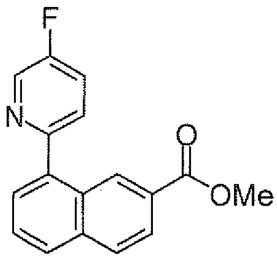 |
| 308 | | 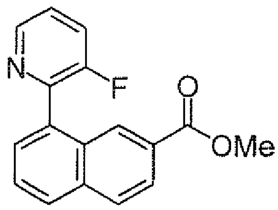 |
| 309 | | 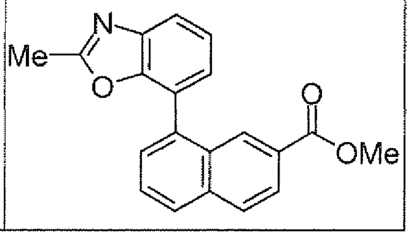 |

| REx | Sal | Str |
|---|---|---|
| 310 | | 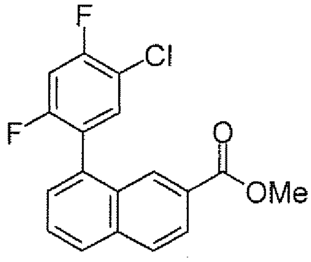 |
| 311 | | 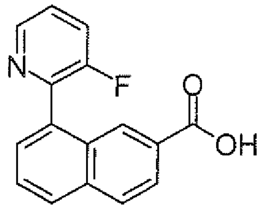 |
| 312 | | 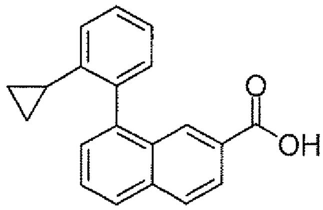 |
| 313 | | 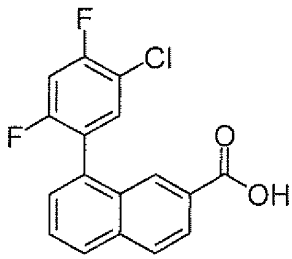 |
| 314 | | 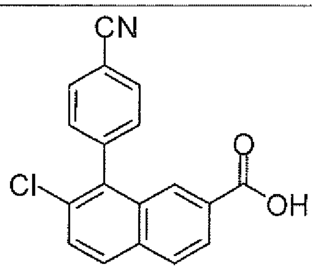 |
| 315 | | 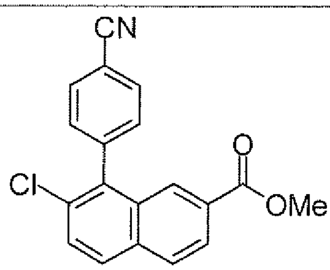 |

[Table 27]

| REx | Sal | Str |
|---|---|---|
| 316 | | 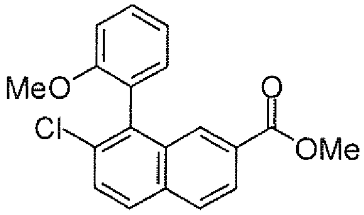 |
| 317 | | 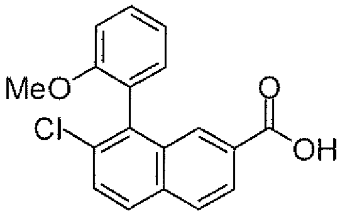 |
| 318 | | 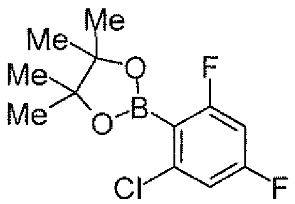 |
| 319 | | 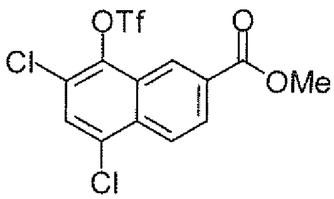 |
| 320 | | 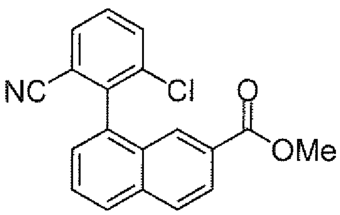 |
| 321 | | 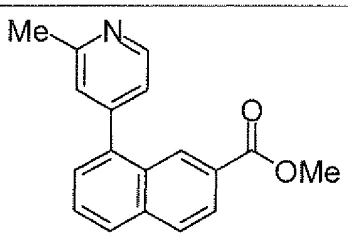 |

| REx | Sal | Str |
|---|---|---|
| 322 | | 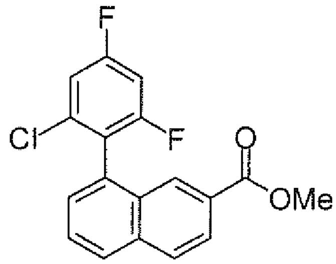 |
| 323 | | 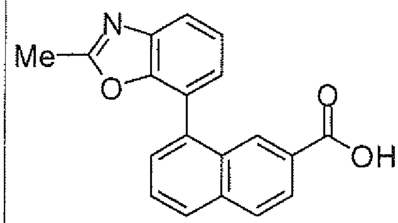 |
| 324 | | 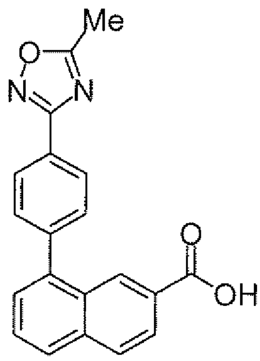 |
| 325 | | 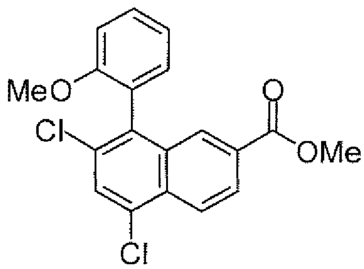 |
| 326 | | 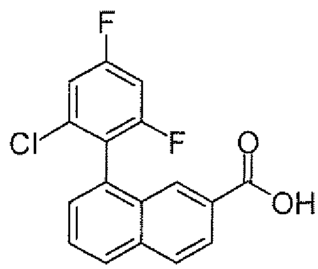 |

[Table 28]

| REx | Sal | Str |
|---|---|---|
| 327 | HCl | 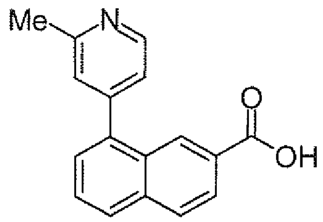 |
| 328 | | 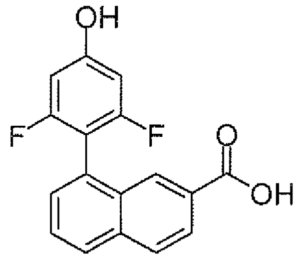 |
| 329 | | 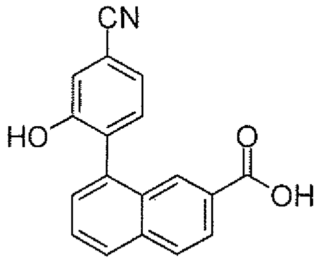 |
| 330 | | 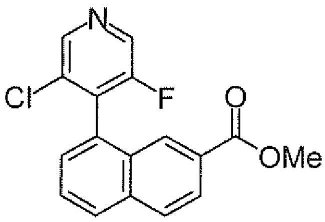 |
| 331 | | 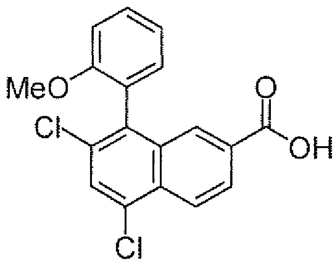 |
| 332 | | 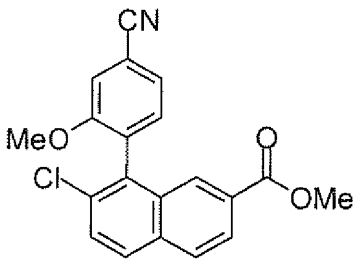 |

| REx | Sal | Str |
|---|---|---|
| 333 | | 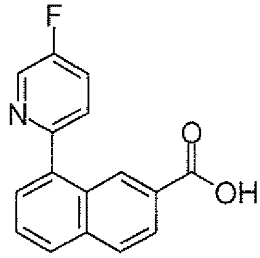 |
| 334 | HCl | 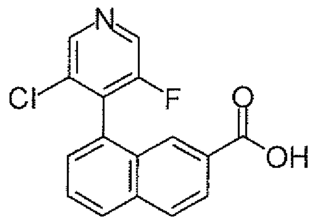 |
| 335 | | 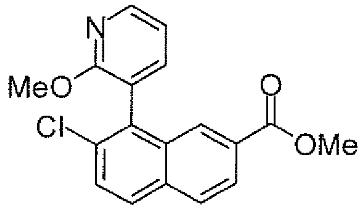 |
| 336 | | 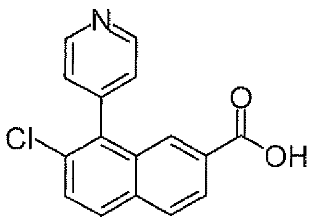 |
| 337 | | 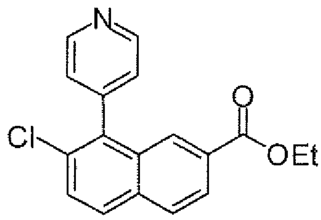 |
| 338 | | 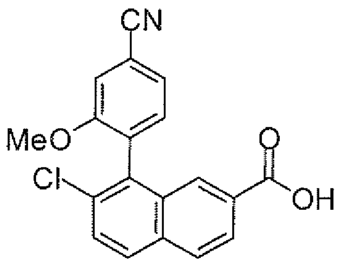 |

[Table 29]

| REx | Sal | Str |
|---|---|---|
| 339 | | 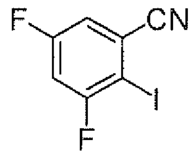 |
| 340 | | 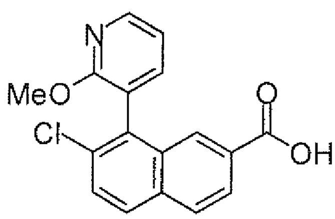 |
| 341 | | 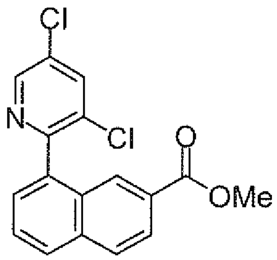 |
| 342 | | 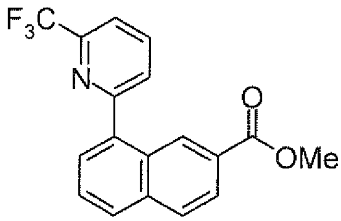 |
| 343 | | 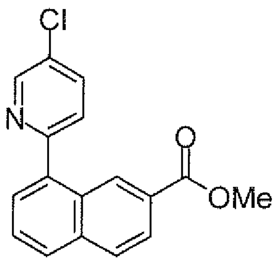 |
| 344 | | 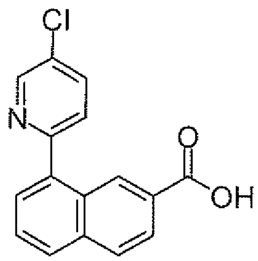 |
| 345 | | 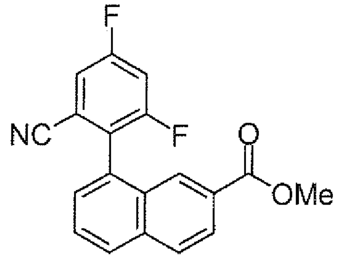 |
| 346 | | 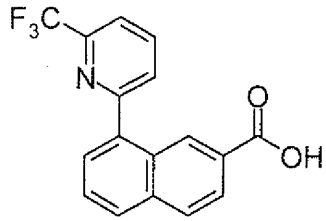 |
| 347 | | 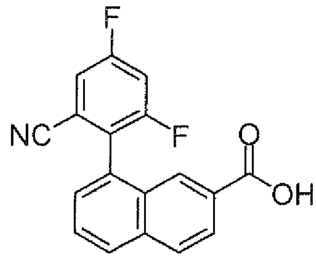 |
| 348 | | 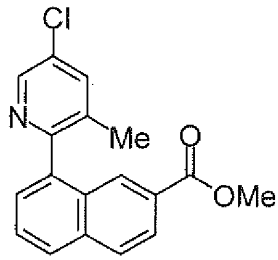 |
| 349 | | 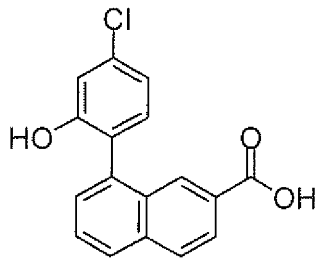 |
| 350 | | 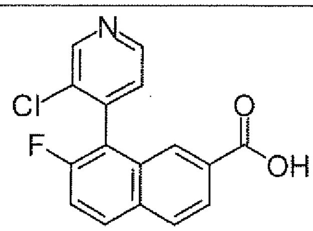 |

[Table 30]

| REx | Sal | Str |
|---|---|---|
| 351 | | 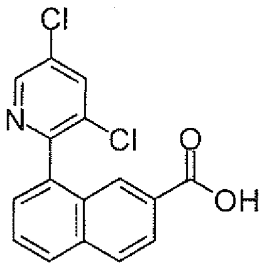 |
| 352 | | 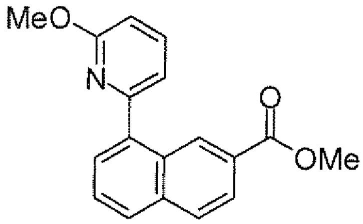 |
| 353 | | 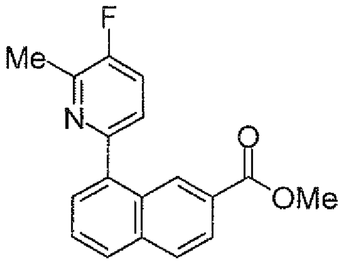 |
| 354 | | 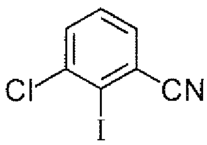 |
| 355 | | 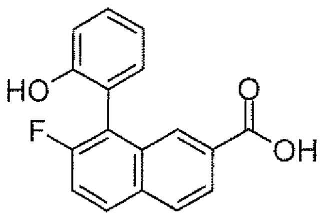 |
| 356 | | 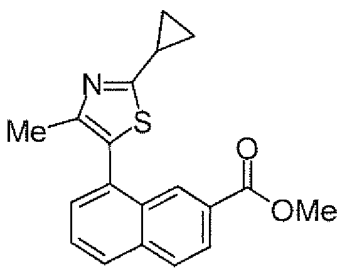 |

| REx | Sal | Str |
|---|---|---|
| 357 | | 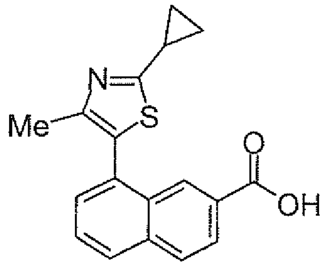 |
| 358 | | 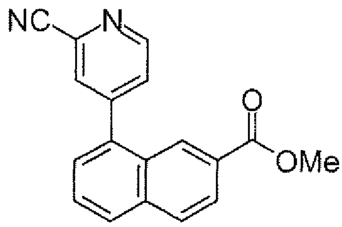 |
| 359 | | 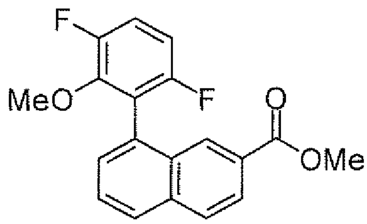 |
| 360 | | 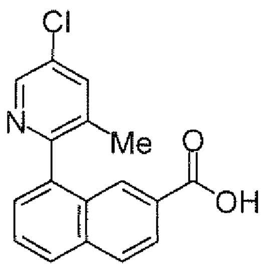 |
| 361 | | 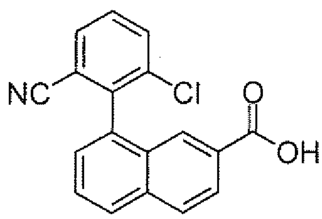 |
| 362 | | 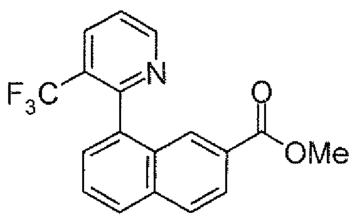 |

[Table 31]

| REx | Sal | Str |
|---|---|---|
| 363 | | 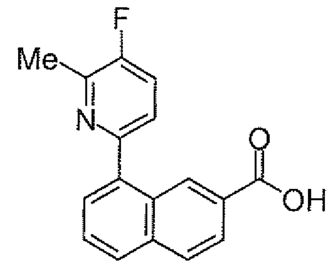 |
| 364 | | 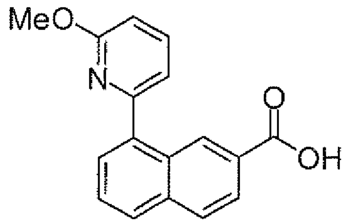 |
| 365 | | 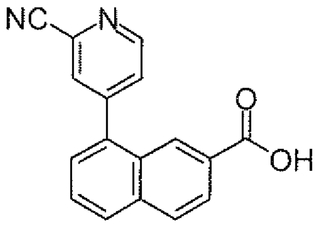 |
| 366 | | 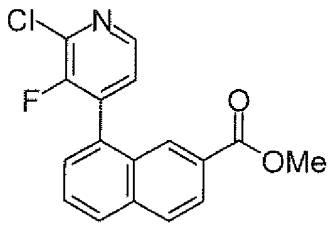 |
| 367 | | 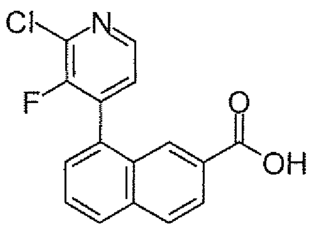 |
| 368 | | 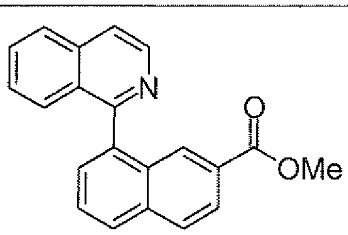 |

| REx | Sal | Str |
|---|---|---|
| 369 | | 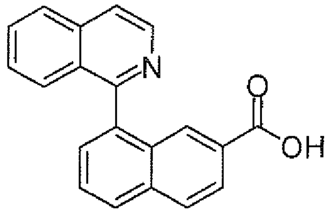 |
| 370 | | 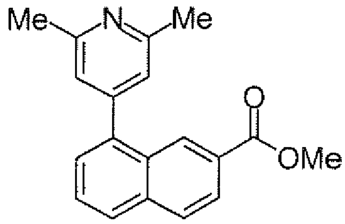 |
| 371 | HCl | 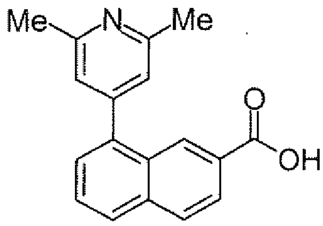 |
| 372 | | 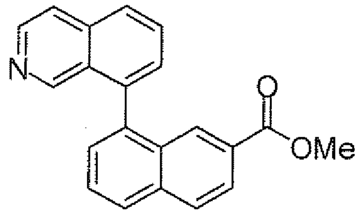 |
| 373 | | 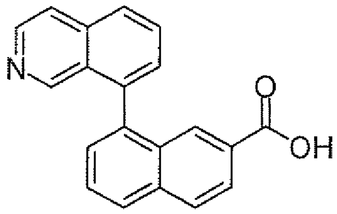 |
| 374 | | 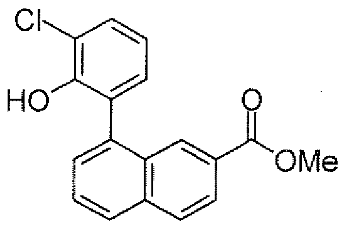 |

[Table 32]

| REx | Sal | Str |
|---|---|---|
| 375 | | 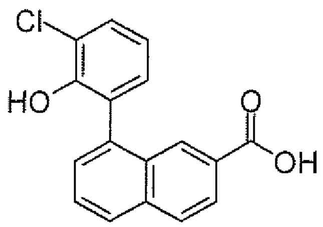 |
| 376 | | 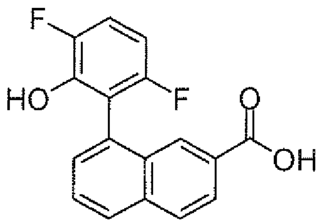 |
| 377 | | 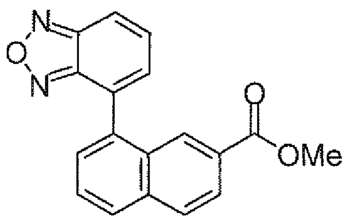 |
| 378 | | 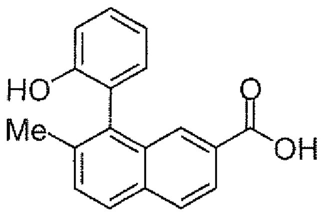 |
| 379 | | 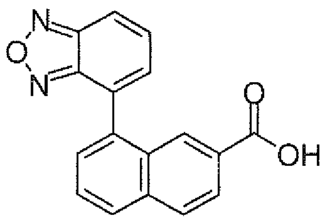 |
| 380 | | 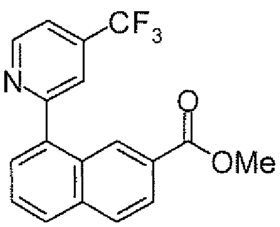 |
| 381 | | 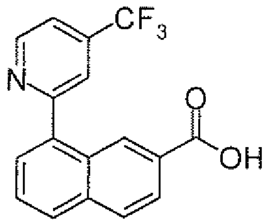 |
| 382 | | 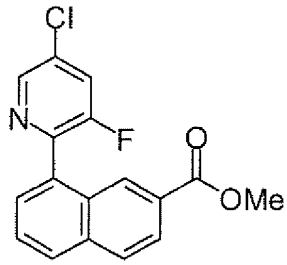 |
| 383 | | 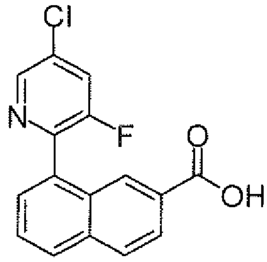 |
| 384 | | 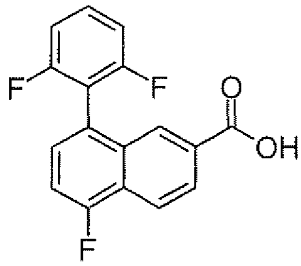 |
| 385 | | 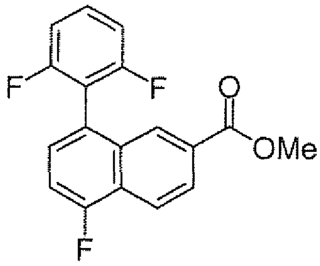 |

[Table 33]

| REx | Sal | Str |
|---|---|---|
| 386 | | 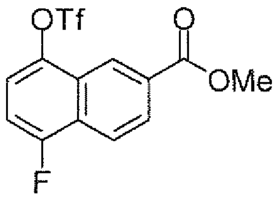 |
| 387 | | 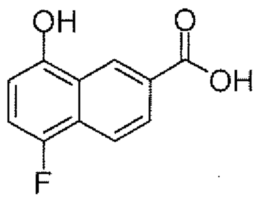 |
| 388 | | 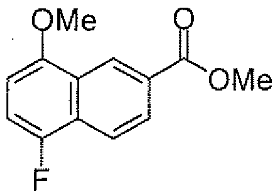 |
| 389 | | 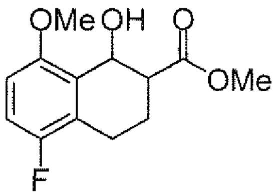 |
| 390 | | 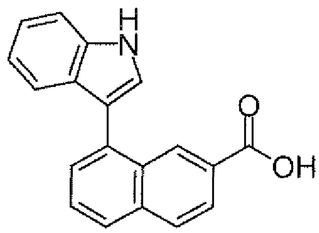 |
| 391 | | 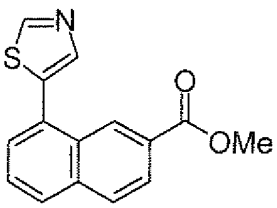 |
| 392 | | 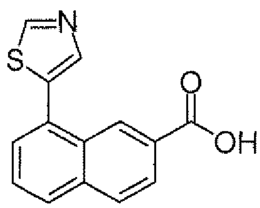 |
| 393 | | 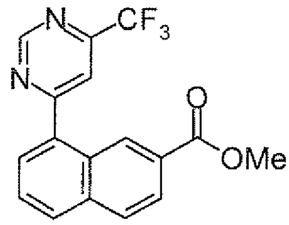 |
| 394 | | 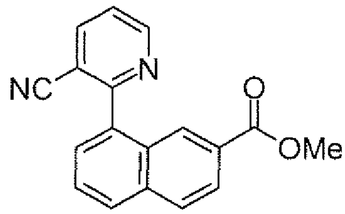 |
| 395 | | 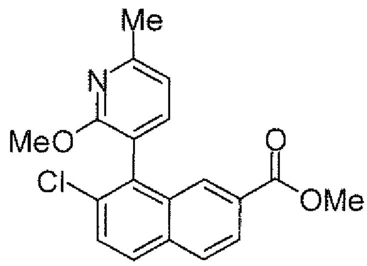 |
| 396 | | 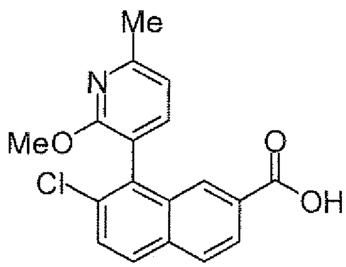 |
| 397 | | 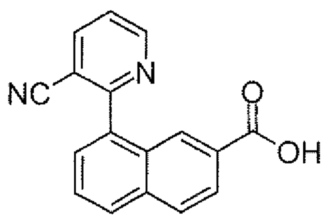 |
| 398 | | 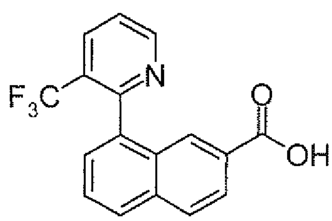 |

[Table 34]

| REx | Sal | Str |
|---|---|---|
| 399 | | 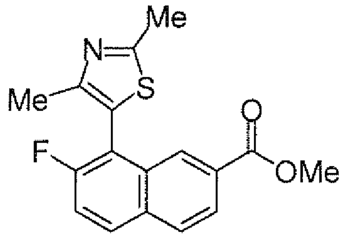 |
| 400 | | 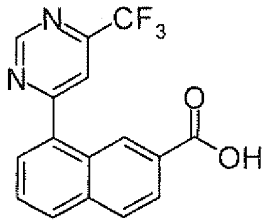 |
| 401 | | 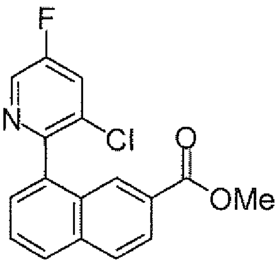 |
| 402 | | 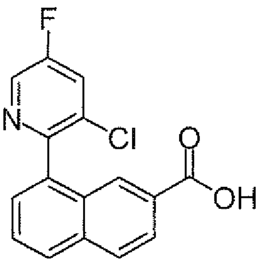 |
| 403 | | 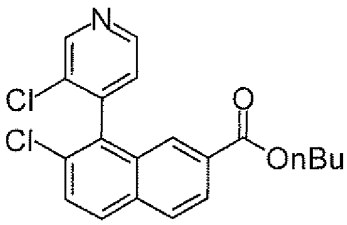 |
| 404 | | 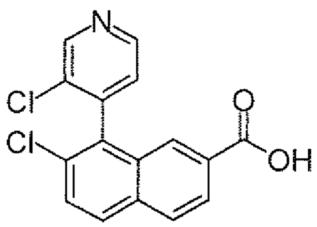 |

| REx | Sal | Str |
|---|---|---|
| 405 | | 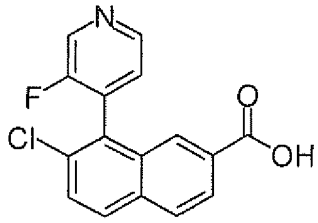 |
| 406 | | 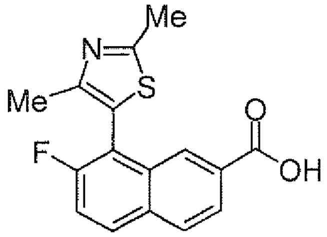 |
| 407 | | 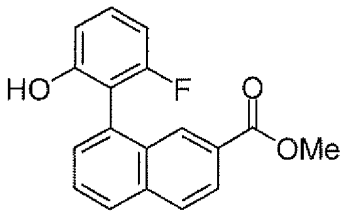 |
| 408 | | 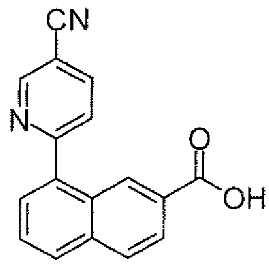 |
| 409 | | 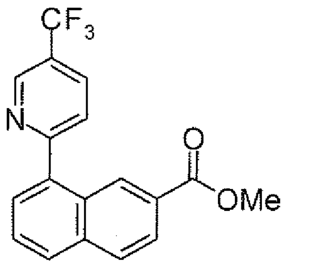 |
| 410 | | 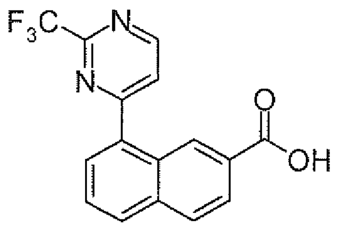 |

[Table 35]

| REx | Sal | Str |
|---|---|---|
| 411 | | 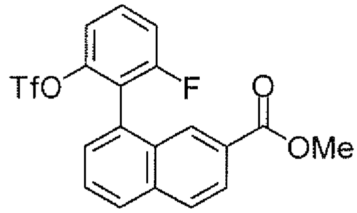 |
| 412 | | 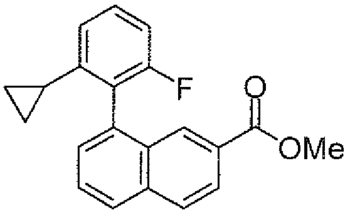 |
| 413 | | 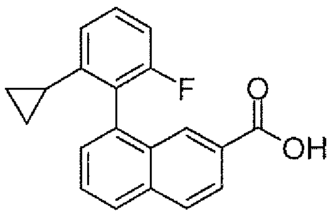 |
| 414 | | 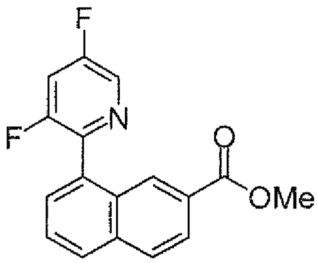 |
| 415 | | 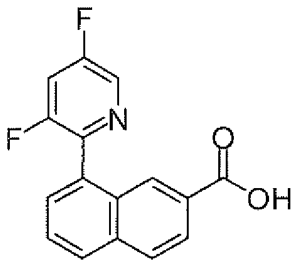 |
| 416 | | 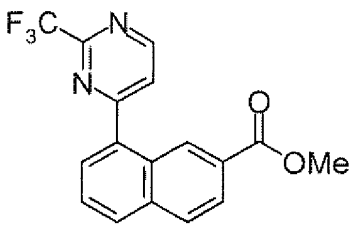 |

| REx | Sal | Str |
|---|---|---|
| 417 | | 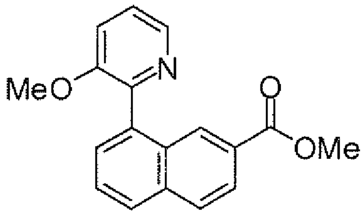 |
| 418 | | 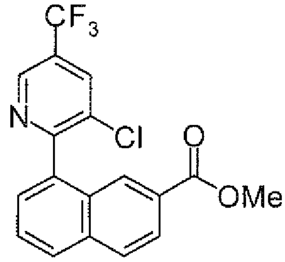 |
| 419 | | 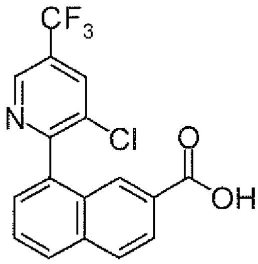 |
| 420 | | 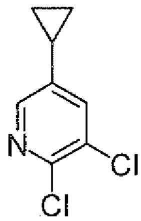 |
| 421 | | 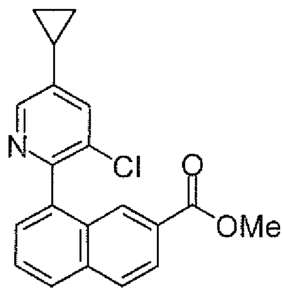 |

[Table 36]

| REx | Sal | Str |
|---|---|---|
| 422 | | 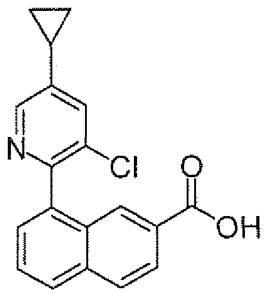 |
| 423 | | 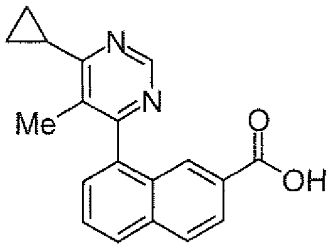 |
| 424 | | 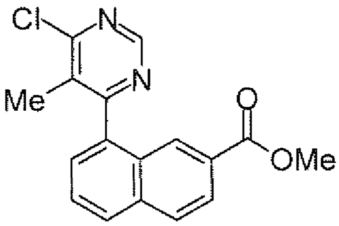 |
| 425 | | 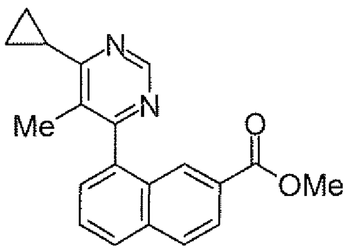 |
| 426 | | 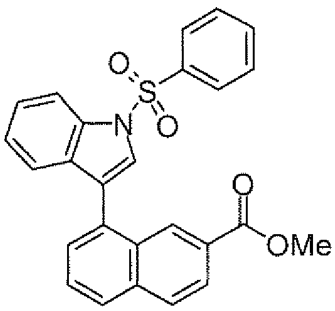 |

| REx | Sal | Str |
|---|---|---|
| 427 | | 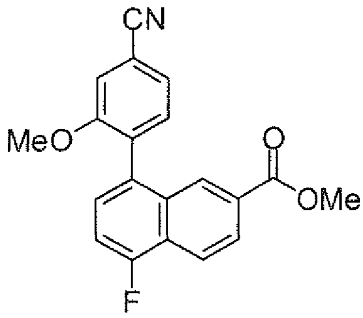 |
| 428 | | 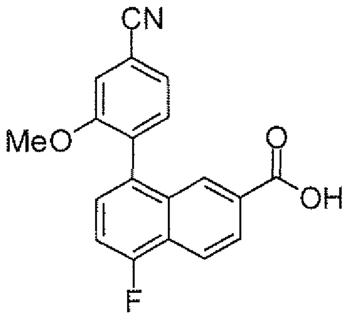 |
| 429 | | 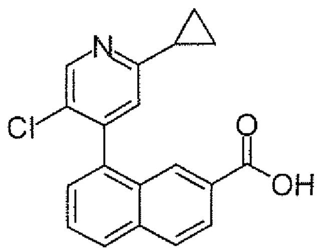 |
| 430 | | 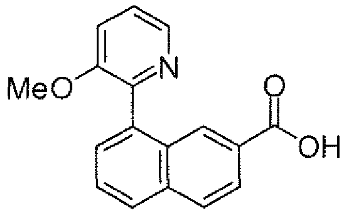 |
| 431 | | 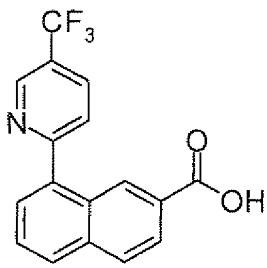 |

[Table 37]

| REx | Sal | Str |
|---|---|---|
| 432 | | 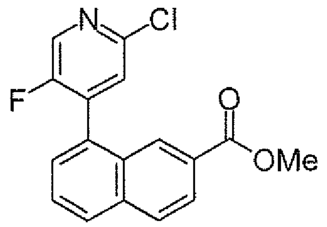 |
| 433 | | 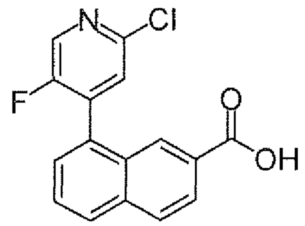 |
| 434 | | 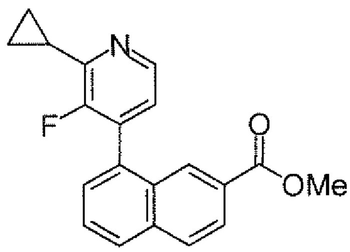 |
| 435 | | 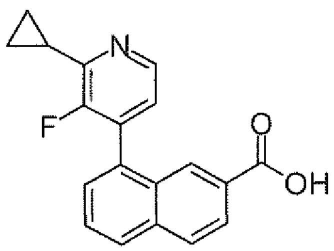 |
| 436 | | 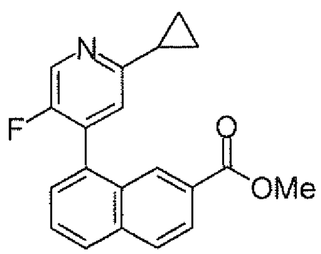 |
| 437 | | 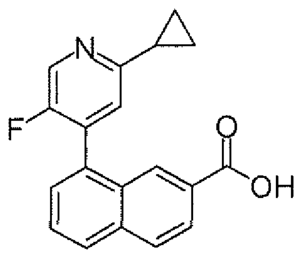 |

| REx | Sal | Str |
|---|---|---|
| 438 | | 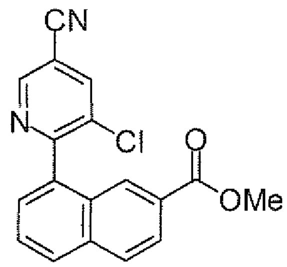 |
| 439 | | 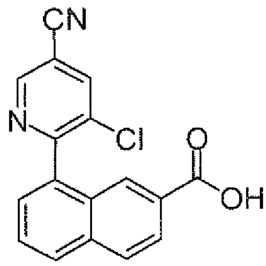 |
| 440 | | 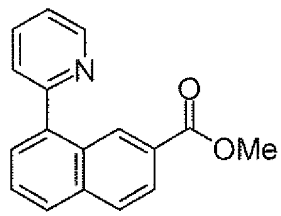 |
| 441 | | 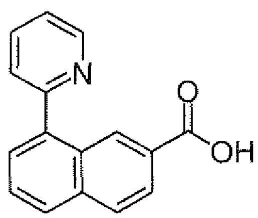 |
| 442 | | 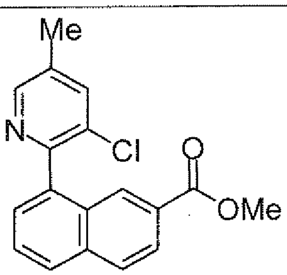 |
| 443 | | 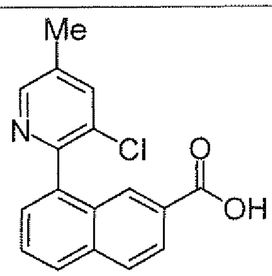 |

[Table 38]

| REx | Sal | Str |
|---|---|---|
| 444 | | 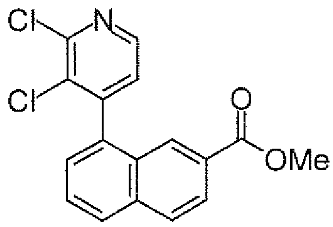 |
| 445 | | 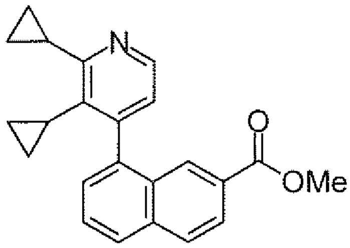 |

| REx | Sal | Str |
|---|---|---|
| 446 | | 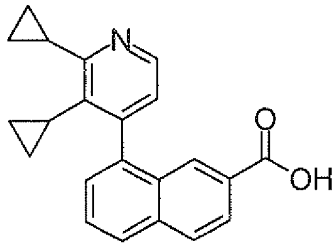 |

[Table 39]

| REx | RSyn | Dat | | REx | RSyn | Dat |
|---|---|---|---|---|---|---|
| 1 | 1 | EI+: 260 | | 39 | 16 | EI+: 281 |
| 2 | 2 | A/E+: 281 | | 40 | 25 | EI+: 259 |
| 3 | 3 | EI+: 240 | | 41 | 16 | FAB+: 349 |
| 4 | 4 | ESI-: 235 | | 42 | 34 | ESI+: 307 |
| 6 | 6 | EI+: 258 | | 43 | 34 | EI+: 301 |
| 7 | 7 | ESI+: 147 | | 44 | 34 | EI+: 310 |
| 9 | 9 | ESI+: 219 | | 45 | 34 | EI+: 287, FAB+: 288 |
| 10 | 10 | EI+: 328 | | 46 | 34 | EI+: 317 |
| 11 | 11 | ESI-: 299 | | 47 | 11 | ESI-. 296 |
| 12 | 12 | ESI-: 308 | | 48 | 11 | ESI-: 272 |
| 13 | 13 | A/E+: 218, 220 | | 49 | 11 | FAB-: 302 |
| 14 | 14 | ESI+: 301 | | 51 | 8 | EI+: 220 |
| 15 | 15 | ESI+: 166, 168 | | 52 | 16 | EI+: 352 |
| 16 | 16 | FAB+: 335 | | 53 | 34 | EI+: 322 |
| 17 | 17 | ESI+: 321 | | 54 | 34 | EI+: 280 |
| 18 | 18 | ESI+ 343 | | 55 | 34 | EI+: 296 |
| 19 | 19 | ESI- 219 | | 56 | 34 | FAB+: 310 |
| 20 | 20 | ESI+: 345 | | 57 | 34 | EI+: 305 |

(continued)

| REx | RSyn | Dat | | REx | RSyn | Dat |
|---|---|---|---|---|---|---|
| 21 | 21 | ESI+: 212, 214 | | 58 | 11 | ESI+: 309 |
| 22 | 22 | ESI+: 342 | | 59 | 11 | ESI-: 265 |
| 23 | 23 | EI+: 211 | | 60 | 11 | ESI-: 290 |
| 24 | 24 | A/E+: 140 | | 61 | 34 | EI+: 294 |
| 25 | 25 | ESI+: 313 | | 62 | 34 | EI+: 306 |
| 26 | 26 | ESI+: 223 | | 63 | 11 | ESI+: 292 |
| 27 | 27 | ESI+: 188 | | 64 | 11 | FAB-: 279 |
| 28 | 28 | ESI-: 181 | | 65 | 11 | ESI+: 280 |
| 29 | 29 | EI+: 241 | | 66 | 34 | EI+: 278 |
| 30 | 30 | ESI+: 305 | | 67 | 34 | EI+: 316 |
| 31 | 31 | ESI+: 253 | | 69 | 11 | ESI-: 263 |
| 32 | 32 | ESI+: 237 | | 70 | 34 | EI+: 317 |
| 34 | 34 | ESI+: 315 | | 71 | 11 | ESI+: 302 |
| 35 | 35 | ESI+: 294 | | 72 | 34 | ESI+: 294 |
| 36 | 36 | ESI+: 338 | | 73 | 11 | ESI+: 280 |
| 37 | 37 | ESI+: 358 | | | | |
| 38 | 38 | EI+: 263 | | | | |

[Table 40]

| REx | RSyn | Dat | | REx | RSyn | Dat |
|---|---|---|---|---|---|---|
| 74 | 11 | ESI-: 286 | | 107 | 25 | ESI+: 263 |
| 76 | 35 | ESI+: 306 | | 108 | 34 | ESI+: 321 |
| 77 | 35 | ESI+: 300 | | 109 | 11 | ESI-: 305 |
| 78 | 34 | ESI+: 311 | | 110 | 35 | EI+: 305 |
| 79 | 11 | ESI-: 290 | | 111 | 35 | EI+: 321 |
| 80 | 11 | ESI-: 282 | | 112 | 11 | EI+: 312 |
| 81 | 11 | ESI-: 295 | | 113 | 11 | FAB-: 290 |
| 82 | 34 | EI+: 264 | | 114 | 11 | FAB-: 306 |
| 83 | 34 | ESI+: 298 | | 115 | 34 | EI+: 310 |
| 84 | 11 | ESI-: 282 | | 116 | 34 | EI+: 298 |
| 85 | 34 | A/E+: 288 | | 117 | 34 | EI+: 298 |
| 86 | 11 | ESI-: 272 | | 118 | 34 | EI+: 330 |
| 87 | 34 | EI+: 281 | | 119 | 11 | FAB-: 295 |
| 88 | 34 | EI+: 277 | | 120 | 11 | FAB-: 283 |
| 89 | 34 | EI+: 281 | | 121 | 11 | FAB-: 283 |
| 90 | 11 | ESI+: 250 | | 122 | 11 | ESI+: 317 |
| 91 | 11 | ESI+: 268 | | 123 | 34 | ESI+: 332 |

(continued)

| REx | RSyn | Dat | | REx | RSyn | Dat |
|---|---|---|---|---|---|---|
| 92 | 11 | ESI+: 264 | | 124 | 34 | EST+: 332 |
| 93 | 11 | ESI+: 268 | | 125 | 34 | EI+: 310 |
| 94 | 35 | EI+: 305 | | 126 | 34 | EI+: 310 |
| 95 | 34 | EI+: 310 | | 127 | 11 | ESI-: 316 |
| 96 | 34 | ESI-: 269 | | 128 | 11 | ESI-: 316 |
| 97 | 11 | ESI+: 255 | | 129 | 11 | FAB+: 297 |
| 98 | 35 | ESI+: 305 | | 130 | 11 | FAB+: 297 |
| 99 | 34 | EI+: 317 | | 131 | 35 | EI+: 328 |
| 100 | 11 | FAB-: 290 | | 132 | 35 | EI+: 332 |
| 101 | 11 | EI+: 296 | | 133 | 11 | EI+: 314 |
| 102 | 11 | FAB-: 290 | | 134 | 11 | FAB-: 317 |
| 103 | 11 | FAB-: 302 | | 135 | 35 | EI+: 297 |
| 104 | 35 | EI+: 299 | | 136 | 35 | EI+: 331 |
| 105 | 11 | ESI+: 286 | | | | |
| 106 | 34 | ESI+: 327 | | | | |

[Table 41]

| REx | RSyn | Dat | | REx | RSyn | Dat |
|---|---|---|---|---|---|---|
| 137 | 35 | EI+: 281 | | 169 | 11 | FAB-: 299 |
| 138 | 35 | EI+: 297 | | 170 | 11 | ESI+: 317 |
| 139 | 11 | ESI+: 284 | | 171 | 34 | ESI+: 299 |
| 140 | 11 | ESI+ 318 | | 172 | 34 | ESI+: 315 |
| 141 | 11 | ESI+: 268 | | 173 | 34 | ESI+: 315 |
| 142 | 11 | ESI+: 284 | | 174 | 11 | ESI+: 285 |
| 143 | 35 | ESI+: 269 | | 175 | 11 | ESI+: 301 |
| 144 | 35 | ESI+: 269 | | 176 | 11 | ESI+: 301 |
| 145 | 11 | ESI-: 253 | | 177 | 34 | EI+: 298 |
| 146 | 11 | ESI-: 253 | | 178 | 35 | EI+: 317 |
| 147 | 34 | EI+: 281 | | 179 | 35 | EI+: 297 |
| 148 | 34 | EI+: 297 | | 180 | 35 | EI+: 297 |
| 149 | 35 | ESI+: 283 | | 181 | 11 | ESI+: 285 |
| 150 | 35 | ESI+: 303 | | 182 | 11 | FAB-: 302 |
| 151 | 35 | ESI+: 294 | | 183 | 11 | ESI+: 284 |
| 152 | 35 | ESI+: 316[M+Na] | | 184 | 34 | EI+: 315 |
| 153 | 11 | ES1 -: 267 | | 185 | 34 | EI+: 330 |
| 154 | 11 | ESI-: 287 | | 186 | 34 | EI+: 330 |
| 155 | 11 | ESI-: 278 | | 187 | 11 | EST+: 284 |

(continued)

| REx | RSyn | Dat | REx | RSyn | Dat |
|---|---|---|---|---|---|
| 156 | 11 | ESI-: 278 | 188 | 11 | ESI+: 302 |
| 157 | 35 | EI+: 281 | 189 | 11 | EI+: 316 |
| 158 | 35 | EI+: 288 | 190 | 11 | ESI+: 317 |
| 159 | 11 | ESI+: 268 | 191 | 28 | ESI+: 176 |
| 160 | 11 | ESI+: 284 | 192 | 28 | APCI-: 192 |
| 161 | 11 | ESI+: 268 | 193 | 28 | ESI+: 209 |
| 162 | 35 | EI+: 326 | 194 | 34 | ESI+: 328 |
| 163 | 34 | EI+: 314 | 195 | 34 | ESI+: 323 |
| 164 | 34 | EI+: 314 | 196 | 34 | ESI+: 316 |
| 165 | 34 | EI+: 330 | 197 | 34 | ESI+: 334 |
| 166 | 11 | ESI+: 275 | 198 | 34 | ESI+: 349 |
| 167 | 11 | ESI+: 313 | | | |
| 168 | 11 | FAB-: 299 | | | |

[Table 42]

| REx | RSyn | Dat | REx | RSyn | Dat |
|---|---|---|---|---|---|
| 199 | 35 | EI+: 314 | 231 | 34 | EI+: 316 |
| 200 | 35 | EI+: 314 | 232 | 34 | ESI+: 294 |
| 201 | 11 | ESI-: 312 | 233 | 35 | ESI+: 307 |
| 202 | 11 | A/E+: 319 | 234 | 34 | ESI+: 294 |
| 203 | 11 | ESI-: 300 | 235 | 11 | FAB-: 301 |
| 204 | 11 | ESI-: 344 | 236 | 11 | ESI+: 280 |
| 205 | 11 | ESI-: 360 | 237 | 11 | ESI-: 313 |
| 206 | 11 | EI+: 300 | 238 | 35 | EI+: 328 |
| 207 | 11 | EI+: 300 | 239 | 35 | EI+: 346 |
| 208 | 35 | EI+: 305 | 240 | 11 | ESI-: 331 |
| 209 | 35 | EI+: 305 | 241 | 11 | ESI+: 253 |
| 210 | 35 | EI+: 310 | 242 | 11 | ESI+: 287 |
| 211 | 34 | EI+: 328 | 243 | 34 | EI+: 317 |
| 212 | 34 | ESI+: 267 | 244 | 35 | ESI+: 278 |
| 213 | 11 | FAB+: 292 | 245 | 35 | ESI+: 339 |
| 214 | 11 | FAB+: 292 | 246 | 11 | ESI-: 289 |
| 215 | 11 | ESI+: 297 | 248 | 11 | ESI+: 304 |
| 216 | 11 | EI+: 314 | 249 | 11 | ESI+: 264 |
| 217 | 34 | EI+: 321 | 250 | 11 | ESI+: 325 |
| 218 | 34 | EI+: 295 | 251 | 34 | ESI+: 294 |
| 219 | 34 | ESI+: 384 | 252 | 34 | ESI+: 313 |

(continued)

| REx | RSyn | Dat | | REx | RSyn | Dat |
|---|---|---|---|---|---|---|
| 220 | 11 | FAB-: 306 | | 253 | 11 | ESI+: 299 |
| 221 | 11 | ESI+: 282 | | 254 | 2 | A/E+: 297 |
| 222 | 34 | EI+: 262 | | 255 | 2 | ESI+ 282 |
| 223 | 34 | EI+: 280 | | 256 | 35 | A/E+: 342 |
| 224 | 34 | EI+: 296 | | 257 | 11 | A/E+: 282 |
| 225 | 34 | EI+: 292 | | 258 | 34 | ESI+: 300 |
| 226 | 11 | ESI+: 249 | | 259 | 34 | ESI+: 296 |
| 227 | 11 | ESI+: 267 | | 260 | 11 | FAB+: 280 |
| 228 | 11 | ESI+: 283 | | 261 | 11 | ESI+: 280 |
| 229 | 11 | ESI+: 279 | | | | |
| 230 | 11 | ESI-: 291 | | | | |

[Table 43]

| REx | RSyn | Dat | | REx | RSyn | Dat |
|---|---|---|---|---|---|---|
| 262 | 11 | ESI+: 286 | | 294 | 34 | ESI+: 315 |
| 263 | 11 | ESI+: 282 | | 295 | 38 | A/E+: 316 |
| 264 | 11 | A/E+: 327 | | 296 | 11 | ESI-: 327 |
| 265 | 34 | ESI+: 328 | | 297 | 34 | EI+: 302 |
| 266 | 35 | ESI+: 314 | | 298 | 11 | FAB-: 313 |
| 267 | 11 | ESI+: 300 | | 299 | 35 | EI+: 334 |
| 268 | 3 | EI+: 240 | | 300 | 11 | ESI-: 319 |
| 269 | 35 | EI+: 346 | | 301 | 35 | ESI+: 314 |
| 270 | 2 | ESI+: 298 | | 302 | 11 | ESI+: 300 |
| 271 | 16 | EI+: 368 | | 303 | 35 | ESI+: 314 |
| 272 | 25 | EI+: 330 | | 304 | 11 | ESI+: 300 |
| 273 | 11 | ESI-: 331 | | 305 | 35 | EST+: 314 |
| 274 | 11 | ESI-: 211 | | 306 | 11 | ESI+: 300 |
| 275 | 2 | ESI+: 300 | | 307 | 35 | ESI+: 282 |
| 276 | 34 | ESI+: 314 | | 308 | 35 | ESI+: 282 |
| 277 | 34 | ESI+: 330 | | 309 | 35 | ESI+: 318 |
| 278 | 11 | ESI+: 300 | | 310 | 34 | EI+: 332 |
| 279 | 11 | ESI+: 316 | | 311 | 11 | ESI+: 268 |
| 280 | 35 | ESI+: 360 | | 312 | 11 | EST+: 289 |
| 281 | 11 | ESI+: 346 | | 313 | 11 | ESI+: 319 |
| 282 | 34 | ESI+: 346 | | 314 | 11 | ESI-: 306 |
| 283 | 35 | ESI+: 314 | | 315 | 34 | ESI+: 322 |
| 284 | 11 | EST+: 300 | | 316 | 34 | ESI+: 327 |

(continued)

| REx | RSyn | Dat | | REx | RSyn | Dat |
|---|---|---|---|---|---|---|
| 285 | 35 | ESI+: 327 | | 317 | 11 | EST-: 311 |
| 286 | 2 | FAB+: 300 | | 318 | 25 | EI+: 274 |
| 287 | 2 | A/E-: 317 | | 319 | 16 | FAB+: 403 |
| 288 | 11 | ESI+: 332 | | 320 | 35 | EI+: 321 |
| 289 | 11 | ESI+: 250 | | 321 | 34 | ESI+: 278 |
| 290 | 16 | EI+: 410 | | 322 | 34 | EI+: 332 |
| 291 | 35 | ESI+: 298 | | 323 | 11 | ESI+: 304 |
| 292 | 11 | ESI+: 284 | | | | |
| 293 | 11 | ESI-: 299 | | | | |

[Table 44]

| REx | RSyn | Dat | | REx | RSyn | Dat |
|---|---|---|---|---|---|---|
| 324 | 11 | ESI+: 331 | | 357 | 11 | ESI+: 310 |
| 325 | 34 | FAB+: 360, 362 | | 358 | 35 | ESI+: 289 |
| 326 | 11 | ESI+: 319 | | 359 | 34 | EI+: 328 |
| 327 | 11 | ESI+: 264 | | 360 | 11 | ESI+: 298 |
| 328 | 2 | FAB+: 300 | | 361 | 11 | A/E-: 306 |
| 329 | 2 | A/E-: 288 | | 362 | 35 | ESI+: 332 |
| 330 | 35 | A/E+: 316 | | 363 | 11 | ESI+: 282 |
| 331 | 11 | ESI-: 345 | | 364 | 11 | ESI+: 280 |
| 332 | 34 | EI: 351 | | 365 | 11 | ESI+: 275 |
| 333 | 11 | ESI+: 268 | | 366 | 34 | ESI+: 316 |
| 334 | 11 | ESI+: 302 | | 367 | 11 | ESI+: 302 |
| 335 | 34 | ESI+: 328 | | 368 | 35 | ESI+: 314 |
| 336 | 11 | EST+: 284 | | 369 | 11 | ESI+: 300 |
| 337 | 34 | ESI+: 312 | | 370 | 35 | ESI+: 292 |
| 338 | 11 | ESI-: 336 | | 371 | 11 | ESI+: 278 |
| 339 | 29 | EI+: 265 | | 372 | 34 | ESI+: 314 |
| 340 | 11 | ESI+: 31.4 | | 373 | 11 | ESI+: 300 |
| 341 | 35 | ESI+: 332 | | 374 | 34 | ESI+: 313 |
| 342 | 35 | EST+: 332 | | 375 | 11 | FAB-: 299 |
| 343 | 35 | ESI+: 298 | | 376 | 2 | FAB+: 300 |
| 344 | 11 | ESI+: 284 | | 377 | 35 | A/E-: 304 |
| 345 | 35 | EI+: 323 | | 378 | 2 | A/E-: 277 |
| 346 | 11 | ESI+: 318 | | 379 | 11 | FAB-: 289 |
| 347 | 11 | FAB-: 308 | | 380 | 35 | ESI+: 332 |
| 349 | 2 | ESI+: 298 | | 381 | 11 | ESI+: 318 |

(continued)

| REx | RSyn | Dat | | REx | RSyn | Dat |
|---|---|---|---|---|---|---|
| 350 | 11 | ESI+: 302 | | 382 | 35 | ESI+: 316, 318 |
| 351 | 11 | ESI+: 318 | | 383 | 11 | ESI+: 302 |
| 352 | 35 | ESI+: 294 | | 384 | 11 | ESI+: 303 |
| 353 | 35 | ESI+: 296 | | 385 | 34 | EI: 316 |
| 354 | 29 | EI+: 263 | | 386 | 16 | EI: 352 |
| 355 | 2 | A/E-: 281 | | | | |
| 356 | 35 | ESI+: 324 | | | | |

[Table 45]

| REx | RSyn | Dat | | REx | RSyn | Dat |
|---|---|---|---|---|---|---|
| 387 | 2 | ESI-: 205 | | 418 | 35 | ESI+: 366 |
| 389 | 10 | FAB+: 254 | | 419 | 11 | ESI+: 352, 354 |
| 390 | 11 | ESI+: 288 | | 420 | 36 | ESI+: 188 |
| 391 | 35 | ESI+: 270 | | 421 | 35 | ESI+: 338 |
| 392 | 11 | ESI+: 256 | | 422 | 11 | ESI+: 324 |
| 393 | 35 | ESI+: 333 | | 423 | 11 | ESI+: 305 |
| 394 | 35 | ESI+: 289 | | 424 | 35 | ESI+: 313 |
| 395 | 34 | ESI+: 342 | | 425 | 36 | A/E+: 319 |
| 396 | 11 | East+: 328 | | 426 | 35 | ESI+: 442 |
| 397 | 11 | ESI+: 275 | | 427 | 34 | ESI+: 336 |
| 398 | 11 | ESI+: 318 | | 428 | 11 | ESI-: 320 |
| 399 | 35 | EI+: 315 | | 429 | 11 | A/E+: 324 |
| 400 | 11 | ESI+: 319 | | 430 | 11 | ESI+: 280 |
| 401 | 35 | ESI+: 316 | | 431 | 11 | ESI+: 318 |
| 402 | 11 | ESI+: 302, 304 | | 432 | 35 | A/E+: 316 |
| 403 | 37 | ESI+: 374, 376 | | 433 | 11 | ESI+: 302 |
| 404 | 11 | ESI+: 318 | | 434 | 36 | A/E+: 322 |
| 405 | 11 | ESI+: 302 | | 435 | 11 | A/E+: 308 |
| 406 | 11 | ESI+: 302 | | 436 | 36 | A/E+: 322 |
| 407 | 2 | EI+: 296 | | 437 | 11 | A/E+: 308 |
| 408 | 11 | ESI+: 275 | | 438 | 35 | ESI+: 323, 325 |
| 409 | 35 | ESI+: 332 | | 439 | 11 | A/E+: 309 |
| 410 | 11 | ESI+: 319 | | 440 | 35 | A/E+: 264 |
| 411 | 16 | ESI+: 429 | | 441 | 11 | ESI+: 250 |
| 412 | 34 | EI+: 320 | | 442 | 35 | A/E+: 312 |
| 413 | 11 | ESI+: 307 | | 443 | 11 | A/E+: 298 |
| 414 | 35 | ESI+: 300 | | 444 | 34 | A/E+: 332, 334 |

(continued)

| REx | RSyn | Dat | REx | RSyn | Dat |
|---|---|---|---|---|---|
| 415 | 11 | ESI+: 286 | 445 | 36 | ESI+: 344 |
| 416 | 35 | ESI+: 333 | 446 | 11 | A/E+: 330 |
| 417 | 35 | ESI+: 294 | | | |

[Table 46]

| REx | RSyn | Dat |
|---|---|---|
| 5 | 5 | MR-$CDCl_3$: 4.01 (3H, s), 6.12 (1H, brs), 7.61 (1H, s), 8.17-8.23 (2H, m), 8.99-9.00 (1H, m) |
| 8 | 8 | NMR-$CDCl_3$: 2.43 (3H, s), 3.99 (3H, s), 5.38 (1H, brs), 7.36 (1H, d, J = 7.6 Hz), 7.40 (1H, d, J = 7.6 Hz), 7.80 (1H, d, J = 8.8 Hz), 7.97-8.03 (1H, m), 8.94 (1H, s) |
| 33 | 33 | NMR-$CDCl_3$: 1.41 (3H, t, J = 7.2 Hz), 2.72-2.79 (2H, m), 3.06-3.16 (2H, m), 3.90 (3H, s), 4.43 (2H, q, J = 7.2 Hz), 7.19 (1H, d, J = 7.2 Hz), 7.78-7.88 (2H, m) |
| 50 | 6 | NMR-$CDCl_3$: 1.46 (3H, t, J = 6.8 Hz), 3.99 (3H, s), 4.43 2H, q, J = 6.8 Hz), 7.44-7.49 (1H, m), 7.77-7.81 (1H, m), 7.91 (1H, d, J = 8.8 Hz), 8.06-8.09 (1H, m), 8.72-8.74 (1H, m) |
| 68 | 11 | NMR-DMSO$d_6$: 7.43-7.51 (2H, m), 7.66 (1H, d, J = 3.2 Hz), 7.76-7.81 (1H, m), 8.03-8.20 (4H, m) |
| 75 | 11 | NMR-$CDCl_3$: 7.31-7.48 (4H, m), 7.54-7.60 (1H, m), 7.62-7.69 (1H, m), 7.89-8.00 (2H, m), 8.06-8.14 (1H, m), 8.33 (1H, s) |
| 247 | 34 | NMR-$CDCl_3$: 0.78-8.22 (2H, m), 1.12-1.17 (2H, m), 2.41-2.48 (1H, m), 4.01 (3H, s), 7.30 (1H, d, J = 7.8 Hz), 7.49 (1H, t, J = 7.8 Hz), 7.73 (1H, t, J = 8,3 Hz), 7.88 (1H, d, J = 8.3 Hz), 8.08 (1H, d, J = 8.8 Hz), 9.18 (1H, s) |
| 348 | 35 | NMR-$CDCl_3$: 2.09 (3H, s), 3.90 (3H, s), 7.46 (1H, d, J = 8 Hz), 7.66 (1H, t, J = 8 Hz), 7.71 (1H, s), 7.96 (2H, m), 8.08 (1H, d, J = 8 Hz), 8.15 (1H, s), 8.58 (1H, s) |
| 388 | 6 | NMR-$CDCl_3$: 3.99 (3H, s), 4.01 (3H, s), 6.71-6.75 (1H, m), 7.13-7.18 (1H, m), 8.06-8.08 (1H, m), 8.13-8.16 (1H, m), 9.00 (1H, s) |

[Table 47]

| Ex | Sal | Str |
|---|---|---|
| 1 | HCl | 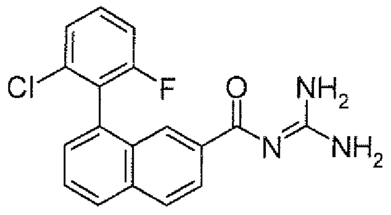 |
| 2 | HCl | 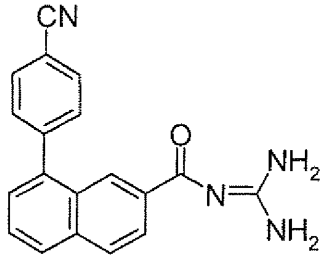 |
| 3 | HCl | 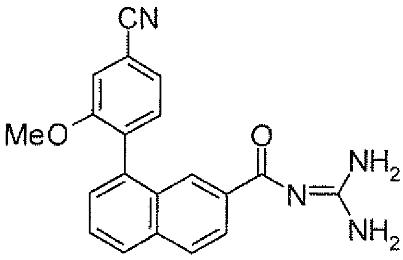 |
| 4 | HCl | 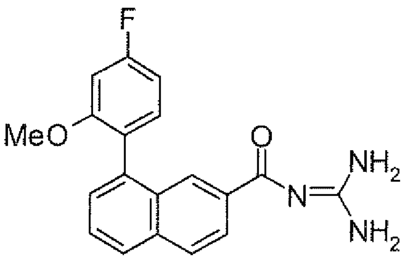 |
| 5 | HCl | 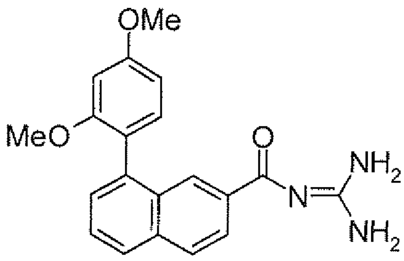 |
| 6 | HCl | 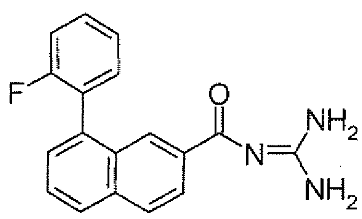 |
| 7 | HCl | 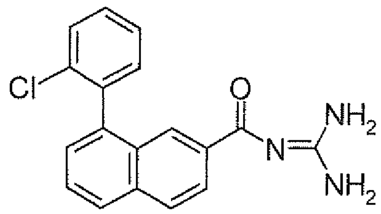 |
| 8 | HCl | 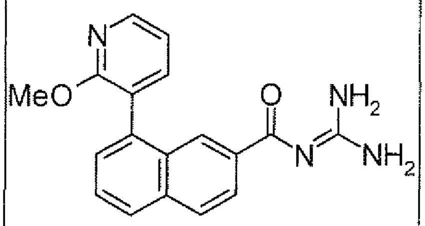 |
| 9 | HCl | 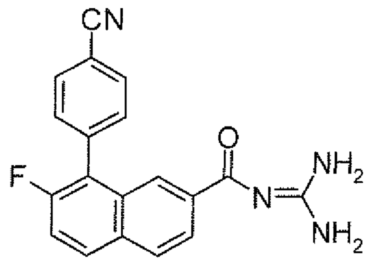 |
| 10 | HCl | 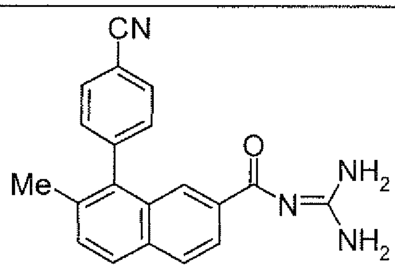 |
| 11 | HCl | 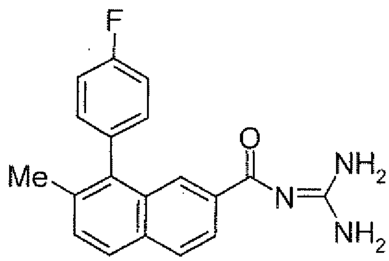 |
| 12 | HCl | 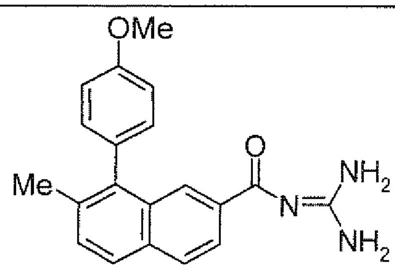 |

[Table 48]

| Ex | Sal | Str |
|---|---|---|
| 13 | HCl | 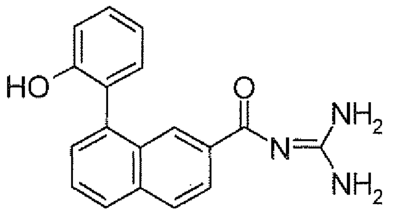 |
| 14 | HCl | 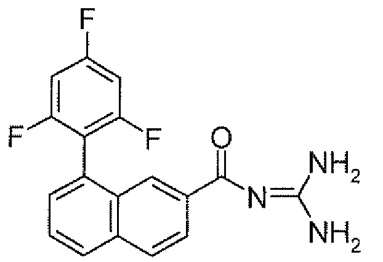 |
| 15 | HCl | 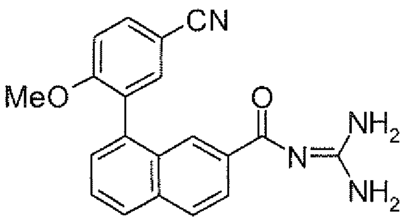 |
| 16 | HCl | 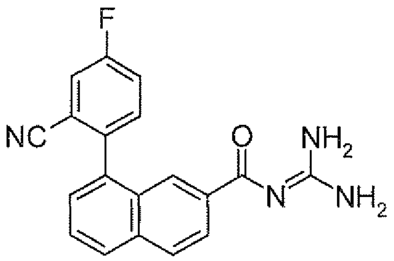 |
| 17 | HCl | 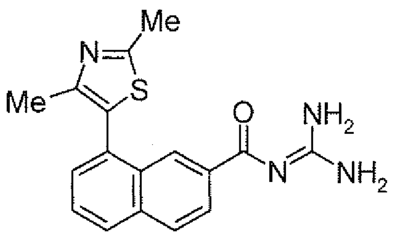 |
| 18 | HCl | 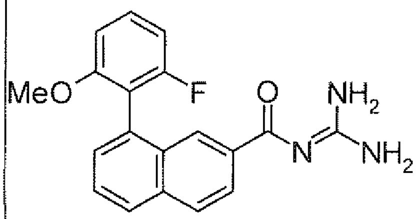 |

| Ex | Sal | Str |
|---|---|---|
| 19 | HCl | 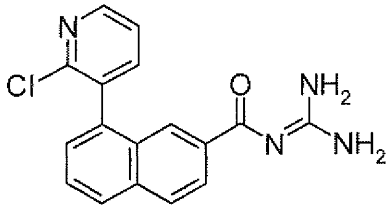 |
| 20 | HCl | 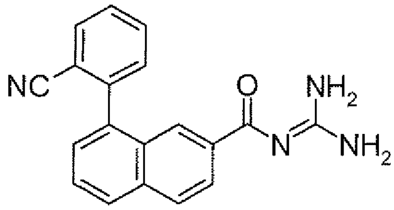 |
| 21 | 2HCl | 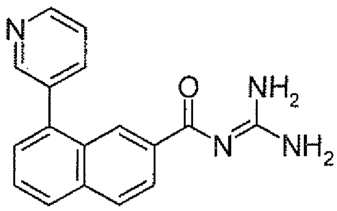 |
| 22 | HCl | 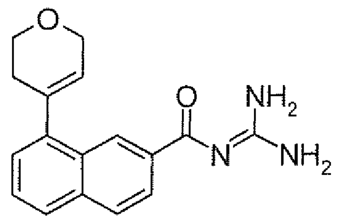 |
| 23 | HCl | 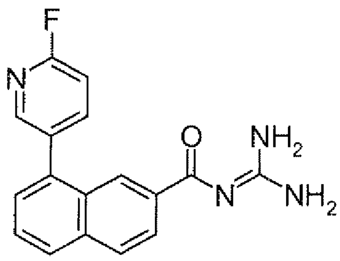 |
| 24 | 2HCl | 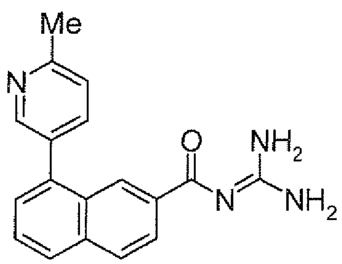 |

[Table 49]

| Ex | Sal | Str |
|---|---|---|
| 25 | 2HCl | 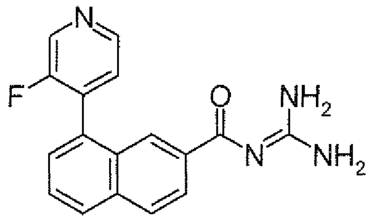 |
| 26 | HCl | 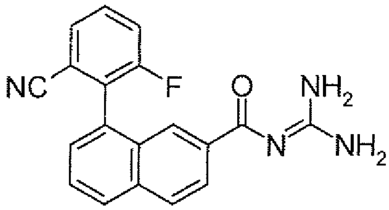 |
| 27 | HCl | 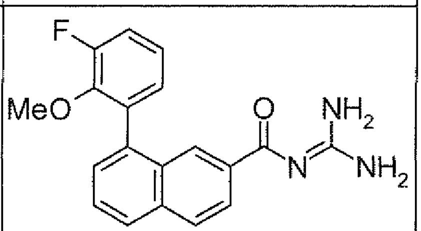 |
| 28 | HCl | 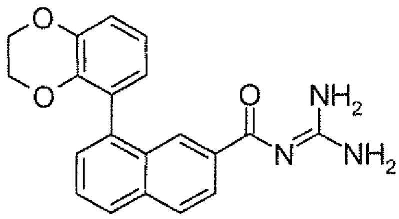 |
| 29 | HCl | 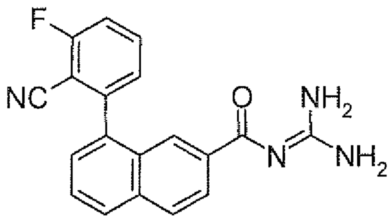 |
| 30 | HCl | 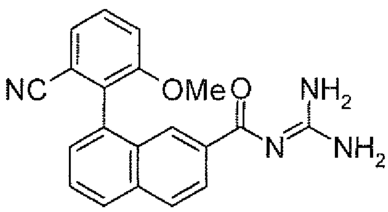 |

| Ex | Sal | Str |
|---|---|---|
| 31 | 2HCl | 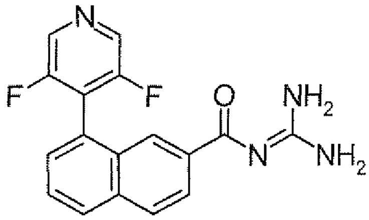 |
| 32 | HCl | 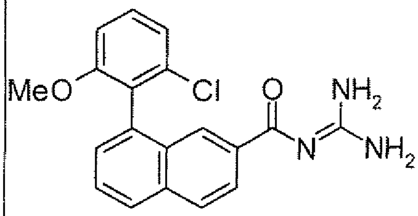 |
| 33 | HCl | 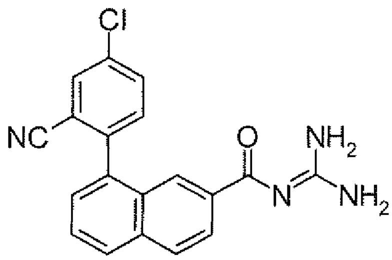 |
| 34 | HCl | 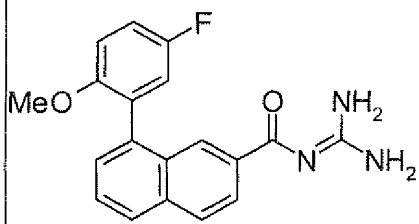 |
| 35 | HCl | 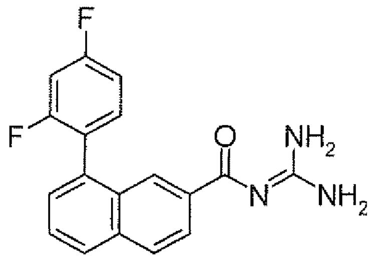 |
| 36 | HCl | 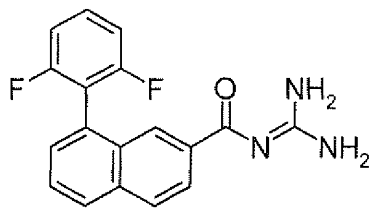 |

[Table 50]

| Ex | Sal | Str |
|---|---|---|
| 37 | HCl | 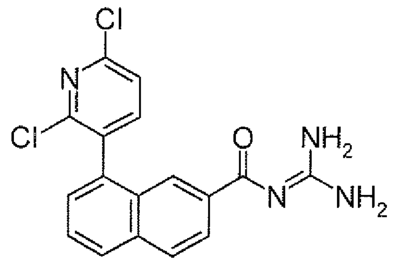 |
| 38 | HCl | 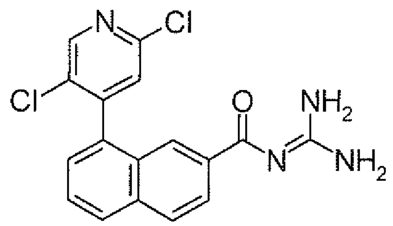 |
| 39 | HCl | 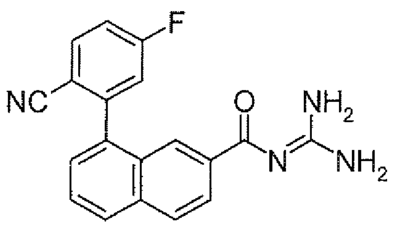 |
| 40 | HCl | 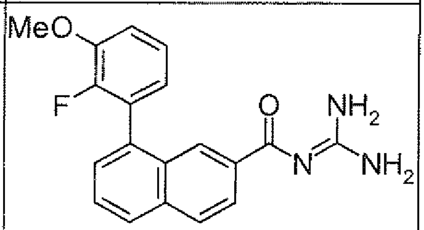 |
| 41 | HCl | 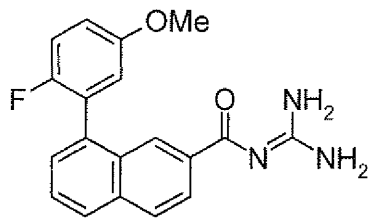 |
| 42 | HCl | 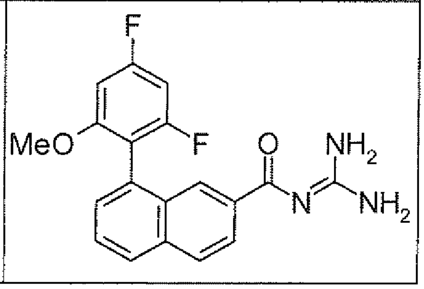 |
| 43 | HCl | 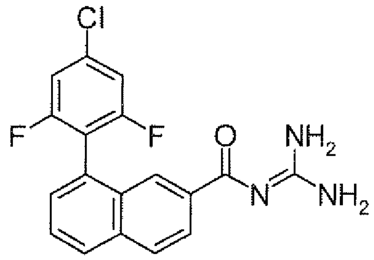 |
| 44 | 2HCl | 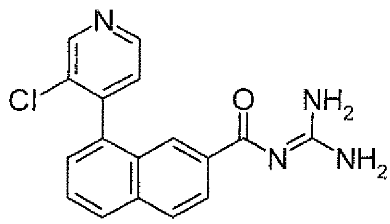 |
| 45 | 2HCl | 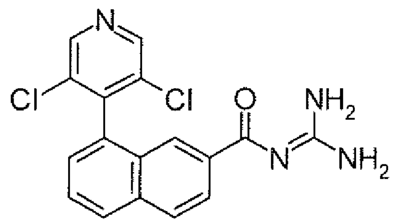 |
| 46 | HCl | 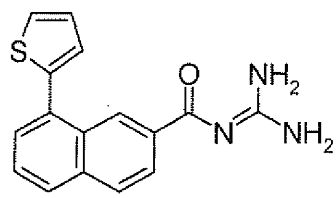 |
| 47 | HCl | 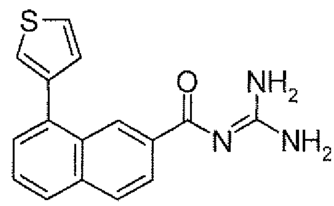 |
| 48 | HCl | 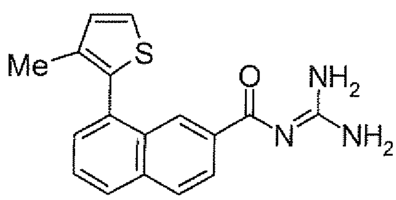 |

[Table 51]

| Ex | Sal | Str |
|---|---|---|
| 49 | HCl | 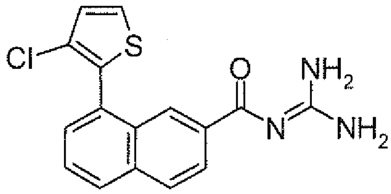 |
| 50 | HCl | 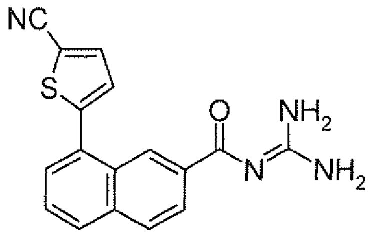 |
| 51 | HCl | 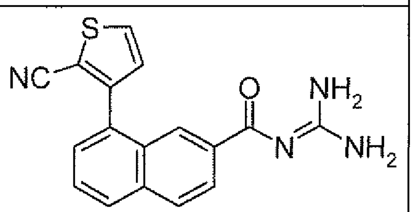 |
| 52 | 2HCl | 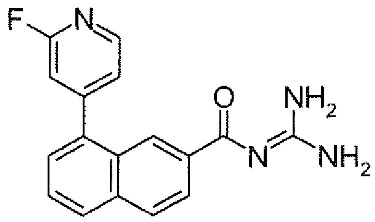 |
| 53 | 2HCl | 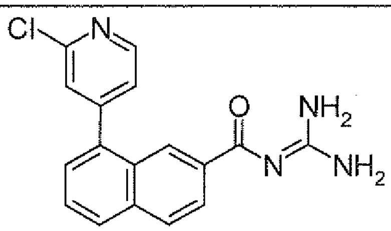 |
| 54 | 2HCl | 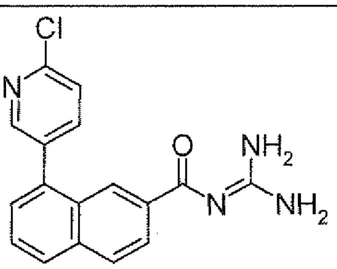 |
| 55 | 2HCl | 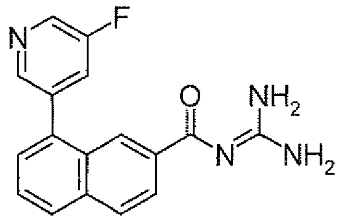 |
| 56 | HCl | 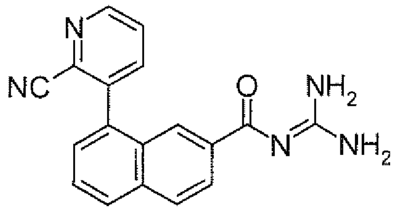 |
| 57 | HCl | 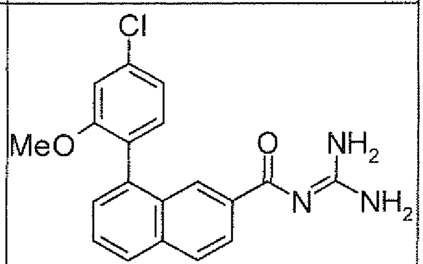 |
| 58 | HCl | 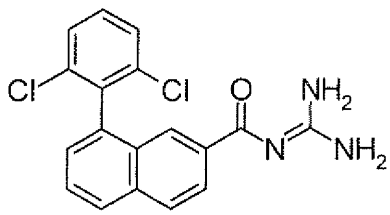 |
| 59 | HCl | 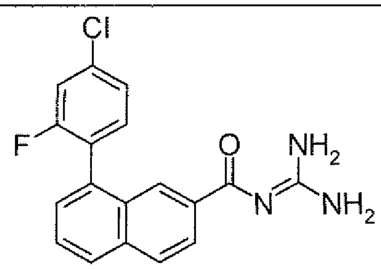 |
| 60 | HCl | 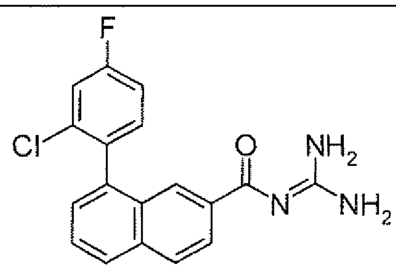 |

[Table 52]

| Ex | Sal | Str |
|---|---|---|
| 61 | HCl | 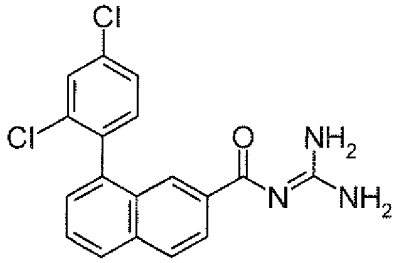 |
| 62 | HCl | 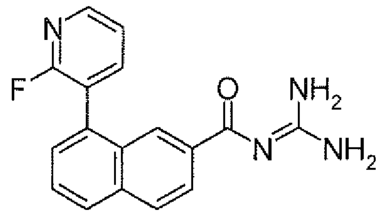 |
| 63 | 2HCl | 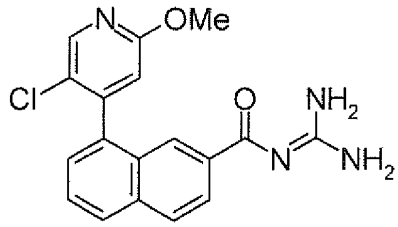 |
| 64 | HCl | 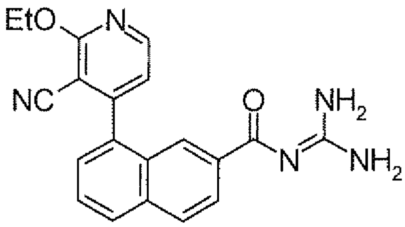 |
| 65 | HCl | 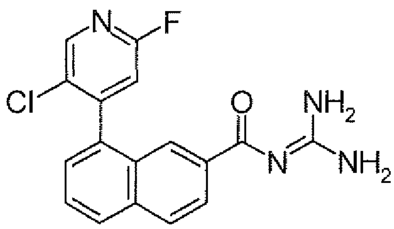 |
| 66 | HCl | 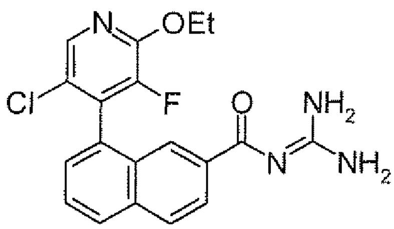 |
| 67 | HCl | 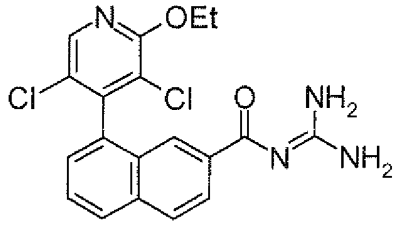 |
| 68 | HCl | 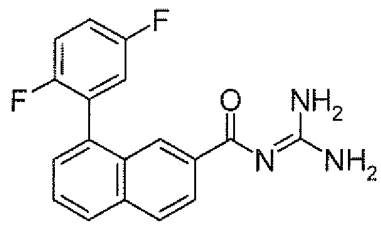 |
| 69 | HCl | 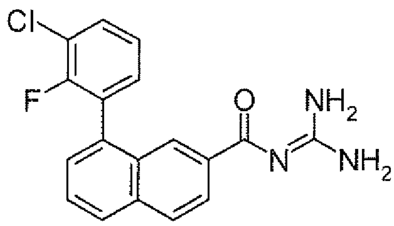 |
| 70 | HCl | 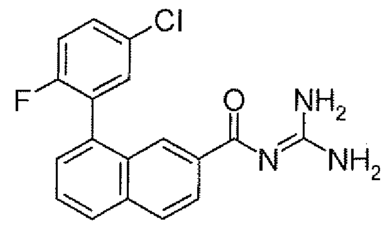 |
| 71 | HCl | 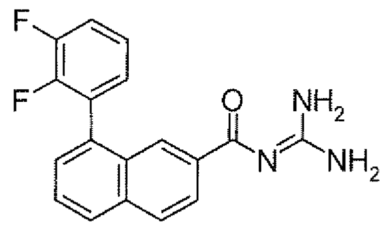 |
| 72 | HCl | 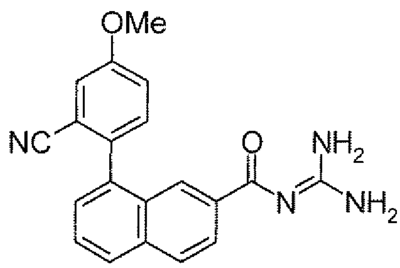 |

[Table 53]

| Ex | Sal | Str |
|---|---|---|
| 73 | 2HCl | 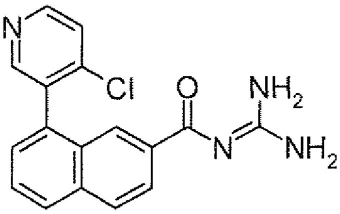 |
| 74 | 2HCl | 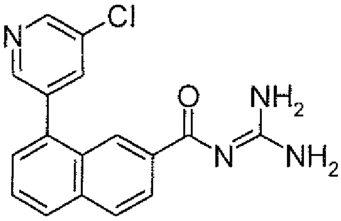 |
| 75 | HCl | 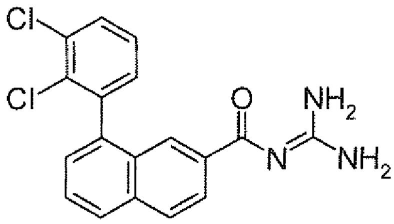 |
| 76 | HCl | 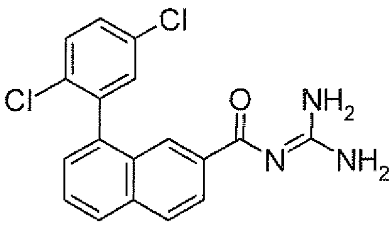 |
| 77 | HCl | 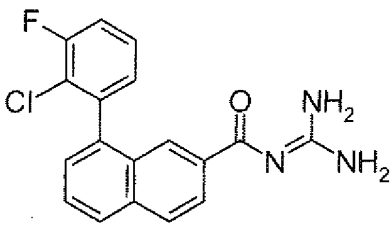 |
| 78 | HCl | 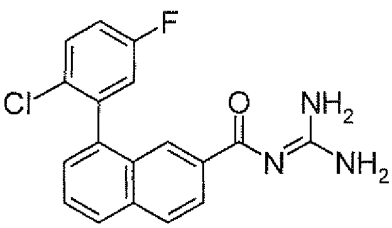 |
| 79 | HCl | 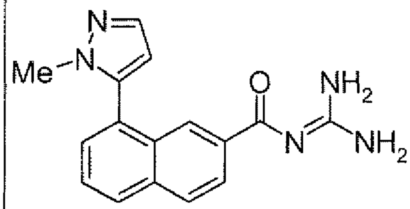 |
| 80 | HCl | 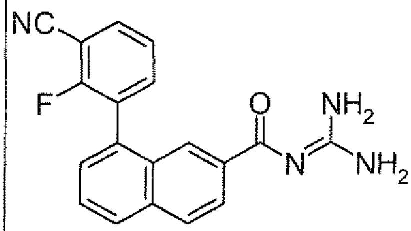 |
| 81 | HCl | 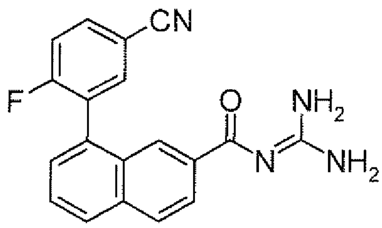 |
| 82 | HCl | 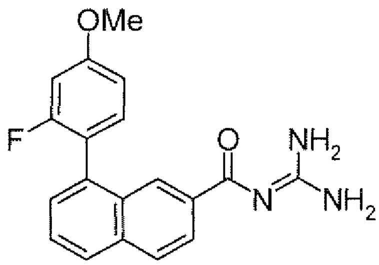 |
| 83 | HCl | 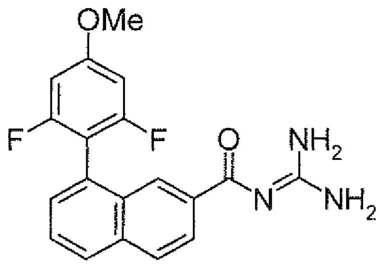 |
| 84 | HCl | 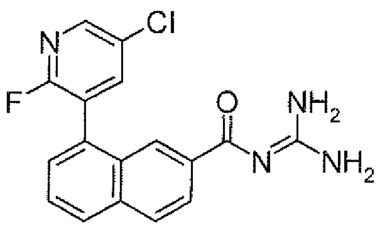 |

[Table 54]

| Ex | Sal | Str |
|---|---|---|
| 85 | HCl | 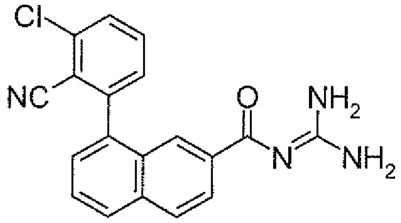 |
| 86 | 2HCl | 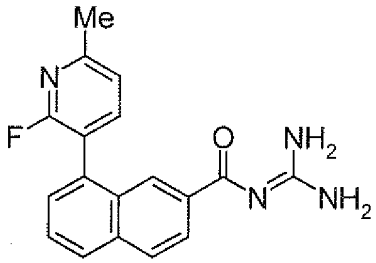 |
| 87 | HCl | 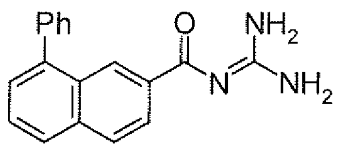 |
| 88 | HCl | 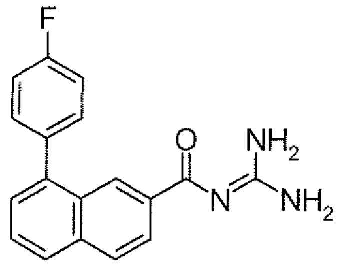 |
| 89 | HCl | 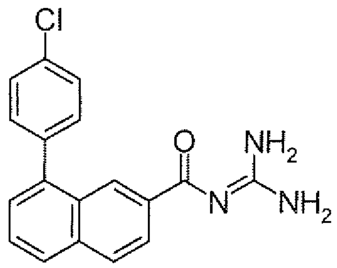 |
| 90 | HCl | 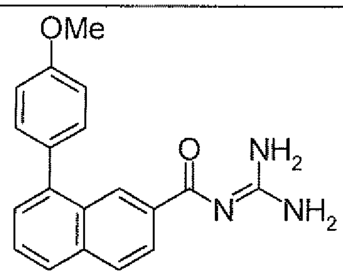 |
| 91 | HCl | 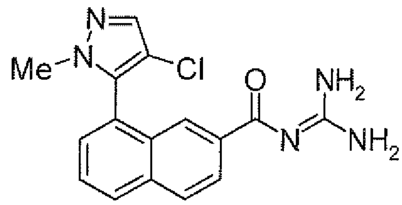 |
| 92 | HCl | 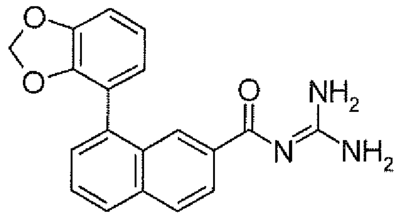 |
| 93 | HCl | 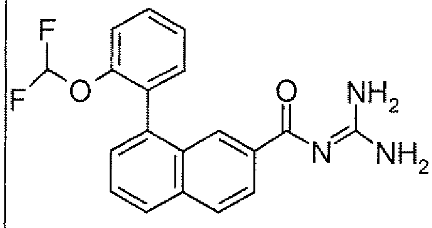 |
| 94 | HCl | 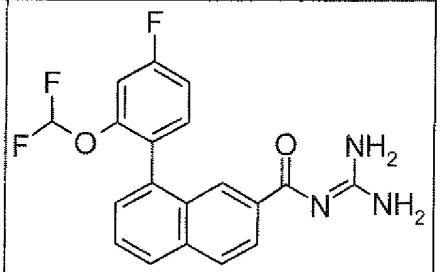 |
| 95 | HCl | 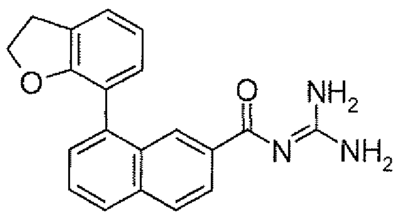 |
| 96 | HCl | 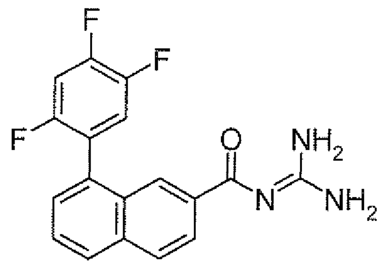 |

[Table 55]

| Ex | Sal | Str |
|---|---|---|
| 97 | 2HCl | 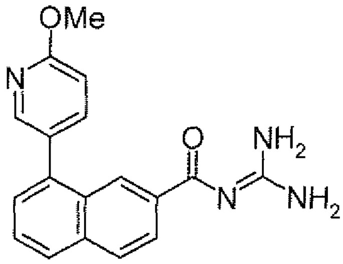 |
| 98 | 2HCl | 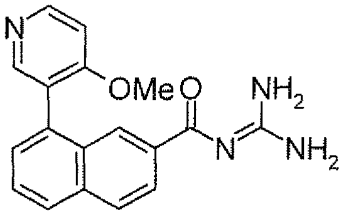 |
| 99 | HCl | 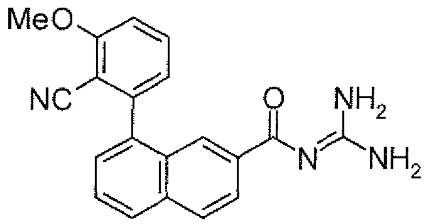 |
| 100 | 2HCl | 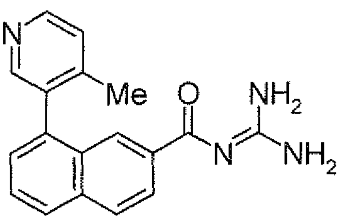 |
| 101 | HCl | 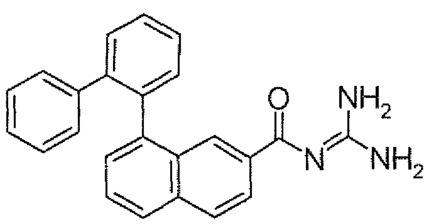 |
| 102 | HCl | 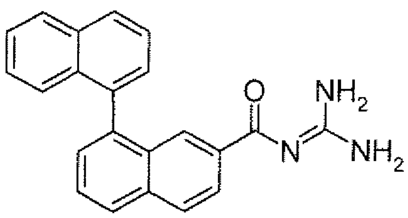 |
| 103 | HCl | 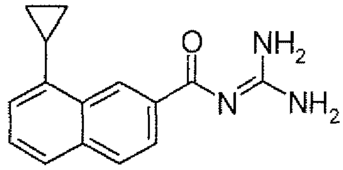 |
| 104 | HCl | 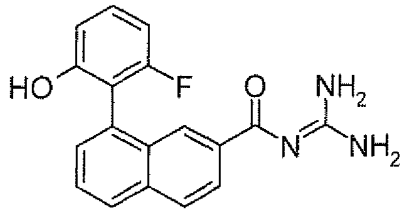 |
| 105 | HCl | 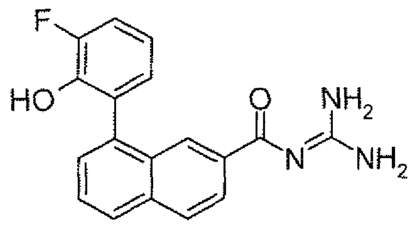 |
| 106 | HCl | 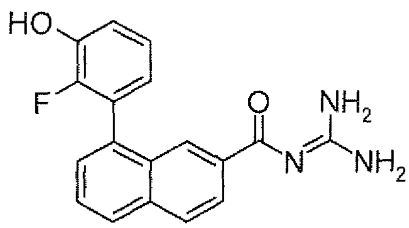 |
| 107 | HCl | 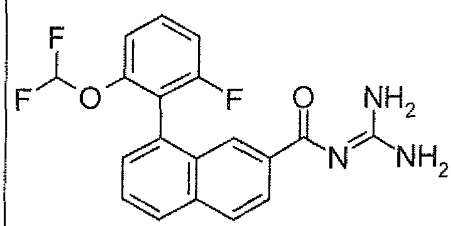 |
| 108 | 2HCl | 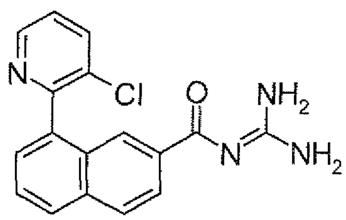 |

[Table 56]

| Ex | Sal | Str |
|---|---|---|
| 109 | 2HCl | 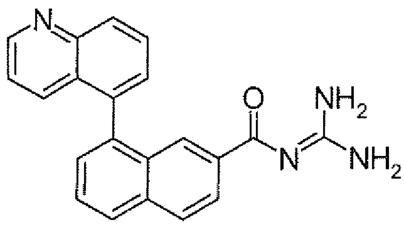 |
| 110 | HCl | 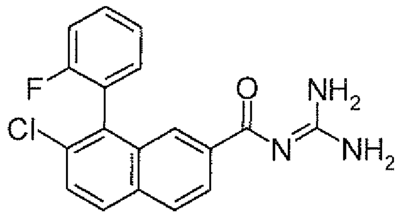 |
| 111 | 2HCl | 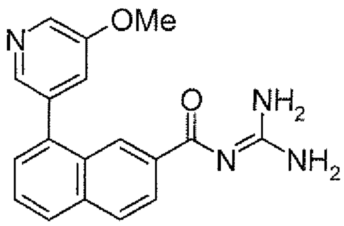 |
| 112 | 2HCl | 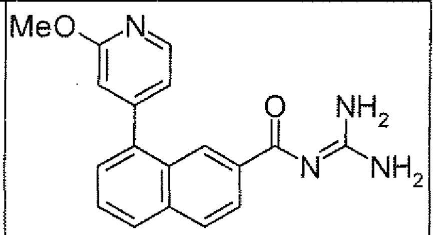 |
| 113 | 2HCl | 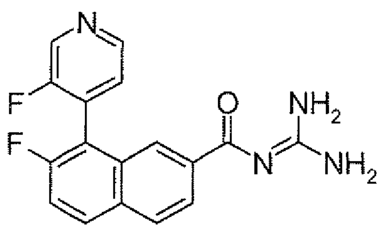 |
| 114 | 2HCl | 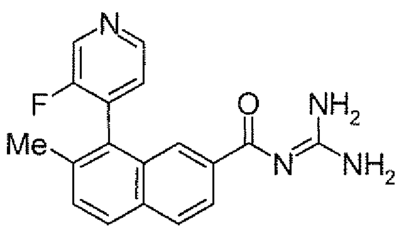 |
| 115 | HCl | 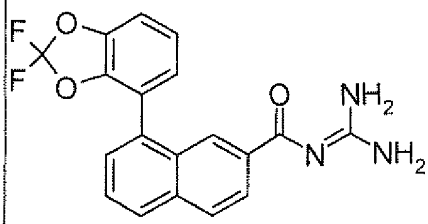 |
| 116 | HCl | 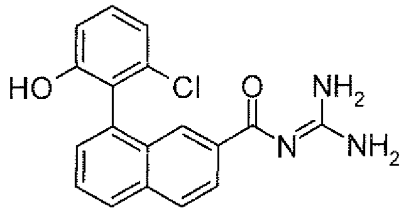 |
| 117 | HCl | 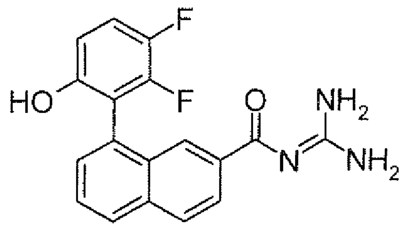 |
| 118 | HCl | 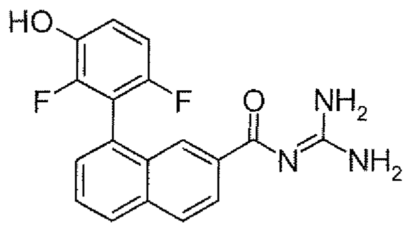 |
| 119 | HCl | 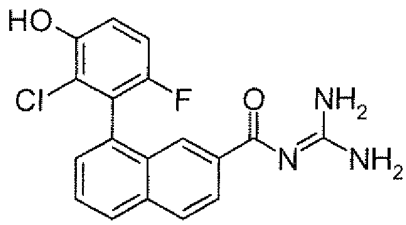 |
| 120 | HCl | 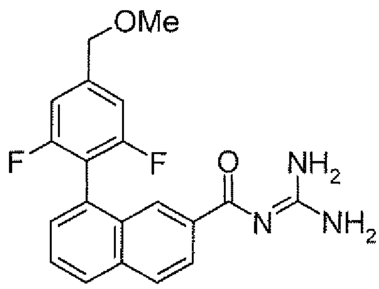 |

[Table 57]

| Ex | Sal | Str |
|---|---|---|
| 121 | Fum | 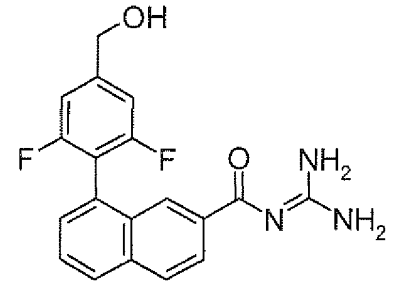 |
| 122 | 2HCl | 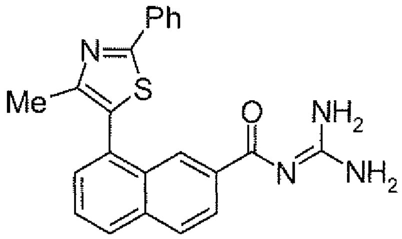 |
| 123 | 2HCl | 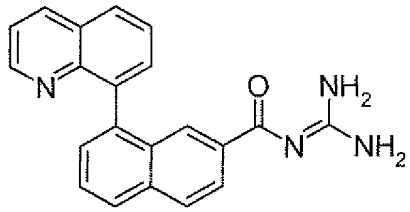 |
| 124 | HCl | 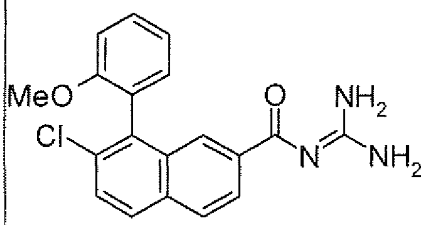 |
| 125 | HCl | 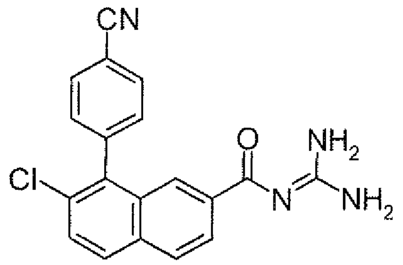 |
| 126 | HCl | 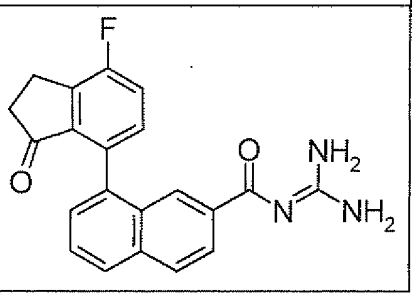 |
| 127 | HCl | 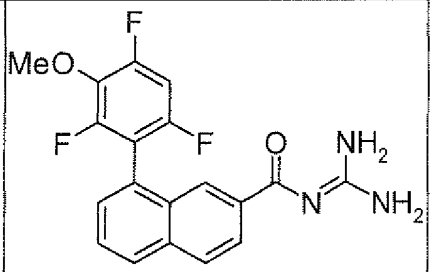 |
| 128 | 2HCl | 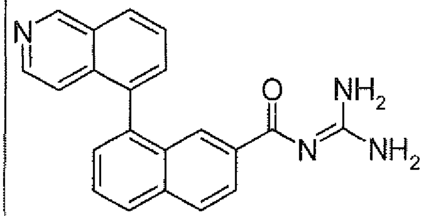 |
| 129 | 2HCl | 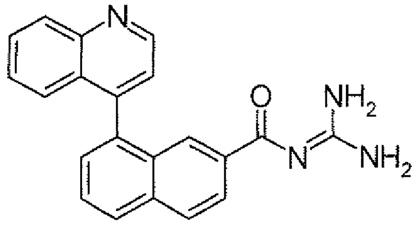 |
| 130 | 2HCl | 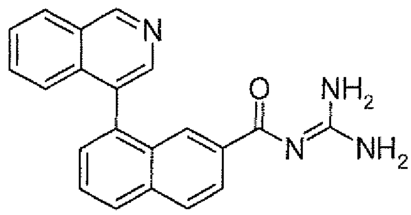 |
| 131 | HCl | 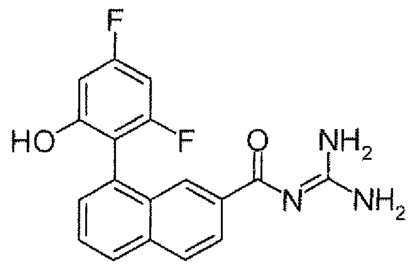 |
| 132 | HCl | 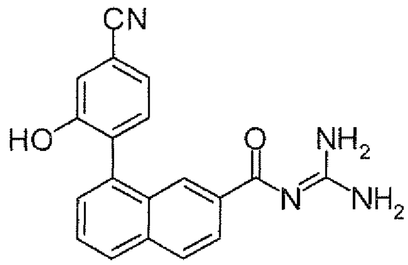 |

[Table 58]

| Ex | Sal | Str |
|---|---|---|
| 133 | 2HCl | 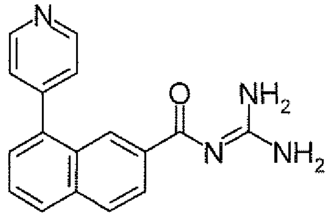 |
| 134 | HCl | 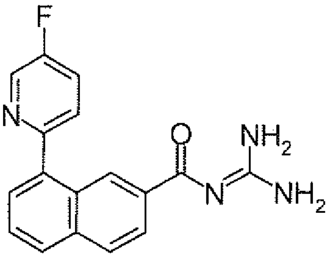 |
| 135 | HCl | 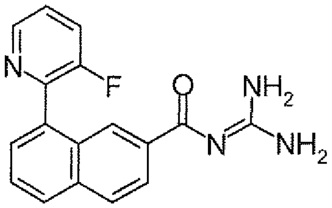 |
| 136 | HCl | 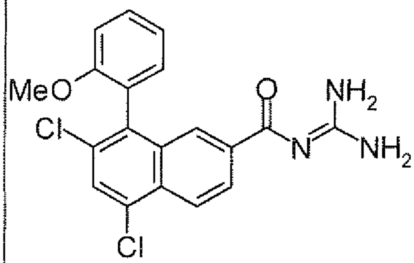 |
| 137 | | 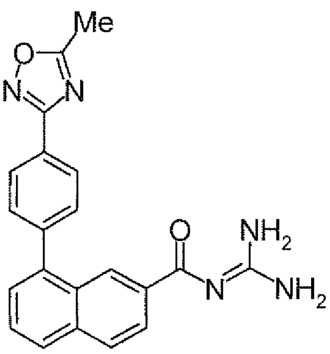 |
| 138 | HCl | 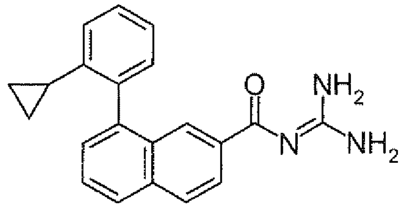 |
| 139 | HCl | 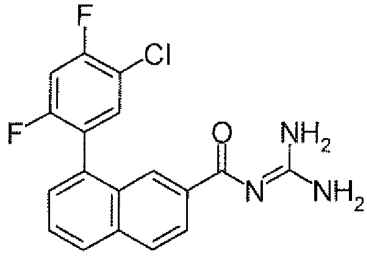 |
| 140 | HCl | 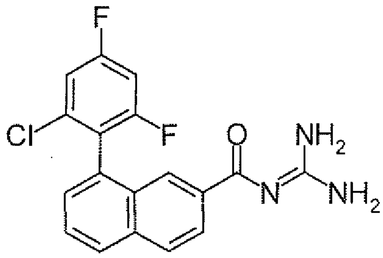 |
| 141 | 2HCl | 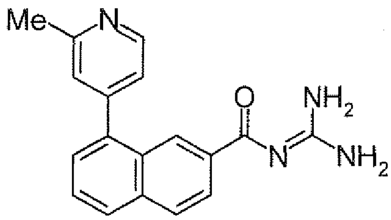 |
| 142 | HCl | 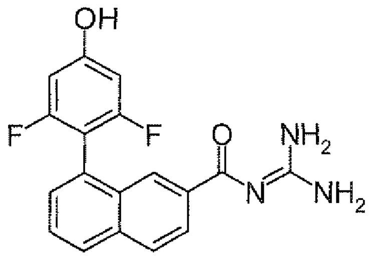 |
| 143 | HCl | 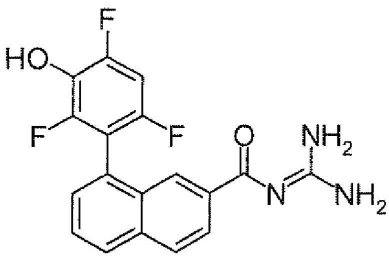 |

[Table 59]

| Ex | Sal | Str |
|---|---|---|
| 144 | Fum | 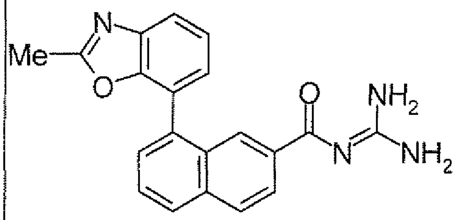 |
| 145 | HCl | 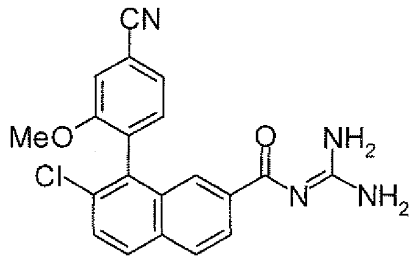 |
| 146 | 2HCl | 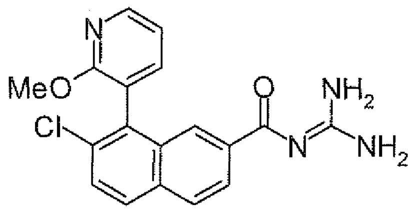 |
| 147 | 2HCl | 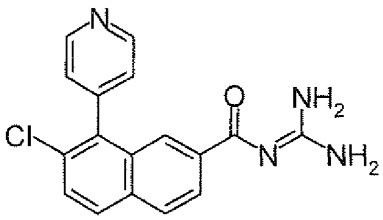 |
| 148 | HCl | 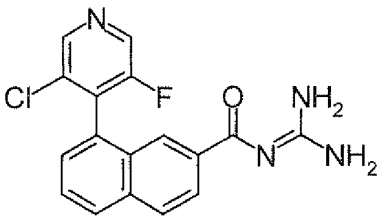 |
| 149 | HCl | 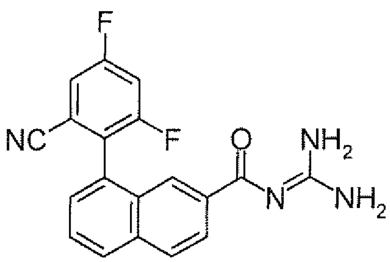 |
| 150 | HCl | 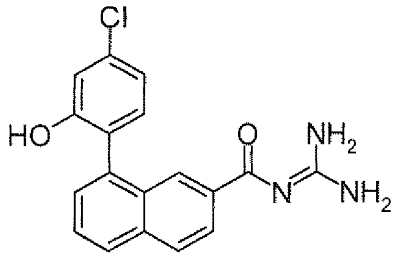 |
| 151 | HCl | 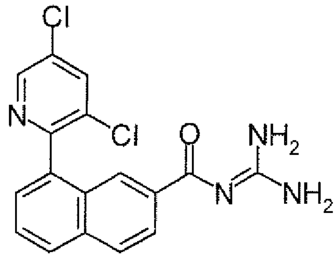 |
| 152 | HCl | 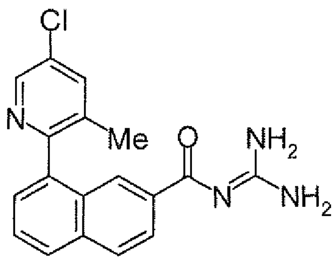 |
| 153 | 2HCl | 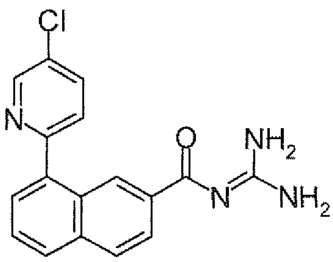 |
| 154 | HCl | 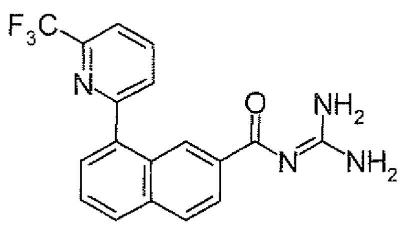 |

[Table 60]

| Ex | Sal | Str |
|---|---|---|
| 155 | Fum | 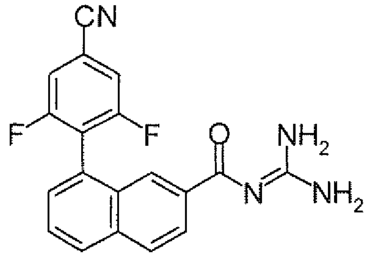 |
| 156 | 2HCl | 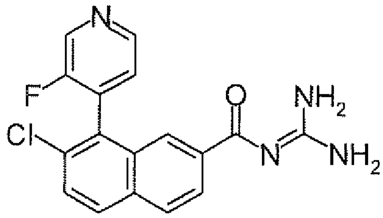 |
| 157 | 2HCl | 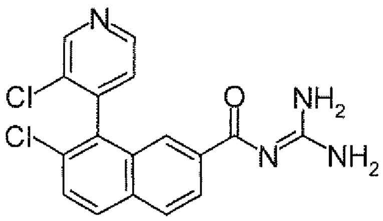 |
| 158 | 2HCl | 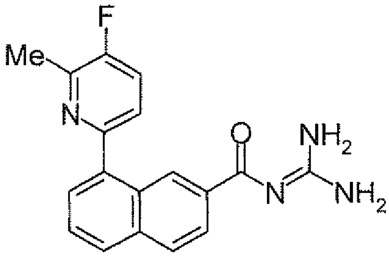 |
| 159 | HCl | 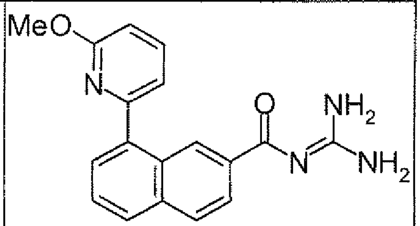 |
| 160 | HCl | 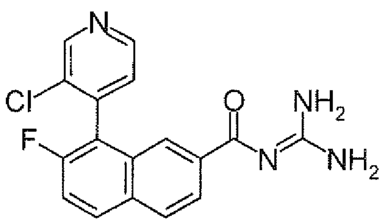 |

| Ex | Sal | Str |
|---|---|---|
| 161 | HCl | 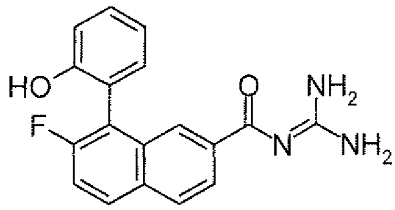 |
| 162 | HCl | 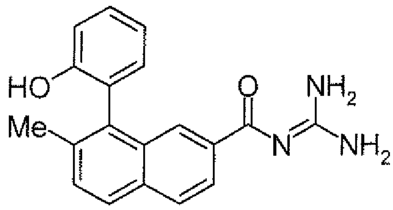 |
| 163 | HCl | 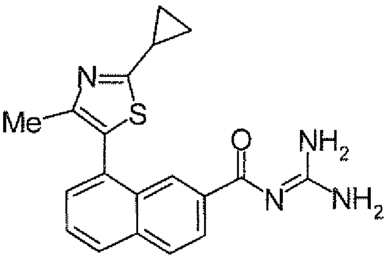 |
| 164 | HCl | 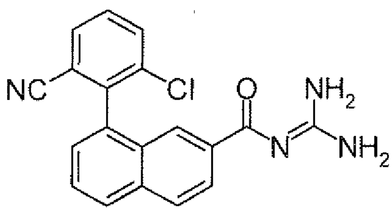 |
| 165 | 2HCl | 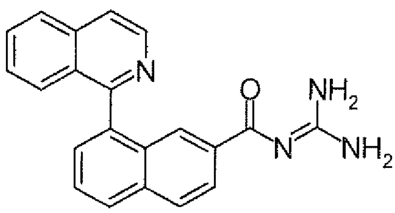 |
| 166 | 2HCl | 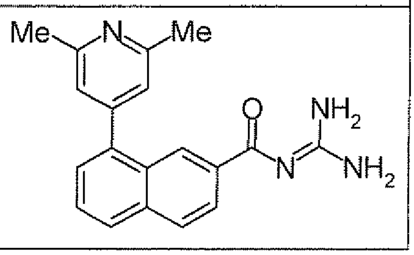 |

[Table 61]

| Ex | Sal | Str |
|---|---|---|
| 167 | 2HCl | 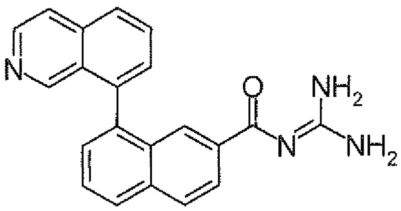 |
| 168 | HCl | 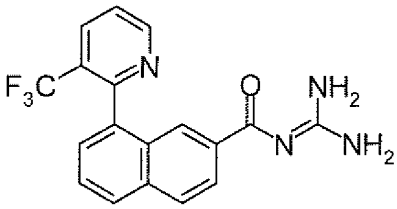 |
| 169 | HCl | 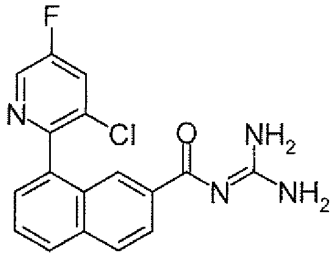 |
| 170 | 2Fum | 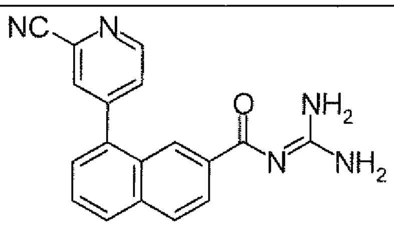 |
| 171 | 2HCl | 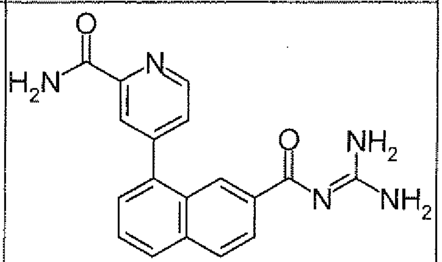 |
| 172 | 2HCl | 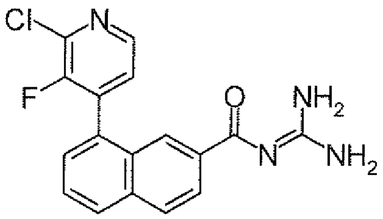 |
| 173 | HCl | 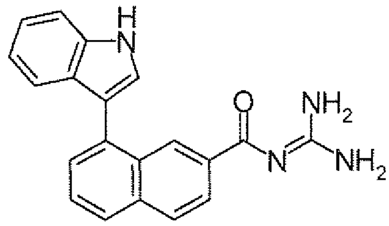 |
| 174 | 2HCl | 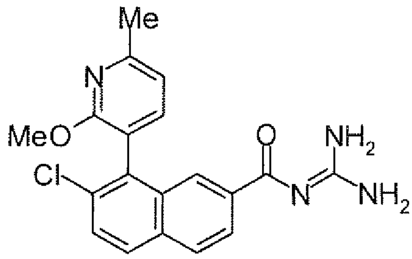 |
| 175 | HCl | 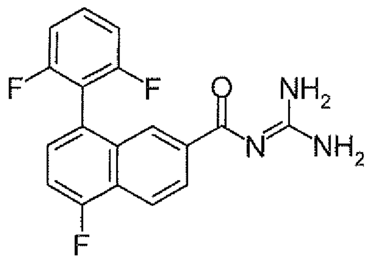 |
| 176 | HCl | 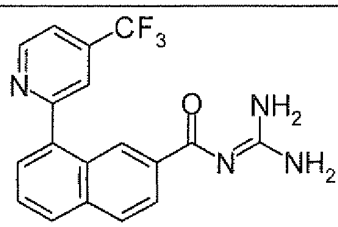 |
| 177 | HCl | 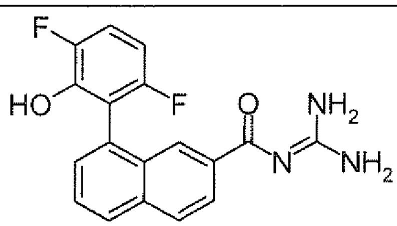 |
| 178 | HCl | 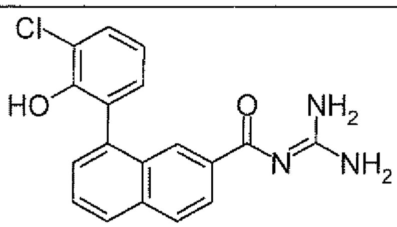 |

[Table 62]

| Ex | Sal | Str |
|---|---|---|
| 179 | HCl | 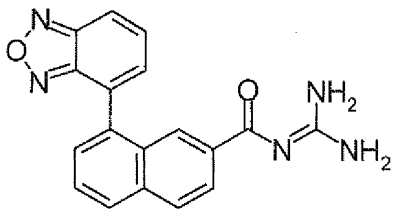 |
| 180 | HCl | 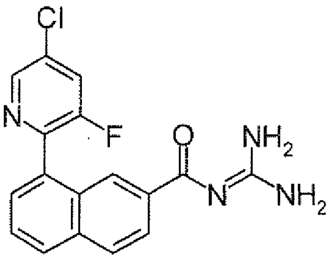 |
| 181 | 2HCl | 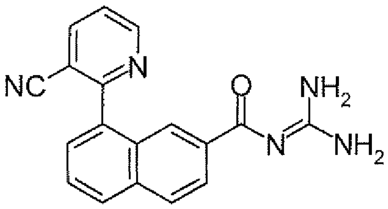 |
| 182 | 2HCl | 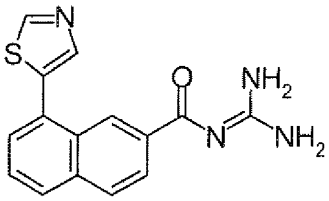 |
| 183 | HCl | 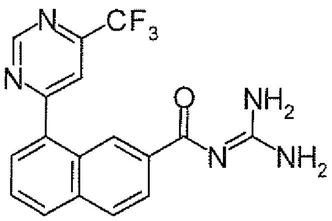 |
| 184 | 2HCl | 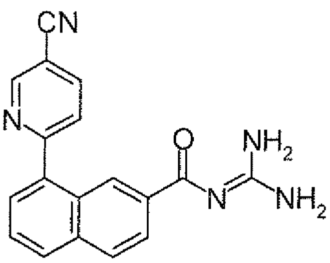 |
| 185 | 2HCl | 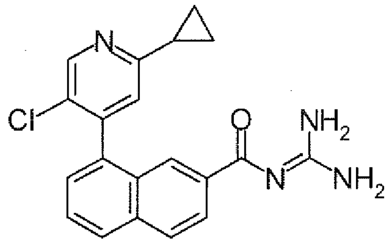 |
| 186 | 2HCl | 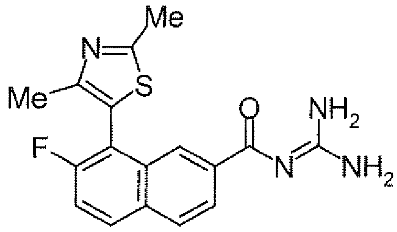 |
| 187 | HCl | 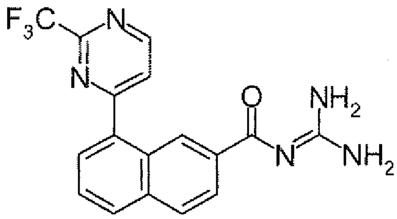 |
| 188 | HCl | 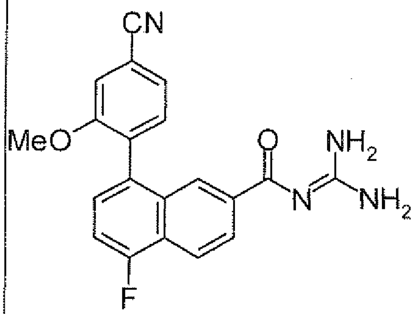 |
| 189 | 2HCl | 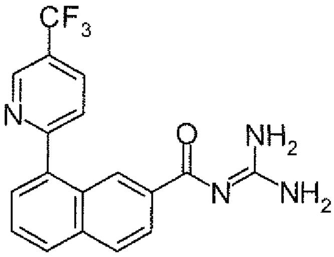 |

[Table 63]

| Ex | Sal | Str |
|---|---|---|
| 190 | HCl | 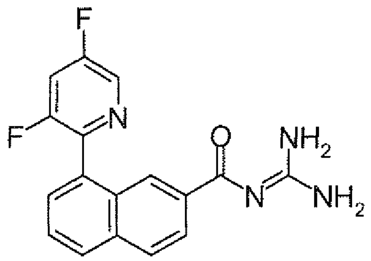 |
| 191 | HCl | 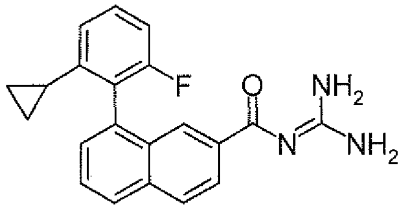 |
| 192 | 2HCl | 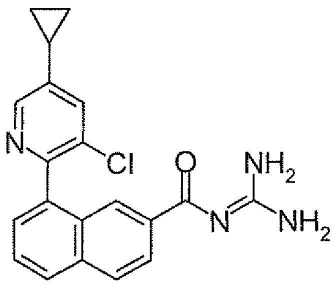 |
| 193 | Fum | 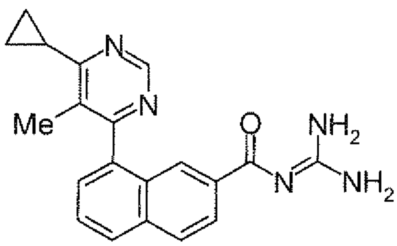 |
| 194 | HCl | 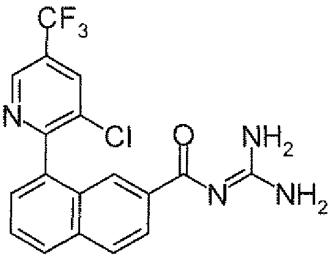 |
| 195 | 2HCl | 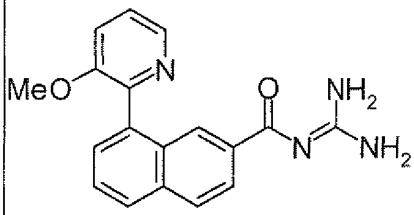 |
| 196 | HCl | 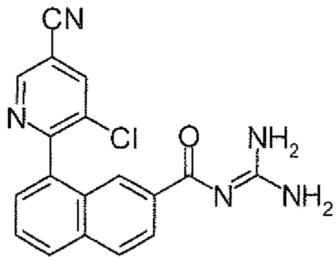 |
| 197 | 2HCl | 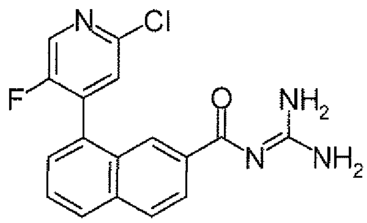 |
| 198 | 2HCl | 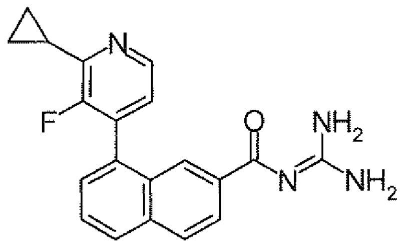 |
| 199 | 2HCl | 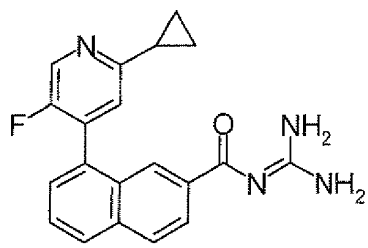 |
| 200 | 2HCl | 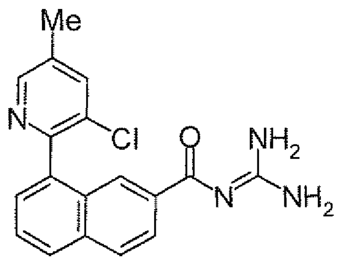 |

[Table 64]

| Ex | Sal | Str | Ex | Sal | Str |
|---|---|---|---|---|---|
| 201 | 2HCl | 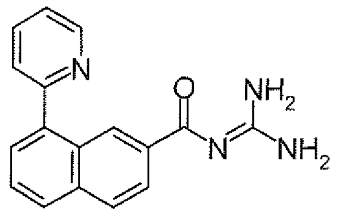 | 202 | 2HCl | 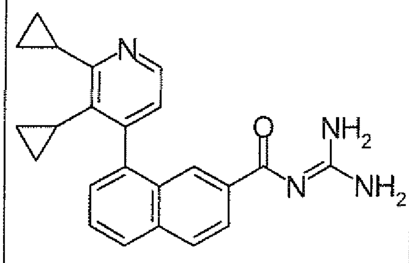 |

[Table 65]

| Ex | Dat | | Ex | Dat | | Ex | Dat |
|---|---|---|---|---|---|---|---|
| 1 | FAB+: 342 | | 31 | ESI+: 327 | | 61 | ESI+: 358 |
| 2 | FAB+: 315 | | 32 | ESI+: 354 | | 62 | ESI+: 309 |
| 3 | FAB+: 345 | | 33 | ESI+: 349 | | 63 | ESI+: 355 |
| 4 | FAB+: 338 | | 34 | ESI+: 338 | | 64 | ESI+; 360 |
| 5 | ESI+: 350 | | 35 | ESI+: 326 | | 65 | ESI+: 343 |
| 6 | ESI+: 308 | | 36 | ESI+: 326 | | 66 | ESI+: 387 |
| 7 | ESI+: 324 | | 37 | ESI+: 359 | | 67 | ESI+: 403 |
| 8 | ESI+: 321 | | 38 | ESI+: 359 | | 68 | ESI+: 326 |
| 9 | ESI+: 333 | | 39 | ESI+: 333 | | 69 | ESI+: 342 |
| 10 | ESI+: 329 | | 40 | ESI+: 338 | | 70 | ESI+: 342 |
| 11 | ESI+: 322 | | 41 | ESI+: 338 | | 71 | ESI+: 326 |
| 12 | ESI+: 334 | | 42 | ESI+: 356 | | 72 | ESI+: 345 |
| 13 | ESI+: 306 | | 43 | ESI+: 360 | | 73 | ESI+: 325 |
| 14 | ESI+: 344 | | 44 | ESI+: 325 | | 74 | ESI+: 325 |
| 15 | ESI+: 345 | | 45 | ESI+: 359 | | 75 | ESI+: 358 |
| 16 | ESI+: 333 | | 46 | ESI+: 296 | | 76 | ESI+: 358 |
| 17 | ESI+: 325 | | 47 | ESI+: 296 | | 77 | ESI+: 342 |
| 18 | ESI+: 338 | | 48 | ESI+: 310 | | 78 | ESI+: 342 |
| 19 | ESI+: 325 | | 49 | ESI+: 330 | | 79 | ESI+: 294 |
| 20 | ESI+: 315 | | 50 | ESI+: 321 | | 80 | ESI+: 333 |
| 21 | ESI+: 291 | | 51 | ESI+: 321 | | 81 | ESI+: 333 |
| 22 | FAB+: 296 | | 52 | ESI+: 309 | | 82 | ESI+: 338 |
| 23 | ESI+: 309 | | 53 | ESI+: 325 | | 83 | ESI+: 356 |
| 24 | ESI+: 305 | | 54 | ESI+: 325 | | 84 | ESI+: 343 |
| 25 | ESI+: 309 | | 55 | ESI+: 309 | | 85 | ESI+: 349 |

(continued)

| Ex | Dat | | Ex | Dat | | Ex | Dat |
|---|---|---|---|---|---|---|---|
| 26 | ESI+: 333 | | 56 | ESI+: 316 | | 86 | ESI+: 323 |
| 27 | ESI+: 338 | | 57 | ESI+: 354 | | 87 | ESI+: 290 |
| 28 | ESI+: 348 | | 58 | ESI+: 358 | | 88 | ESI+: 308 |
| 29 | ESI+: 333 | | 59 | ESI+: 342 | | 89 | ESI+: 324 |
| 30 | ESI+: 345 | | 60 | ESI+: 342 | | 90 | ESI+: 320 |

[Table 66]

| Ex | Dat | | Ex | Dat | | Ex | Dat |
|---|---|---|---|---|---|---|---|
| 91 | ESI+: 328 | | 121 | ESI+: 356 | | 151 | ESI+: 359, 361 |
| 92 | ESI+: 334 | | 122 | ESI+: 387 | | 152 | ESI+: 339, 341 |
| 93 | ESI+: 356 | | 123 | ESI+: 341 | | 153 | ESI+: 325, 327 |
| 94 | ESI+: 374 | | 124 | ESI+: 354 | | 154 | ESI+: 359 |
| 95 | ESI+: 332 | | 125 | ESI+: 349 | | 155 | ESI+: 351 |
| 96 | ESI+: 344 | | 126 | ESI+: 362 | | 156 | ESI+: 343, 345 |
| 97 | ESI+: 321 | | 127 | ESI+: 374 | | 157 | ESI+: 359, 361 |
| 98 | ESI+: 321 | | 128 | ESI+: 341 | | 158 | ESI+: 323 |
| 99 | ESI+: 345 | | 129 | ESI+: 341 | | 159 | ESI+: 321 |
| 100 | ESI+: 305 | | 130 | ESI+: 341 | | 160 | ESI+: 343 |
| 101 | ESI+: 366 | | 131 | ESI+: 342 | | 161 | ESI+: 324 |
| 102 | ESI+: 340 | | 132 | ESI+: 331 | | 162 | ESI+: 320 |
| 103 | ESI+: 254 | | 133 | ESI+: 291 | | 163 | ESI+: 351 |
| 104 | ESI+: 324 | | 134 | ESI+: 309 | | 164 | ESI+: 349 |
| 105 | ESI+: 324 | | 135 | ESI+: 309 | | 165 | ESI+: 341 |
| 106 | ESI+: 324 | | 136 | ESI+: 388 | | 166 | ESI+: 319 |
| 107 | ESI+: 374 | | 137 | ESI+: 372 | | 167 | ESI+: 341 |
| 108 | ESI+: 325 | | 138 | ESI+: 330 | | 168 | ESI+: 359 |
| 109 | ESI+: 341 | | 139 | ESI+: 360 | | 169 | ESI+: 343 |
| 110 | ESI+: 342 | | 140 | ESI+: 360 | | 170 | ESI+: 316 |
| 111 | ESI+: 321 | | 141 | ESI+: 305 | | 171 | ESI+: 334 |
| 112 | ESI+: 321 | | 142 | A/E+: 342 | | 172 | ESI+: 343 |
| 113 | ESI+: 327 | | 143 | ESI+: 360 | | 173 | ESI+: 329 |
| 114 | ESI+: 323 | | 144 | ESI+: 345 | | 174 | ESI+: 369, 371 |
| 115 | ESI+: 370 | | 145 | ESI+: 379 | | 175 | ESI+: 344 |
| 116 | ESI+: 340 | | 146 | ESI+: 355 | | 176 | ESI+: 359 |
| 117 | ESI+: 342 | | 147 | ESI+: 325, 327 | | 177 | ESI+: 342 |
| 118 | ESI+: 342 | | 148 | ESI+: 343 | | 178 | ESI+: 340 |
| 119 | ESI+: 358 | | 149 | ESI+: 351 | | 179 | ESI+: 332 |

(continued)

| Ex | Dat | | Ex | Dat | | Ex | Dat |
|---|---|---|---|---|---|---|---|
| 120 | ESI+: 370 | | 150 | ESI+: 340 | | 180 | ESI+: 343 |

[Table 67]

| Ex | Dat | | Ex | Dat | | Ex | Dat |
|---|---|---|---|---|---|---|---|
| 181 | ESI+: 316 | | 189 | ESI+: 359 | | 197 | ESI+: 343, 345 |
| 182 | ESI+: 297 | | 190 | ESI+: 327 | | 198 | ESI+: 349 |
| 183 | ESI+: 360 | | 191 | ESI+: 348 | | 199 | ESI+: 349 |
| 184 | ESI+: 316 | | 192 | ESI+: 365 | | 200 | ESI+: 339 |
| 185 | ESI+: 365, 367 | | 193 | ESI+: 346 | | 201 | ESI+: 291 |
| 186 | ESI+: 343 | | 194 | ESI+: 393, 395 | | 202 | ESI+: 371 |
| 187 | ESI+: 360 | | 195 | ESI+: 321 | | | |
| 188 | ESI+: 363 | | 196 | ESI+: 350 | | | |

[Table 68]

| Ex | Dat (NMR-DMSO-$d_6$) |
|---|---|
| 1 | 7.43-7.51 (1H, m), 7.56-7.68 (3H, m), 7.81-7.87 (1H, m), 8.03 (1H, s), 8.20 (1H, d, J = 8.3 Hz), 8.24-8.31 (2H, m), 8.49 (2H, brs), 8.61 (2H, brs), 11.95 (1H, brs) |
| 3 | 3.72 (3H, s), 7.48-7.56 (2H, m), 7.61 (1H, dd, J = 7.6, 1.6 Hz), 7.72 (1H, d, J = 1.2 Hz), 7.75-7.81 (1H, m), 8.07-8.14 (2H, m), 8.15-8.23 (2H, m), 8.37-8.62 (2H, m), 11.79 (1H, brs) |
| 8 | 3.76 (3H, s), 7.19-7.22 (1H, m), 7.56 (1H, d, J = 8 Hz), 7.76-7.79 (2H, m), 8.10-8.12 (1H, d, J = 8 Hz), 8.17-8.21 (2H, m), 8.30-8.36 (2H, m), 8.57 (2H, brs), 8.75 (2H, brs), 12.11 (1H, brs) |
| 10 | 2.22 (3H, s), 7.53-7.59 (2H, m), 7.71 (1H, d, J = 8.6 Hz), 7.90-7.97 (1H, m), 8.00-8.10 (3H, m), 8.13-8.23 (2H, m), 8.48 (2H, brs), 8.66 (2H, brs), 11.97 (1H, brs) |
| 14 | 7.39-7.51 (2H, m), 7.70 (1H, d, J = 7.2 Hz), 7.79-7.87 (1H, m), 8.17-8.30 (4H, m), 8.50 (2H, brs), 8.65 (2H, brs), 12.04 (1H, brs) |
| 26 | 7.76-7.91 (4H, m), 7.96-7.99 (1H, m), 8.13 (1H, brs), 8.25-8.34 (3H, m), 8.54 (2H, brs), 8.71 (2H, brs), 12.15 (1H, brs) |
| 29 | 7.56 (1H, d, J = 7.8 Hz), 7.67-7.75 (2H, m), 7.83-7.88 (1H, m), 7.92-7.98 (1H, m), 8.21-8.31 (4H, m), 8.48 (2H, brs), 8.64 (2H, brs), 12.05 (1H, brs) |
| 31 | 7.79 (1H, d, J = 6.8 Hz), 7.85-7.90 (1H, m), 8.27 (1H, brs), 8.29 (3H, brs), 8.54 (2H, brs), 8.77 (4H, brs), 12.28 (1H, brs) |
| 34 | 3.62 (3H, s), 7.17-7.25 (2H, m), 7.31-7.37 (1H, m), 7.52-7.55 (1H, m), 7.74-7.79 (1H, m), 8.09 (1H, d, J=8.4Hz), 8.16-8.26 (3H, m), 8.49 (2H, brs), 8.62 (2H, brs), 11.87 (1H, brs) |
| 36 | 7.32-7.38 (2H, m), 7.62-7.71 (2H, m), 7.81-7.87 (1H, m), 8.25-8.30 (3H, m), 8.30-8.36 (1H, m), 8.55 (2H, brs), 8.69 (2H, brs), 12.08 (1H, brs) |
| 38 | 7.62-7.67 (1H, m), 7.79 (1H, s), 7.81-7.87 (1H, m), 8.17-8.28 (4H, m), 8.50 (2H, brs), 8.72 (1H, s), 8.74 (2H, brs) |
| 42 | 3.68 (3H, s), 7.01-7.10 (2H, m), 7.55 (1H, d, J = 7.0 Hz), 7.78 (1H, t, J = 7.7 Hz), 8.07-8.15 (2H, m), 8.21 (2H, brs), 8.48 (2H, brs), 8.61 (2H, brs), 11.90 (1H, brs) |
| 44 | 7.61-7.65 (2H, m), 7.82-7.87 (1H, m), 8.12 (1H, brs), 8.20-8.32 (3H, m), 8.52 (2H, brs), 8.68-8.79 (3H, m), 8.89 (1H, s), 12.14 (1H, brs) |

[Table 69]

| Ex | Dat (NMR-DMSO-$d_6$) |
|---|---|
| 45 | 7.63 (1H, d, J = 7.0 Hz), 7.85-7.90 (1H, m), 8.07 (1H, s), 8.22-8.30 (3H, m), 8.52 (2H, brs), 8.77 (2H, brs), 8.86 (2H, brs), 12.23 (1H, brs) |
| 57 | 3.68 (3H, s), 7.17-7.21 (1H, m), 7.30-7.33 (2H, m), 7.49-7.52 (1H, m), 7.73-7.78 (1H, m), 8.08 (1H, d, J = 8.3 Hz), 8.2-8.21 (2H, m), 8.22-8.28 (1H, m), 8.51 (2H, brs), 8.68 (2H, brs), 11.98 (1H, brs) |
| 60 | 7.39-7.45 (1H, m), 7.52-7.60 (2H, m), 7.66-7.72 (1H, m), 7.78-7.83 (1H, m), 8.09 (1H, brs), 8.16 (1H, d, J = 8.4 Hz), 8.21-8.31 (2H, m), 8.52 (2H, brs), 8.69 (2H, brs), 12.04 (1H, brs) |
| 62 | 7.56-7.62 (1H, m), 7.67-7.71 (1H, m), 7.81-7.86 (1H, m), 8.12-8.32 (5H, m), 8.40-8.44 (1H, m), 8.54 (2H, brs), 8.69 (2H, brs), 12.12 (1H, brs) |
| 68 | 7.40-7.53 (3H, m), 7.63-7.79 (1H, m), 7.79-7.85 (1H, m), 8.18 (1H, d, J=8.4Hz), 8.22-8.34 (3H, m), 8.53 (2H, brs), 8.68 (2H, brs), 12.07 (1H, brs) |
| 109 | 7.55-7.61 (1H, m), 7.66-7.71 (1H, m), 7.77-7.81 (1H, m), 7.86-7.92 (2H, m), 8.00-8.09 (2H, m), 8.22-8.27 (2H, m), 8.28-8.33(2H, m), 8.42 (2H, brs), 8.59 (2H, brs), 9.06-9.10 (1H, m) |
| 113 | 7.72-7.77 (1H, m), 7.84 (1H, t, J=9.2Hz), 8.20 (1H, s), 8.23-8.28 (1H, m), 8.29-8.39 (2H, m), 8.54 (2H, brs), 8.63-8.80 (3H, brs), 8.86 (1H, s), 12.23 (1H, brs) |
| 125 | 7.65 (1H, d, J = 8 Hz), 7.90 (1H, d, J = 8 Hz), 7.95 (1H, s), 8.07 (2H, d, J= 8 Hz), 8.21 (2H, d, J = 8 Hz), 8.26-8.32 (2H, m), 8.51 (2H, brs), 8.68 (2H, brs), 12.13 (1H, brs) |
| 151 | 7.70 (1H, d), 7.84 (1H, t), 8.20-8.27 (4H, m), 8.45 (2H, brs), 8.49 (1H, d, J = 2 Hz), 8.64 (2H, brs), 8.81 (1H, d, J = 2 Hz), 12.09 (1H, brs) |
| 169 | 7.67 (1H, d, J = 8 Hz), 7.83 (1H, t, J = 8 Hz), 8.18-8.20 (3H, m), 8.25 (1H, d, J = 8 Hz), 8.33-8.36 (1H, m), 8.46 (2H, brs), 8.60 (2H, brs), 8.79-8.80 (1H, m), 11.95 (1H, brs) |
| 175 | 7.35 (2H, t, J = 8 Hz), 7.63-7.75 (3H, m), 8.17 (1H, s), 8.37 (2H, s), 8.59 (4H, brs), 12.08 (1H, brs) |

(Test Examples)

Pharmacological activities of compound of the present invention were confirmed by the following tests.

Test Example 1: Acquisition of HEK293 cells for forced expressions of a human 5-$HT_{5A}$ receptor

The ORF (open reading frame; protein coding region) of a human 5-$HT_{5A}$ receptor (Genbank AF498985) was cloned from a human hippocampus cDNA library, and then inserted into a pCR2.1 vector (Invitrogen), and *Escherichia coli* containing the plasmid was cultured in a large amount. Next, the full-length cDNA sequence of the human 5-$HT_{5A}$ receptor was analyzed, and recombined into a pCDNA3.1 vector (Invitrogen) as an expression vector and cultured in a large amount. HEK293 established cells (ATCC) derived from the human fetal kidney were seeded, the expression plasmid (1 μg) obtained above were added thereto with LIPOFECTAMINE 2000 (Invitrogen; 2 μl), the gene was transfected into HEK293 cells, and the expression cells were screened with a drug-resistant marker, Geneticin (G418 sulfate 500 μg/ml; Kanto Chemical Co., Inc.). Thus prepared recombinant cells which expressed the gene were cultured in a medium containing D-MEM (Dulbecco's modified eagle medium, Sigma), 10% FCS (Fetal calf serum: fetal bovine serum), 1% Pc./Sm (Penicillin/Streptomycin, Invitrogen), and 500 μg/ml G418 for 3 days. These experimental operations followed a manual for gene operation experiment and an instruction appended in a reagent, and the like, such as a known method (Sambrook, J. et al, Molecular Cloning-A Laboratory Manual", Cold Spring Harabor laboratory, NY, 1989).

Test Example 2: Test on a human 5-$HT_{5A}$ receptor binding inhibition

(1) Preparation of a membrane from HEK293 cells for forced expressions of a human 5-$HT_{5A}$ receptor

HEK293 cells for forced expressions of a human 5-$HT_{5A}$ receptor were cultured in a F500 plate, and scraped with a scraper. After centrifugation, the precipitate was collected, and an incubation buffer (50 mM Tris (HCl) (pH 7.4), 10 mM $MgSO_4$ and 0.5 mM EDTA (ethylenediamine tetraacetic acid)) was added thereto. After homogenization, it was

further centrifuged, and the incubation buffer was added to the precipitate, followed by thoroughly suspending. The operation was repeated, and protein concentration was measured, thereby completing preparation of the membrane.

(2) Test on a human 5-$HT_{5A}$ receptor binding inhibition

A solution of the compound to be tested and 100 μM 5-CT (5-carboxamidetriptamine) in DMSO was added to a 96-well plate at 2 μl/well, suspended in an incubation buffer, and a membrane from HEK293 cells for forced expressions of a human 5-$HT_{5A}$ receptor prepared at 200 μg/ml was added at 100 μl/well. After incubation at room temperature for 15 minutes, a [$^3$H]5-CT solution (2 nM [$^3$H]5-CT, incubation buffer) was added thereto at 100 μl/well. Separately, 100 μl of the solution was distributed into a liquid scintillation vial, and 2 ml of Aquasol II (registered trademark) was added thereto, followed by stirring. Then, radioactivity was measured by a liquid scintillation counter. It was incubated at 37°C for 60 minutes. The reaction mixture was sucked into 96-well GF/C filter plate that had been pre-treated with 0.2% polyethyleneimine, and washed six times with an ice-cooled, 50 mM Tris (pH 7.5) buffer. The GF/C filter plate was dried.
Microscint TMPS (registered trademark) was added thereto at 40 μl/well. Radioactivity remaining on the GF/C filter plate was measured by a top counter.
The [$^3$H]5-CT binding inhibiting activity by the compound to be tested in each experiment was determined as an $IC_{50}$ value with a radioactivity upon addition of DMSO alone being 0% inhibition, and a radioactivity upon addition of 1 μM 5-CT being 100% inhibition. Separately, Ki values were calculated from the Kd value of the [$^3$H]5-CT determined from Scatchard analysis, by the following equation.

$$Ki = IC_{50} \text{ (1+Concentraion of ligand added/Kd (4.95 nM))}$$

As a result, it was demonstrated that compound of formula (I) as an active ingredient of the medicine of the present invention has a potent human 5-$HT_{5A}$ receptor binding inhibiting activity.
For example, the compound of Example 1 gave a Ki value of 0.96 nM. Furthermore, the compounds of Examples 2-7, 9-14, 18, 25, 26, 31, 32, 35, 36, 42-50, 57-62, 66-71, 73, 75-78, 80-83, 85, 87-90, 92, 95, 96, 104-107, 109, 110, 113, 114, 116-119, 121, 124, 125, 128, 129, 131, 132, 138-140, 142, 143, 145-151, 155-157, 160, 161, 167, 169, 174, 175, 177, 178, 185, 186, 188, 190, 191, 197 and 198 gave Ki values ranging between 0.3 nM and 3 nM respectively, the compounds of Examples 8, 15-17, 19-24, 27-30, 33, 34, 37, 38, 40, 41, 51-56, 63-65, 72, 74, 79, 84, 86, 91, 93, 94, 97, 99, 100, 102, 103, 108, 112, 115, 120, 122, 123, 127, 130, 133-137, 141, 144, 152-154, 159, 162-166, 170, 172, 173, 179, 180, 182-184, 187, 189, 192, 194, 196 and 199-202 gave Ki values ranging between 3nM and 30nM respectively, and the compounds of Examples 39, 98, 101, 111,126,158,168,171,176,181,193 and 195 gave Ki values ranging between 30nM and 300nM respectively.
As described above, it was confirmed that compound of formula (I) has 5-$HT_{5A}$ receptor affinity.

Test Example 3: Improvement effect on increase in motion induced by methamphetamine or MK-801 in mice.

The improvement effect of compound of formula (I) was evaluated by measuring the quantity of motion by IR irradiation when a compound was administered to a mouse in which hyperactivity was caused by methamphetamine (hereinafter, simply referred to as "MAP") or MK-801, known as an animal model of schizophrenia.

(1) Animal

Species: Male ICR mouse

(2) Operation procedure

The animal was taken out of the breeding cage, orally administered with a compound to be tested, and then placed into a cage for breeding. After 30 minutes, the animal was put into a cage for measurement, and motion with the compound to be tested alone was measured. After 30 to 90 minutes, the animal was taken out, and intraperitoneally administered with a drug for increasing the motion (MAP; 1 mg/kg or MK-801; 0.3 mg/kg, dissolved in a physiological saline, respectively). Then, motion for a certain period of time (60 minutes) was measured by using a motion measurement device (CompACT AMS from Muromachi Kikai Co., Ltd.) by means of an infrared sensor.

(3) Analysis

For normal mouse (a mouse administered with physiological saline) and mouse administered with a drug for increasing the motion, a Student's T test was performed for evaluation for each interval. For a mouse group administered with the compound to be tested, an assay was performed using a solvent (vehicle) group and a Dunnett's T test. For the evaluation, if there was a significant difference ($P<0.05$), it was considered that there is an effect.
As a result, compound of formula (I) inhibited the increase in the motion of the mouse induced by the drug. For example, the compound of Example 1 significantly inhibited the hyperactivity caused by MK-801 at a dose of 0.1 mg/kg.
As described above, it was confirmed that compound of formula (1) has an effect of improving schizophrenia.

Test Example 4: Improvement effect of spontaneous alternation behavior caused by Scoporamine or MK-801 in mice

Effect of compound of formula (I) on improvement on cognitive impairment was evaluated by using a known performance test method as a model with short-term learning disorder.

(1) Animal

Species: Male ddY mouse

(2) Measurement method

A mouse was placed at the end of one arm of a Y-maze having arms with the same length in three directions, and then explored freely and the number of arm entries was counted for 8 minutes. Spontaneous alternation behavior was defined as entries into all three different arms on consecutive occasions. The ratio of the number of this behavior to the total number of entries was calculated as an alternation rate by the following formula:
Alternation rate (%) = Number of spontaneous alternation behaviors/(Total number of entries - 2)$\times$100.
The compound to be tested was orally administered 50 minutes prior to test, and after 30 minutes, 0.5 mg/kg scopolamine or 0.15 mg/kg MK-801 (in the case of a normal group, physiological saline was administered) was intraperitoneally administered. In addition, a vehicle was orally administered to the normal group (to which physiological saline was administered) and a control group (to which 0.5 mg/kg scopolamine or 0.15 mg/kg MK-801 was administered), when the compound to be tested was administered thereto. Physiological saline was intraperitoneally administered to the normal group, when scopolamine was administered thereto.

(3) Data analysis

If a significant difference between the normal group and the control group (Student's t test) was approved in the alternation rate (%), it was considered to have learning disorder by the administration of Scoporamine or MK-801. By carrying out a Dunnett's test on the group administered with the compound to be tested relative to the control group, the presence or absence of improvement effect of the compound to be tested on learning disorder was evaluated. For each assay, it was considered that there was a significant difference when $p<0.05$.
As a result of this test, it was confirmed that compound of formula (I) shows improvement effect on learning disorder and has an effect on cognitive impairment.

Test Example 5: Improvement effect for a disorder of PCP-induced prepulse inhibition (PPI) in rats

When a sound stimulus is given to a human, a startle reaction occurs, but for a normal human, this startle reaction is inhibited when the sound stimulus is preceded by a weak sound stimulus. This inhibiting action is lowered in a patient with schizophrenia. It is known that when a rat is administered with PCP (phencyclidine), a similar symptom to human schizophrenia occurs and is known as a model for evaluating information processing disorder as cognitive impairment of schizophrenia.
Effect of compound of formula (I) on improvement of schizophrenia was evaluated by using this model with prepulse inhibition disorder caused by PCP. Specifically, it followed the method as described in "Neuropsychopharmacology, 1989; 2: 61-66, Mansbach, R.S. and Geyer, M.A. and Brain Research, 1998; 781: 227-235".
As a result of this test, it was confirmed that compound of formula (I) shows improvement effect on a prepulse inhibition disorder and has an effect on information processing disorder included in cognitive impairment of schizophrenia.

Test Example 6: Evaluation for water maze learning disorder in old rats

An effect of compound of formula (I) on dementia was evaluated by using a model with water maze learning disorder known as a disease model for dementia. Specifically, it followed the method described in J Pharmacol Exp Ther, 1996; 279: 1157-73, Yamazaki M. *et al.*
As a result of this test, it was confirmed that compound of formula (I) has improvement effect on learning disorder and an effect for dementia.
From the test results of Test examples 1 to 6, it is suggested that compounds of the present invention are useful for treating or preventing diseases, in which 5-$HT_{5A}$ is concerned, for example treating or preventing dementia, schizophrenia (including symptoms such as positive symptoms, negative symptoms, cognitive impairment and mood disorders), bipolar disorder, attention deficit hyperactivity disorder, psychological disorders (such as panic disorder and obsessive disorder), autism, mood disorders (including anxiety disorder and depression disorder), somnipathy, neurodegenerative diseases and cerebral infarction.
A pharmaceutical preparation containing one or two or more kinds of compound of formula (I) or a salt thereof as an active ingredient can be prepared by using pharmaceutical carriers, excipients, and the like that are each usually used in the art, by a method that is usually used.
Administration may be made in any form for either oral administration by tablets, pills, capsules, granules, powders, and solutions, or parenteral administration by injections for intraarticular injection, intravenous injection, and intramuscular injection, suppositories, ophthalmic solutions, ophthalmic ointments, percutaneous liquids, oinments, percutaneous patches, transmucosal liquids, transmucosal patches, and inhalations.
Regarding the solid composition for oral administration according to the present invention, tablets, powders, granules, or the like are used. In such a solid composition, one, or two or more active ingredients are mixed with at least one inactive excipient such as lactose, mannitol, glucose, hydroxypropyl cellulose, microcrystalline cellulose, starch, polyvinyl pyrrolidone, and/or magnesium meta-silicate alminate. According to a conventional method, the composition may contain inactive additives; for example, a lubricant such as magnesium stearate, a disintegrator such as carboxymethylstarch sodium, a stabilizing agent, and a dissolution promotor. As occasion demands, tablets or pills may be coated with a sugar, or a film of a gastric or enteric material.
The liquid composition for oral administration includes pharmaceutically acceptable emulsions, solutions, suspensions, syrups, elixirs, and the like, and contains an inert diluent that is commonly used, such as purified water or ethanol. In addition to the inert diluent, this liquid composition may contain an auxiliary agent such as a solubilizing agent, a moistening agent, and a suspending agent, a sweetener, a flavor, an aroma, and an antiseptic.
Injections for parenteral administration include aqueous or non-aqueous sterile solutions, suspensions, and emulsions. Examples of the aqueous solvent include distilled water for injection, and physiological saline. Examples of the non-aqueous solvent include propylene glycol, polyethylene glycol, vegetable oils such as olive oil, alcohols such as ethanol, and Polysorbate 80 (Pharmacopeia). Such a composition may further contain a tonicity agent, an antiseptic, a moistening agent, an emulsifying agent, a dispersing agent, a stabilizing agent, and a dissolution promotor. These are sterilized, for example, by filtration through a bacterium-retaining filter, blending of bactericides, or irradiation. In addition, these can also be used by producing a sterile solid composition, and dissolving or suspending it in sterile water or a sterile solvent for injection prior to its use.
Examples of the drug for external use include ointments, plasters, creams, jellies, cataplasms, sprays, lotions, ophthalmic solutions, and ophthalmic ointments. The drug contains commonly used ointment bases, lotion bases, aqueous or non-aqueous solutions, suspensions, emulsions, and the like. Examples of the ointment bases or lotion bases include polyethylene glycol, propylene glycol, white vaseline, bleached bee wax, polyoxyethylene hydrogenated castor oil, glyceryl monostearate, stearyl alcohol, cetyl alcohol, lauromacrogol, and sorbitan sesquioleate.
A transmucosal agent such as an inhalations and a transmucosal agent can be used in a solid, liquid or semi-solid state, and may be produced in accordance with a conventionally known method. For example, a known excipient, and also a pH adjusting agent, an antiseptic, a surfactant, a lubricant, a stabilizer, a viscosity-increasing agent, and the like may be appropriately added thereto. For their administration, an appropriate device for inhalation or blowing may be used. For example, a compound may be administered alone or as a powder of a formulated mixture, or as a solution or suspension by combining it with a pharmaceutically acceptable carrier, using a conventionally known device or sprayer, such as a measured administration inhalation device. The dry powder inhaler or the like may be for single or multiple administration use, and a dry powder or a powder-containing capsule may be used. Alternatively, this may be in a form such as a high pressure aerosol spray which uses an appropriate propellant, for example, a suitable gas such as chlorofluoroalkane, hydrofluoroalkane, or carbon dioxide.
It is suitable that the daily dose is usually from about 0.0001 to 100 mg/kg per body weight in the case of oral administration, preferably 0.0001 to 10 mg/kg, and even more preferably 0.0001 to 1 mg/kg, and the preparation is administered in one portion or dividing it into 2 to 4 portions. Also, in the case of intravenous administration, the daily dose is administered suitably in a range from about 0.00001 to 1 mg/kg per body weight, and the preparation is administered once a day or two or more times a day. In the case of drugs for external use or transmucosal administration, the drug is administered usually in a range from about 0.0001 to 10 mg/kg per body weight, once a day or two or more times a day. The dose is appropriately decided, depending on individual cases by taking into consideration the symptom, age, sex and the like. The content of the active ingredients in the preparation is from 0.0001 to 50%, and more preferably 0.001 to 50%.

Compound of formula (I) can be used in combination with various therapeutic agents or prophylactic agents for the diseases, in which compound of formula (I) is considered effective, as described above. The combined preparation may be administered simultaneously; or separately, and continuously or at a desired time interval. The preparations to be co-administered may be a blend, or prepared individually.

Industrial Applicability

Compounds of the present invention have potent 5-$HT_{5A}$ receptor modulating action, and excellent pharmacological action based on the 5-$HT_{5A}$ receptor modulating action. Pharmaceutical compositions of the present invention can be used for prevention or treatment of 5-$HT_{5A}$ receptor-mediated diseases, and in particular, for prevention or treatment of dementia, schizophrenia, bipolar disorder, or attention deficit hyperactivity disorder.

## Claims

1. A compound of formula (I) or a pharmaceutically acceptable salt thereof:

[Chem. 8]

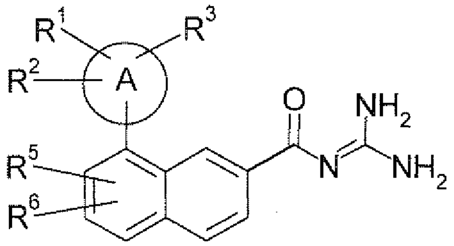

(I)

(wherein symbols have the following meanings:

[Chem. 9]

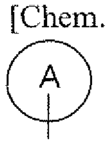

represents phenyl, naphthyl, cycloalkyl, monocyclic or bicyclic heteroaryl, or a saturated or partially unsaturated monocyclic oxygen-containing heterocyclic group;
$R^1$, $R^2$, $R^3$ and $R^4$ are the same as or different from each other and represent H, lower alkyl, halogen, halogeno-lower alkyl, -CN, -$NO_2$, -$NR^bR^c$, -$OR^a$, -O-halogeno-lower alkyl, -C(O)$NR^bR^c$, -C(O)$R^a$, -$CO_2R^a$, $NR^b$C(O)$R^a$, lower alkylene-$OR^a$, phenyl, or, monocyclic nitrogen-containing heteroaryl, or $R^1$ and $R^2$ are combined together to form -O-$(CH_2)_n$-O-, -O-$CF_2$-O-, -O-$C_2H_4$-, or -CO-$C_2H_4$-,
in which the monocyclic nitrogen-containing heteroaryl may be substituted with lower alkyl;
n is 1, 2 or 3;
$R^a$, $R^b$ and $R^c$ are the same as or different from each other and represent H or lower alkyl; and
$R^5$ and $R^6$ are the same as or different from each other and represent H, halogen or lower alkyl).

2. The compound according to claim 1 or a salt thereof, wherein

[Chem. 10]

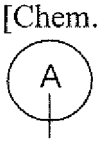

represents phenyl, naphthyl, cyclopropyl, pyridyl, pyrimidinyl, thienyl, thiazolyl, pyrazolyl, oxadiazolyl, quinolyl, isoquinolyl, indolyl, benzoxazolyl, tetrahydropyranyl or dihydropyranyl group.

**3.** The compound according to claim 1 or a salt thereof, wherein

[Chem. 11]

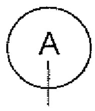

represents phenyl or pyridyl group.

**4.** The compound according to claim 2 or a salt thereof, wherein $R^1$, $R^2$, $R^3$ and $R^4$ are the same as or different from each other and represent H, lower alkyl, halogen, halogeno-lower alkyl, -CN, $-OR^a$, -O-halogeno-lawer alkyl, -C(O) $NR^bR^c$, lower alkylene-$OR^a$, phenyl or oxadiazolyl optionally substituted with methyl group.

**5.** The compound according to claim 2 or a salt thereof, wherein $R^1$, $R^2$, $R^3$ and $R^4$ are the same as or different from each other and represent H, F, Cl, CN or $-OR^a$.

**6.** The compound according to claim 2 or a salt thereof, wherein $R^1$ and $R^2$ are combined together to form $-O-(CH_2)_n-O-$, $-O-CF_2-O-$, $-O-C_2H_4-$, or $-CO-C_2H_4-$.

**7.** The compound according to claim 5 or a salt thereof, wherein $R^5$ and $R^6$ are the same as or different from each other and represent H, F, Cl or methyl.

**8.** The compound according to claim 1 or a salt thereof, which is selected from the group consisting of:

N-(diaminomethylene)-8-(2,4,6-trifluorophenyl)-2-naphthamide,
8-(2-cyano-3-fluorophenyl)-N-(diammomethylene)-2-naphthamide,
N-(diaminomethylene)-8-(3,5-difluoropyridin-4-yl)-2-naphthamide,
8-(3-chloro-5-fluoropyridin-2-yl)-N-(diaminomethylene)-2-naphthamide,
8-(4-cyano-2-methoxyphenyl)-N-(diaminomethylene)-2-naphthamide,
N-(diaminomethylene)-8-(2,5-dichloropyridin-4-yl)-2-naphthamide,
8-(3-chloropyridin-4-yl)-N-(diaminomethylene)-2-naphthamide,
8-(2-chloro-6-fluorophenyl)-N-(diaminomethylene)-2-naphthamide,
N-(diaminomethylene)-8-(2-fluoro-6-hydroxyphenyl)-2-naphthamide,
8-(2-chloro-4-fluorophenyl)-N-(diaminomethylene)-2-naphthamide,
N-(diaminomethylene)-8-quinolin-5-yl-2-naphthamide, and,
N-(diaminomethylene)-8-(2,4-difluoro-6-hydroxyphenyl)-2-naphthamide.

**9.** A pharmaceutical composition comprising the compound according to claim 1 or a salt thereof and a pharmaceutically acceptable carrier.

**10.** The pharmaceutical composition according to claim 9, which is a $5-HT_{5A}$ receptor modulator.

**11.** The pharmaceutical composition according to claim 10, which is an agent for preventing or treating dementia,

schizophrenia, bipolar disorder or attention deficit hyperactivity disorder.

**12.** Use of the derivative according to claim 1 or a salt thereof for the manufacture of an agent for preventing or treating dementia, schizophrenia, bipolar disorder or attention deficit hyperactivity disorder.

**13.** A method for preventing or treating dementia, schizophrenia, bipolar disorder or attention deficit hyperactivity disorder, comprising administering a therapeutically effective amount of the derivative according to claim 1 or a salt thereof to a patient.

## INTERNATIONAL SEARCH REPORT

International application No.
PCT/JP2010/051756

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
See extra sheet.

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CAplus(STN), REGISTRY(STN), JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| P,A | WO 2009/022633 A1 (Astellas Pharma Inc.), 19 February 2009 (19.02.2009), the entire description (Family: none) | 1-12 |
| A | JP 2008-543886 A (Biotron Ltd.), 04 December 2008 (04.12.2008), the entire specification & US 2009/0099239 A1 & EP 1902017 A1 & WO 2006/135978 A1 & CA 2612403 A & KR 10-2008-0021810 A & CN 101208297 A & ZA 200800256 A | 1-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 March, 2010 (02.03.10) | 16 March, 2010 (16.03.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2010/051756

C (Continuation). DOCUMENTS CONSIDERED TO BE RELEVANT

| Category* | Citation of document, with indication, where appropriate, of the relevant passages | Relevant to claim No. |
|---|---|---|
| A | JP 10-81664 A (Hoechst AG.), 31 March 1998 (31.03.1998), the entire specification & US 6087304 A & EP 810206 A1 & DE 19621483 A & DE 59702802 D & NO 972433 A & NZ 314915 A & BR 9703338 A & HU 9700955 A & AU 710065 B & AU 2364597 A & CA 2206366 A & CZ 9701630 A & HR 970292 A & IL 120924 A & PL 318723 A & SK 67097 A & AT 198320 T & TR 9700428 A & ES 2154002 T & DK 810206 T & SI 810206 T & TW 416944 B & PT 810206 E & ZA 9704665 A & RU 2190600 C & ID 16989 A & GR 3035126 T & CN 1167759 A & MX 9703922 A & NO 972433 A0 | 1-12 |

Form PCT/ISA/210 (continuation of second sheet) (July 2009)

**INTERNATIONAL SEARCH REPORT**

International application No.
PCT/JP2010/051756

Continuation of A. CLASSIFICATION OF SUBJECT MATTER
(International Patent Classification (IPC))

*C07C279/22*(2006.01)i, *A61K31/085*(2006.01)i, *A61K31/167*(2006.01)i, *A61K31/192*(2006.01)i, *A61K31/216*(2006.01)i, *A61K31/235*(2006.01)i, *A61K31/277*(2006.01)i, *A61K31/341*(2006.01)i, *A61K31/351*(2006.01)i, *A61K31/36*(2006.01)i, *A61K31/366*(2006.01)i, *A61K31/381*(2006.01)i, *A61K31/404*(2006.01)i, *A61K31/415*(2006.01)i, *A61K31/423*(2006.01)i, *A61K31/4245*(2006.01)i, *A61K31/426*(2006.01)i, *A61K31/44*(2006.01)i, *A61K31/47*(2006.01)i, *A61K31/472*(2006.01)i, *A61K31/505*(2006.01)i, *A61P25/18*(2006.01)i, *A61P25/28*(2006.01)i, *A61P43/00*(2006.01)i, *C07D209/18*(2006.01)i, *C07D213/56*(2006.01)i, *C07D213/61*(2006.01)i, *C07D213/64*(2006.01)i, *C07D213/68*(2006.01)i, *C07D213/84*(2006.01)i, *C07D215/14*(2006.01)i, *C07D217/02*(2006.01)i, *C07D231/12*(2006.01)i, *C07D231/16*(2006.01)i, *C07D239/26*(2006.01)i, *C07D263/56*(2006.01)i, *C07D271/06*(2006.01)i, *C07D271/12*(2006.01)i, *C07D277/20*(2006.01)i, *C07D277/30*(2006.01)i, *C07D307/80*(2006.01)i, *C07D309/22*(2006.01)i, *C07D317/60*(2006.01)i, *C07D317/62*(2006.01)i, *C07D319/18*(2006.01)i, *C07D333/08*(2006.01)i

(According to International Patent Classification (IPC) or to both national classification and IPC)

Continuation of B. FIELDS SEARCHED
Minimum documentation searched (International Patent Classification (IPC))

C07C279/22

Minimum documentation searched (classification system followed by classification symbols)

Form PCT/ISA/210 (extra sheet) (July 2009)

**INTERNATIONAL SEARCH REPORT**

International application No.
PCT/JP2010/051756

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 13
because they relate to subject matter not required to be searched by this Authority, namely:
Claim 13 pertains to method for treatment of the human body by therapy and thus relates to a subject matter on which the International Searching Authority is not required to carry out a search under the provisions of PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0508287 A
- WO 06135978 A
- WO 04112687 A
- US 6087304 A
- US 6093729 A
- JP 8225513 A
- US 5824691 A


### Non-patent literature cited in the description


- *Neuron,* 1999, vol. 22, 581-591
- *Molecular Brain Research,* 1998, vol. 56, 1-8
- *Neuroreport,* 2000, vol. 11, 2017-2020
- *Mol. Psychiatr,* 2001, vol. 6, 217-219
- *J. Psychiatr. Res.,* 2004, vol. 38, 371-376
- **Takeshi Yamamoto et al.** *Chemical and Pharmaceutical Bulletin,* 1997, vol. 45 (8), 1282-1286
- *Prog. Med.,* 1985, vol. 5, 2157-2161
- Iyakuhin no Kaihatsu. Bunshi Sekkei. Hirokawa Publishing company, 1990, vol. 7, 163-198
- Green's Protective Groups in Organic Synthesis. 2006
- **A. d. Meijere ; F. Diederich et al.** Metal-Catalyzed Cross-Coupling Reactions. VCH Publishers Inc, 1997
- Courses in Experimental Chemistry. Maruzen, 2005, vol. 13
- **P. G. M. Wuts ; T. W. Greene.** Green's Protective Groups in Organic Synthesis. 2006
- **Sambrook, J. et al.** Molecular Cloning-A Laboratory Manual. Cold Spring Harabor laboratory, 1989
- *Neuropsychopharmacology,* 1989, vol. 2, 61-66
- **Mansbach, R.S. ; Geyer, M.A.** *Brain Research,* 1998, vol. 781, 227-235
- **Yamazaki M.** *J Pharmacol Exp Ther,* 1996, vol. 279, 1157-73